(19) Europäisches Patentamt
European Patent Office
Office européen des brevets

(11) EP 3 245 298 B1

(12) **EUROPEAN PATENT SPECIFICATION**

(45) Date of publication and mention of the grant of the patent:
**25.09.2019 Bulletin 2019/39**

(21) Application number: **15877408.3**

(22) Date of filing: **13.01.2015**

(51) Int Cl.:
***C12Q 1/68*** *(2018.01)*

(86) International application number:
**PCT/CN2015/070584**

(87) International publication number:
**WO 2016/112488 (21.07.2016 Gazette 2016/29)**

# (54) BIOMARKERS FOR COLORECTAL CANCER RELATED DISEASES

BIOMARKER FÜR ERKRANKUNGEN IM ZUSAMMENHANG MIT KOLOREKTALKREBS

BIOMARQUEUS DE MALADIES LIÉES AU CANCER COLORECTAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**22.11.2017 Bulletin 2017/47**

(73) Proprietors:
- **BGI Shenzhen Co., Limited**
  **Shenzhen, Guangdong 518083 (CN)**
- **BGI Shenzhen**
  **Shenzhen, Guangdong 518083 (CN)**

(72) Inventors:
- **FENG, Qiang**
  **Shenzhen**
  **Guangdong 518083 (CN)**
- **ZHANG, Dongya**
  **Shenzhen**
  **Guangdong 518083 (CN)**
- **WANG, Jun**
  **Shenzhen**
  **Guangdong 518083 (CN)**

(74) Representative: **Lavoix**
**Bayerstrasse 83**
**80335 München (DE)**

(56) References cited:
**CN-A- 102 936 597** **CN-A- 103 695 560**
**CN-A- 103 710 451**

- **M. CASTELLARIN ET AL: "Fusobacterium nucleatum infection is prevalent in human colorectal carcinoma", GENOME RESEARCH, vol. 22, no. 2, 18 October 2011 (2011-10-18), pages 299-306, XP055080533, ISSN: 1088-9051, DOI: 10.1101/gr.126516.111**
- **MARA ROXANA RUBINSTEIN ET AL: "Fusobacterium nucleatum Promotes Colorectal Carcinogenesis by Modulating E-Cadherin/[beta]-Catenin Signaling via its FadA Adhesin", CELL HOST & MICROBE, vol. 14, no. 2, 1 August 2013 (2013-08-01) , pages 195-206, XP055488664, NL ISSN: 1931-3128, DOI: 10.1016/j.chom.2013.07.012**
- **G. ZELLER ET AL: "Potential of fecal microbiota for early-stage detection of colorectal cancer", MOLECULAR SYSTEMS BIOLOGY, vol. 10, no. 11, 28 November 2014 (2014-11-28), pages 766-766, XP055488670, DOI: 10.15252/msb.20145645**
- **A. D. KOSTIC ET AL: "Genomic analysis identifies association of Fusobacterium with colorectal carcinoma", GENOME RESEARCH, vol. 22, no. 2, 18 October 2011 (2011-10-18), pages 292-298, XP055080536, ISSN: 1088-9051, DOI: 10.1101/gr.126573.111**

EP 3 245 298 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

SEQUENCE LISTING

<110> BGI SHENZHEN CO., LIMITED BGI SHENZHEN

<120> BIOMARKERS FOR COLORECTAL CANCER RELATED DISEASES

<130> IEC150001PCT

<160> 20

<170> PatentIn version 3.5

<210> 1
<211> 816
<212> DNA
<213> Peptostreptococcus anaerobius 653-L

<220>
<223> isolated from gut, Peptostreptococcus anaerobius 653-L

<400> 1

```
atggccaaaa cacctatcgt agataagggg tgcttcatat cgaatgatgt taaaaggtca        60
atagttttaa acctatgtga gactaagtca atggatctaa ttgcaagaga acactgtgta       120
tctcctagta gtgttgccag aatacttcgt ttaactgaag ataggagaag aaaaaattat       180
cttcctagga ttctatcaat agacgaattc aagtcagtaa atacagttga tgcgtctatg       240
agtgtaaatt taactgattt agaaggcggt catatttttg atatcctggt ggataggagg       300
caaagatacc tctttgagta ctttaattcc tatcccttga aggtcagaaa aagggtagaa       360
tatgtgacta cagacatgta taagccatat attgatcttg ccaagaaggt ctttccaaat       420
gccaatattg tggtagataa attccatata gtacagctct tgacaagaga gctaaacaag       480
ttaaggataa atgagatgaa gaagcttaat accaggtcta gagagtataa aatactgaag       540
agatactgga aaatacccct taggaagaag agagacttaa acagtatata tttttacaag       600
aataggcact ttaaaaatat gaccagttca attgatatat tagactatat gttaaaggaa       660
tttcccaact taaaagaggc ctatgatttt tatcaaaact tcctattaag tatatctaat       720
aatgatgtcg ctatgcttga agacattcta aatactagga ctgatgaaat tcccatgtgt       780
tttaggaaga gtataaaaag ccttaaaaag cttaga                                 816
```

<210> 2
<211> 594
<212> DNA
<213> Unknownn

<220>
<223> isolated from gut, unidentified

<400> 2

```
atggcaatgc tcactgtaga aaatatcaat gtatattacg gcgtgatcca cgcccttaaa      60
gacatctcct ttcaggtaaa cgaaggcgag atcgtcgcac tgatcggcgc aaacggtgcc     120
ggcaaaacca ccaccctgca gactgtcagc ggcatgctga gcgcaaagtc cggttcgatc     180
cgatttcagg atcaggagat ttccagaatg ccggagcaca aaatcgtgaa gcagggaatt     240
tcccacgtcc ccgaaggacg ccggatgttc tccaatctga cggttttgga aaacctgaaa     300
atgggcgctt acaccagaaa agacaagcag gaaatcaaca attccctgga aatggtttat     360
gagcggtttc cccgcttaaa ggaacgtacc cgccagctgg caggaactct ttccggcggt     420
gaacagcaga tgcttgcaat gggacgtgca ctgatgtctc atccgaagat catccttctg     480
gatgaaccgt ctatgggact ttcaccgatt tttgtaaatg agattttcga aattatcaag     540
aaagtcagtg cagccggcac gaccgtactt ctggtagagc agaatgcaaa gaaa           594
```

<210> 3
<211> 873
<212> DNA
<213> Unknownn

<220>
<223> isolated from gut, unidentified

<400> 3

```
atgaaacgta ttttattaac tggagcaagt ggatttatag gtaaaaacat taaagagaca      60
ttaaacagta aatatgacat atggagcccg tcaagccagg agctggattt aaaagatacc     120
gaatgcgttg aagcatattt gaagcagcat tctttcgatg taatattgca tgcagcaaat     180
tgtaatgata caaggaattc catatcagca tacgatgtac tcaatggaaa tctcagaatg     240
ttttttaacc tagagagatg ttctcactat tatggaaaaa tgatttattt tgggtctggg     300
gcagaatatg acagaagtaa taacatccct aatatgtcag aggactattt tgataccagt     360
gttccgaaag atgcttacgg actttcaaaa tatattatgg caaaagcctg tttaaatcag     420
aagaacattt atgaattgtg tttatttgga gtatacggaa aatatgagga atgggagaga     480
agatttatct ctaatgcgat atgtcgtgca ttaaagggta tggatattac gcttcataaa     540
aatgtatact ttgattattt gtgggtagat gacctcataa aaattatttc ttttttcatt     600
gagaaagata acttgaggta caagaggtac aatgtgtgta gaggcgagaa ggttgatcta     660
tattcgctgg cagtacaggt aaagaagact ttggatagcg aatgttcaat attagttggt     720
gagcctggat ggaagaggga gtatactgcg gataacaata gaatgttgaa cgaaatgaat     780
ggtttatctt ttacaaaact ggaagtgacg atagctgaat tgtgtgaata ttataaagag     840
catttatcag aaatagttac tgaaaaattg taa                                  873
```

<210> 4
<211> 1062
<212> DNA
<213> Roseburia intestinalis XB6B4

<220>
<223> isolated from gut, Roseburia intestinalis XB6B4

<400> 4

atggaaaaag taaaggcatt ttgtaaacgg aaaaacattg agatatccgt caagcgctac 60
ctgattgatg cacttggtgc gatggcacag ggattatttg catcgctttt gatcggaacg 120
atcatcagta cacttggaac gcagcttaat attccgattc ttgtgacagt cgggacttac 180
gcgaaagcgg cagtcggacc ggcaatggcg atcgcaatcg gatatgcact gcaggcagcg 240
cctttagtac tgttttcact tgcggcagtc ggtgcggcgg caaatgaact tggcggggca 300
ggcggaccgc ttgcggtact tgtggttgca atttttgcag cagaatttgg aaaagcagtt 360
tccaaagaga caaaaatcga tattattgtc actccgtttg tgaccatttt tgtcggggtc 420
gcgctttcta tctggtgggc tccggcgatc ggtgcggcag cgagtgcagt cggtaatgcg 480
atcatgtggg caaccgagct gcagccgttt ttcatgggaa tcattgtatc tgtgatcgtc 540
gggattgcac tgacactgcc gatcagcagc gcagcaatct gtgcagcact tggactgacc 600
ggattagccg gtggtgcagc acttgccgga tgctgtgcgc agatggtcgg atttgcagtg 660
gcaagtttcc gtgaaaataa atggggcgga ttgtttgcac agggaatcgg tacatccatg 720
cttcagatgg gtaatatcgt gaaaaatccg cgcatctggc tgccggcgac attggcgtct 780
gcaatcaccg gaccgatcgc aatgtgtctg ttccatttac agatgaatgg tgcagcagtt 840
tcctccggta tgggaacctg tggactggtc ggacagattg gtgtctatac gggatggatc 900
gcagatattg aagcgggaag caaagctgcc attacaccga tggactggat cggactgatt 960
ttcgtaagct ttcttctgcc gggcgtttta tcatggcttt ttagtgtgtt attccgtaag 1020
atcggctgga tcaaagaagg cgatatgagg ctggacttat aa 1062

<210> 5
<211> 627
<212> DNA
<213> Clostridium hathewayi DSM 13479

<220>
<223> isolated from gut, Clostridium hathewayi DSM 13479

<400> 5

atgcctatac ttcagcagct tctcacatta gtagagcagc acttcggtaa caaatgcgaa 60

atcgtgcttc atgatctgac aaaggattac aaccatacca ttgtcgatat ccgaaacgga 120

gacattaccc atcgttccat cgggggctgc ggaagcaact tagggctgga agtcctgcgc 180

ggaaccgtgc tggatgggga tcgttttaac tatgttacca ccacacagga cggaaagatt 240

ctccgttcct catcgatcta tctaaaaaat gatcagggcg aggtcatcgg atcgatctgc 300

gtgaacctgg atatcacaga gacacttcag tttgaagggt atttacgcca gtttaaccag 360

tttgacagct ttacttccaa cgacgaggag attttcgctc ccgacgtgaa taatcttctc 420

agccatctga ttcagatggg acaggaacag atcggaaagc ctgcgctgga gatgaacaag 480

aacgagaaga ttgagtttat ccgtttcctt gaccagaaag gagcattcct catcacgaag 540

tccggggaac agatctgtga acttctggga atcagcaaat ttacctttta taattacctt 600

gaaagcagcc gcagccagtc ggattcg 627

<210> 6
<211> 1161
<212> DNA
<213> Unknownn

<220>
<223> isolated from gut, unidentified

<400> 6

```
atgaaaatca aacaattagc gaaaagcgca tcattcttgc tggtggcagg ttttatcagt      60
tttactattc cgtcgtgtag cagtgaagaa gaaatcatca tccttcagga tgtaaaagta     120
aacagtgaaa gcttcaatct ggccgaagac ggcagtacga ccatagaagt caaggtagta     180
cccgaaaata ctccaatagc caaagccgta ctcagcacat cattatttaa tgaaagcggt     240
gttttcgaag taacccgact cactcccaaa ggtaacggtg tatggcagat agcagcaaaa     300
gtaaaggact tctcacgcat tcaaaacggt caggacgtaa tactttccgt ctatcaggaa     360
gataatatgt atatccaaac cacattgaaa ataaacgacc catatagcat cgagggtaaa     420
tatacaccgg tccatccgca agcctttact ttctacagtg ccgaagacgg caaactgatg     480
gagattccgt tcatcatcac agccgacaac gcagccgacc ttgccgccat cagctacgac     540
aatataaagg tagtcaatgg caccggaagc tctacaccca gcataagtat cacacatttc     600
gcaatagctc cgatgacagg taaaacaggc ttctatctgc aagtggataa cgcccaactc     660
gaaacggtaa aaaaagccat cacaaccatc gctttttttgg actgccgggt tatgataacc     720
ggccctaacg gccgtgttgc ctatactcct gtgcgcctca ttgtttcttc tccgaagtgc     780
atcatcaagg acgaccaact cagcctgctg catacagaat tgtccgcccc ggagtttaat     840
agacaaatca ccatagatat gacccacgat ttttatcgtt tgggcaaaca gaatgataaa     900
acaacctttg aggcgtttga aaaccgaggc ttgtataact cacaaggaga aatggcagat     960
gcagaccctc agttcatttc gttgggttat accactcagg gcaaaaatac aacatgtaac    1020
gtaactttaa aacatgatgc cacaattcct gcaatcggca cttaccacat ggtagaacgc    1080
ctaaaaggat attgggaata tgacggaaag aaatatccga ccgtttgtac agacctgcaa    1140
ttccaaatca cgattaaata a                                              1161
```

<210> 7
<211> 336
<212> DNA
<213> Coprobacillus sp. 29_1

<220>
<223> isolated from gut, Coprobacillus sp. 29_1

<400> 7

atggcgattg atactgaatt agcaaaaaga ttacgttcat atcgtaattt taaacattta 60

acacaaaaag atgttgctgc gcatttaaat gttcctcatt ctgcaatttc cgatatagaa 120

aatggtaaaa gagacattac tgttagcgag ttaaaagtgt tttcaaattt atatggtaga 180

agtgtagaag aaattatgag cgggaaaaaa tatgactatt ataatattgc caatatcgct 240

cgtttactta ctgaacttcc tgatgatgat ttaaaagaaa tcatgtttat tattgaatat 300

aaaagaaaaa gaaatgaaga acgtcatttg aaataa 336

<210> 8
<211> 945
<212> DNA
<213> Faecalibacterium prausnitzii L2-6

<220>
<223> isolated from gut, Faecalibacterium prausnitzii L2-6

<400> 8

atgaacagag aaacggtgaa catggtgcgc agtccgattt ctgtggaggg gaacatccgg 60

cttgttccgt attatccggc ctacgataca gcacttgcgt ggtatcagga tgcacagctc 120

tgcaaacagg tagataacag ggacttcgtt tatgatttgc cgctgctgaa gcggatgtat 180

cattatctgg acacacacgg ggaactgttt tatattgagt atcggggtgt gctttgtggt 240

gacgtcagcc tgcggacgac cggcgagctg gccatcgtca tctgcaagga gtaccagaat 300

aaacacatcg ggcggaaggt catcgaaaaa atgctggagc tggctcggga aaggggcttg 360

gcggagtgct tcgcgcacat ctattctttc aatacccagt cgcagaaaat gtttgaatcc 420

attggctttg tcccacagga cgaagaacgc tatatctaca aattgcaaaa aggagaaccg 480

actatgacaa aactgactct ggaagaaaag caggagctca tccggatggc ccttgcggcc 540

agggagaggg cttacgtgcc ttacagcgac tttatggtgg gcgctgccct gcgcgccgag 600

gatggccgtg tctttaccgg ctgccatgtg gagaatgccg cctttacccc caccagctgc 660

gccgagcgca ccgcgctgtt caaagccgtg agcgagggcg tgaccaaatt tacggacatc 720

gccgtggtag gctcccgccg gggcgagatc aatcagcaga tcacctcgcc ctgcggcgtc 780

tgccgtcagg cactgtttga gtttggcggc ccggagctga acgtcatcat ggccaaaacg 840

ccggatgatt tcatggagcg cagcatggat gagctgctgc cctttggctt cggtccctcc 900

aatgtggcgg gcaacaaggc cgtggaagag gaagaaaaag gctga 945

<210> 9
<211> 432
<212> DNA
<213> Peptostreptococcus anaerobius 653-L

<220>
<223> isolated from gut, Peptostreptococcus anaerobius 653-L

<400> 9

```
tatttttaca agaataggca ctttaaaaat atgaccagtt cagttgatat attagattat      60
atgttaaaag aatttcccaa cttaaaagat gcctatgatt tttatcaaaa cttcctatta     120
agtatatcta ataatgatgt ggctatgctt gaagatattc taaatactag gactgataaa     180
ataccaatgt gttttaggaa gagtataaaa agccttaaaa agtttagaaa gtatgtggta     240
aattcactga aatatgacta tacgaatgcc atggtggagg gtaaaaacaa caagataaag     300
gtaattaaaa gagtatccta cggatatagg agttttagga attttaaggc aaggataatg     360
ctaatggaaa ggtataaaat acaaaagggc aacatccata gttatcagtt tgctatggat     420
gctgccgcat aa                                                         432
```

<210> 10
<211> 777
<212> DNA
<213> Unknownn

<220>
<223> isolated from gut, unidentified

<400> 10

```
atgaagaata tgataaaaat atttgaaaat gacgaattcg gaaaagtgag aacagtcatt      60
aaggacggcg aaccgtggct tgtaggaaaa gatgttgcgg aaattttagg gtattccaac     120
acaagggacg ctctttcacg tcatgtggat accgaggata aaaccaccgt cgtgatttcc     180
gacagtggtt caaattacaa gagcaagacc actattatca atgaaagcgg cttttacagc     240
ttagttctct caagcaaaat gccgagagcc aaagagttca ggcgttgggt gaccgccgaa     300
gtcctcccca ccatcagacg caccggcggc tacgtttcca acgaggatat gttcatcaaa     360
aactatctcc cctttctcga cgagccatac cgtgacctgt tccgacttca aatgaccatt     420
atcaacaagc tgaatgaacg tatccgccac gatcagccgc tggtggagtt tgcgaatcag     480
gtgtcaaata ccgataatct tatcgacatg aacgcaatgg caaagcttgc gagagcggaa     540
aatatccccg tcggcagaaa caagctttac ggctggctga aaggaaaagg tgtgcttatg     600
gcaaacaatc tgccgtatca ggcttttatc gaccgcggat atttttccgt aaaggagtcg     660
gtgtttgaaa ctgcgactat gacaaagact tatcagcaga cgtttgttac gggcaggggg     720
cagcagttcg tcataaattt gctgaagaaa tattatggga aggaggtttt gcaataa        777
```

<210> 11
<211> 1935

<212> DNA
<213> Unknownn

<220>
<223> isolated from gut, unidentified

<400> 11

aatatccgat atggcaacgg agctctggta gtagtccggg caagggaaaa ccttgtacat 60
ggcgaagcag agcagattac cttcaatact aaaatattag aaaggtgcgt gaggcatttg 120
agaaatccga ttgaagtatt gaaaactcta caagagaaag caggcaacga gaactatcaa 180
tttgaacgcc tgtaccgaaa tctgtacaac gaggagtttt tcctattggc atacggaaat 240
ctctctgcaa aagagggaaa tctgaccaag ggaacagacg gcgccacaat agacggaatg 300
ggaatggagc ggattcgcaa gctgattgaa agcctgcgga accacagtta ccagccgtcc 360
cctgcgagac gtgcctatat cccaaaatct aatggaaaac ggcgtccgtt aggcataccc 420
tctgttgacg ataagctggt gcaggaagtt gtgaggttaa ttctcgaaag tgtgtatgaa 480
agcaattttt ctgaacattc gcatggtttt agaccgaaca ggagctgtca cacggcactg 540
acccagattc aaagaaactt cacaggggtt aaatggttca ttgagggga catcaaaggt 600
tattttgaca ccatcgacca ccatatcctt gtggatattt taagaaggcg cataaaggac 660
gaatacctaa tctcgctgat atggaaattt ctgaaagccg gatacttaga agactggaaa 720
ttcaatccta cctattccgg cactccgcaa ggctcggtca tcagtccaat acttgccaat 780
atctacctta acgaattcga tacctatgtt gaagaataca tagagaaatt caaccgtggt 840
aaaagacgtg aaagaaacag tgagtatcgc ttttatagtg atggcgcatc gaaactgagg 900
gtaaagtacc gcgggttatg ggaaataatg acagccgatg aaaaagaaaa agccaaatgt 960
gaagtaaatg agctcatgaa aaaagcaaaa cagattccag ctatgaatcc gatggacagc 1020
aattaccgcc gtctgctcta ttgcaggtat gcggatgatt ttatttgcgg agtaatcgga 1080
agcaaggaag atgcagaaac catcaaggct gattttagcc ggtacctgaa agaaaagctg 1140
ggactggata tgtcggaaga aaagacactg attacacact caaacgaaaa agcggcgttc 1200
cttggctacg aaatcgctgt ttccagaagc aatgaataca aaaagataag caacggacag 1260
aaggcaagaa cctttaatgg gcgtgttcat ctatttatgc cacataataa atgggttaag 1320
aagctgacca gttgcggagc aatggaaatc aaacagcagg acggcaaaga aatatggaaa 1380
ccgcaggcga ggaaagacct catcaacaaa gagccgattg aaatcctaag catttacaat 1440
gccgaaattc gtgggctgta caattattat tgtttggcaa gcaacgtatg caagctgcag 1500
aaatattact acatcatgga atacagcatg taccagacgt ttgcagcgaa gtaccgtgat 1560
aatttgcgga aaacgattaa caagcatacc cgaaacggcg tgtttggtgt cagctacact 1620
acaaaaaccg gcaacgagaa acgggcgaca ttcgtgaaag gaagcttcca aaaacggact 1680
gtcagcttag attacagtga tgaaatcccc tcttatcctg ccgcaaaata tagtcggaaa 1740
aacggcttaa ttgagcggtt acagggtgga aaatgtgaac tatgcggaca gcagaccgac 1800
aatgtaaaag ttcatcatgt caggaagctg aaagaattag ccggtatgaa agaatgggaa 1860

agaaaaatgg ttcagatgaa cagaaaaact ctggttgttt gtaatacatg ttatggaaac 1920

ataacaggca agtaa 1935

<210> 12
<211> 750
<212> DNA
<213> Ruminococcus obeum ATCC 29174

<220>
<223> isolated from gut, Ruminococcus obeum ATCC 29174

<400> 12

atgaaaggaa aaagagttat tgcaggcatt ctgcttgcag gaattttagc agttaccctg 60

gcagggtgta aaaacacaga taacactaaa gaagaatcag aaaagccggt tattaccctc 120

ggcagcgata gctatccacc atacaattat ctgaatgagg atggtgtacc gacgggcata 180

gatgtggaac tagctacaga agctttcaaa agaatgggat atcaggtgaa tgtcgtccaa 240

atcaactggg aggagaaaaa agaactggta gagagtggaa agatcgattg tatcatgggt 300

tgtttttcta tggaaggacg tcttgacgat taccgctggg caggggcgta catagcaagc 360

cgtcaggttg tagcggtaaa tgaggacagt gatatttata aattgagtga ccttgaggga 420

aagaacctgg ctgtccagtc cacaactaaa ccggaagtta tatttctgaa ccggttggat 480

aagagaatcc acaaactggg aaatctgatc agtcttggac accgcgagct gatatataca 540

tttcttggga aaggatatgt agatgcagtt gccgcacatg aggaatcaat catccagtat 600

atgaaggatt atgacataga cttccgtatc ctggaagaat cgctgatgat tacggggata 660

ggtgttgctt tcgcaaaaga tgatgacaga ggaattgtga gcagatggac cagacccttg 720

aagaaatgcg taaggatggc acgtctttga 750

<210> 13
<211> 930
<212> DNA
<213> Parvimonas micra ATCC 33270

<220>
<223> isolated from gut, Parvimonas micra ATCC 33270

<400> 13

```
aatcaattta gaattggttt atcaagaatg gagagagttg ttagagaaag aatgtcaact      60
caagatccag accttgctac gcctcaagga cttattaata taagacctct tgttgcgtct     120
ttaaaagaat tcttcggttc ttcacaatta tcacaattca tggatcaaaa caatccactt     180
gcagaactta ctcataagag aagattatca gcattaggac ctggtggtct tagtagagat     240
agagcaggat acgaagtaag agacgttcat gaaagtcact acggaagaat ttgtccgata     300
gaaactccag aaggtccaaa catcggtctt attacttctc ttacaactta tgcaagagtt     360
gatcaatatg gatttattga aacaccatat cgtgttgtaa ataatggaat tgctacaaag     420

gacattgttt atttaactgc tgatgaagaa gatgaagtta ttatcgctca agccaatgaa     480
ccacttgatg aaaatggacg ttttgtaaac gaaagagtaa gtggtcgtgg tattaatggc     540
gaaaatgata tttatccaag agatacaatt caacttatgg acgtttctcc tcaacaaatt     600
gtatcagttg gtacagcaat gattcctttc cttgaaaatg acgatgctac tcgtgcgttg     660
atgggttcaa acatgcaaag acaagcagtg cctctacttg ttactgaagc tcctattgta     720
ggaaccggta tagaacataa agcggcaaga gatagtggtg ttgttatcat tgctaaaaat     780
tcaggaattg ttacaaaagt tgatagtgat gaaattcata ttaaaagaga tttagataat     840
gtagttgata aatatagatt acttaaattt aaacgttcaa atcaaggaac aacaattaat     900
caaagaccta tagttaatga aaatgacaga                                      930
```

<210> 14
<211> 858
<212> DNA
<213> Faecalibacterium cf. prausnitzii KLE1255

<220>
<223> isolated from gut, Faecalibacterium cf. prausnitzii KLE1255

<400> 14

```
atctccaaac tggaaaaaac gctgcgggca cggttcccga aaacgcagca gggcgaactg      60
ctggccgggg cggtgctggc cttctgcctg ccggtgggca cctttctgct cacaagcgcc     120
gtgtgccttc tggcggcaaa aatcagcccc tggctcggcc ttgccgtgca gatgttctgg     180
tgcgggcagg cgctggcggc aaagggactt gtgcaggaga gccggaacgt ttacaacaag     240
ctggtaaagc ccgacctgcc cgccgcccgc aaggccgtga gccgcatcgt ggggcgggac     300
accgagaacc tgaccgccga gggcgtgacc aaggctgccg tggagactgt ggccgagaat     360
gccagcgacg gcgtgattgc gccgctgctg tacatgctgc tgggcggcgc gccgctggcg     420
ctgacctaca aggccgtcaa caccatggac agcatggtgg gctacaaaaa cgagacctat     480
ctctacttcg gccgggcggc ggcaaagctg gacgatatgg caaactacat tcccagccgc     540
cttgccgccc tgctgtgggc ggcggctgct gccctgaccg gcaacgatgc caaaggcgcg     600
tggcgcatct ggcggcggga ccggcgcaat cacgccagcc ccaacagcgc ccagaccgaa     660
agcgcctgcg ccggtgcgct gggcgtgcag ctggccgggc cggcctacta ctttggcgaa     720
tactacccga aacccaccat cggcgatgcc ctgcgcccca ttgagccgca ggacatcctg     780
cgggccgacc gcatgatgta cgccgccagc attctggcgc tggtgctcgg gcttgtgata     840
cgggggttcg ttgtatga                                                   858
```

<210> 15
<211> 1206
<212> DNA
<213> Unknownn

<220>
<223> isolated from gut, unidentified

<400> 15

```
atgaggttat tttttgatat ggtatgtaac ggcagggcat tgcaaaatgt acaaatgtat      60
aaattgaata tggttttaga tgtacacccc tatgctatta cagcaccgtc aaaaactggt     120
ggccgttggc agacatatgt aaaggaaggt gataagcgta agattataag ggcttcttca     180
aaggaaaaac taatggacaa attatatact gcctattttg ttcaaaatgg tgtttctggt     240
atgaccatgg acaagctttt tctcgaatgg ttagcttata aggaatgtat cacaaatagt     300
atgaatacga ttcgcagaca tgaacaacac tggaaaaagt attttcagga tatttcccca     360
aataaggtat cttcctatga tcgtctggaa ttgcagaaag aatgtaatca gttaataaaa     420
gttaataacc tttcttccaa agaatggcag aatgtaaaaa caattctttt aggtatgttt     480
gactatgcct ttgaaaaagg atatattaat acaaacccca tgcccagtat taaaatcact     540
gttaaattcc gtcaggtcaa taaaaagagt ggtaggactg aaacatatca gacagacgaa     600
tacaaagcac ttatgcaata tctagatgca gaatatacag ctacagaaga ccttgcttta     660
ttggctgtta aatttgattt ttttattgga tgccgtgttg ctgagttggt agctctcaag     720
tggtgtgatg ttgaaaatct acggcattta catatttgta gggaagaggt taaagagtct     780
gtccgtgttg gtgatacctg gaaagatgtt tataccgttt cagagcatac taagacatat     840
acagaccggt ctataaattt agttcctaat gcgattgcta ttttaaatca tatccgtctt     900
aaaatggctt ataatgtatc tgacgatgat tatatcttta cccggaacgg ttcccggatc     960
acttcacgcc agattaatta tattcttgaa aaagcatgta caaaactggg aattatgatt    1020
aagaggtcgc ataaggtaag aaaaacggtt gcaagtcgtc tcaatgtcgg tgaggttccg    1080
ttagattcta ttcgtgagct gttaggtcat gcaaatttaa gcactacact aagttatatt    1140
tataatccgt tatcggaaaa agaaacctat aacctgatgt ccagagcctt ggggaaagtt    1200
caatag                                                               1206
```

<210> 16
<211> 687
<212> DNA
<213> Fusobacterium nucleatum vincentii ATCC 49256

<220>
<223> isolated from gut, Fusobacterium nucleatum vincentii ATCC 49256

<400> 16

```
tctgcaaaag aaaaagttgc tgcattagtt gctgcattaa aagcagatgg atatgatttt      60
actgttggta tccctcttga tacaccaata ggaaaatctg aaagagttgt aagtgctggt     120
aaagggattg gagataaaaa gaatatgaag ctaattgaaa acttagcaaa acaagctgga     180
```

```
gcttctattg gttcttctcg tccagtggca gaaacattgc aatatgtacc tcttgaccgt      240
tatgtaggaa tgtcaggaca aaaatttgtt ggaaaccttt atatagcttg tggaatttca      300
ggagctttac aacatttaaa aggaattaaa gatgcaacaa caatagttgc tataaataca      360
aactcaaatg ctccaatatt taagaatgca gactatggaa tagttggaga tttagcagaa      420
attttacctt tattaactaa ggaattagat aatggagaag ctaaaaaaga tgcaccacct      480
atgaagaaaa tgaagagagt tatacctaga gtagtgtata gtcctcatgt atatgtatgt      540
agtggttgtg gacatgaata caatcctgat ttaggagatg aagattctga cataaaacca      600
ggaactagat ttaaagattt accagaagat tggacttgtc ctgattgtgg agatccaaaa      660
tctggatata tagatgcaaa aaaataa                                          687
```

<210> 17
<211> 1401
<212> DNA
<213> Faecalibacterium prausnitzii M21/2

<220>
<223> isolated from gut, Faecalibacterium prausnitzii M21/2

<400> 17

```
atgccgaacg aacgacatta ctccaatgaa ctgaatctgg aaagcgtggg catcaatctg      60
ccctacaaca tgcaggccga gcagagcgtg ctgggtgcgg tgctgctcaa gccggaaaca     120
ctgaccgacc tggttgagat catccggccg gaaatgttct acacccggca gaacgcccaa     180
atttattcgg aaatgctccg gctgttcacc agcgaccaga ccattgattt cgtcaccctg     240
ctggacgcgg tcatctcaga cggcgtgttt cccagcgcgg acgaggcgaa agtctacctg     300
accggtctgg ccgagacggt gcccagcatc tccaacgtga aagcctacgc ccagatcgtg     360
caggaaaaat atctggtccg ccagctcatg ggtgtggcga aagatatctt gcaggatgcg     420
ggcgacgagc cggacgcgga cctgctgctg gaaaacgccg agcagcgcat ttatgagatc     480
cgctccgggc gggattccag cgccctgacg cccctttctt ccagcatggt ggaaacgctg     540
accaatctgc agaagatcag cggcccggat gccgataagt acaagggcat ccctacaggc     600
ttccgcctgc tggacaccgt gctcaccggc cttggccgcg gcgaccttat tattctggct     660
gcccgccccg gtatgggcaa gaccagtttt gcgctgaaca ttgccacccg cgtggccatg     720
cagcagaaag taccggtggc catcttcagc ctcgaaatga ccaaggagca gctgaccaac     780
cggatcctct cggcggaggc cggcatcgac agccaggcgt tccgcaccgg cgccctccgg     840
gcggaggact gggagtacct ggcccttgcc accgagaagc tccatgacgc gcccatttat     900
atggatgaca cctcgggcat caccatcacc gagatgaaag ccaagatccg ccgggtgaac     960
caggacccca gccgccccaa tgtggggctc atcgtcatcg actatctgca gctgatgacc    1020
acgggccagc gcaccgagaa ccgtgtacag gagatcagct ccatcacccg aaacctcaag    1080
atcatggcca aagagatgaa tgtgcccatc attgcgctga gccagctgtc ccgtgcggtg    1140
gaaaagcagg gcaacaactc ctcccaccgc ccccagctgt ccgacctgcg tgattccggt    1200
tccatcgagc aggacgccga ctgcgtgctg ttcctctacc gtgattctta ttacgccagc    1260
cagaacccgg acggtgccga ggtggacgcc gacacggccg agtgcatcgt ggccaaaaac    1320
cgccacggtg agaccagtac cgtgccgctg ggctgggatg gtgcccacac ccgctttatg    1380
gatgtggact tcaaacgctg a                                              1401
```

<210> 18
<211> 504
<212> DNA
<213> Clostridium symbiosum WAL-14163

<220>
<223> isolated from gut, Clostridium symbiosum WAL-14163

<400> 18

```
atggttgcac ttgtatggct actgattgaa atgaaatata aaatcagtgt cccatctcca      60
ctgttgctca gcatggttta caaacttttg cttccggcta tgcctgccta tcttctggct     120
aaaatcccct ctgggaaatt aacggccagc ttgagaagaa tgccgatttc tacccatatc     180
atgcttgtat tgatcgtcat gctccgcttt gcgccgactg tgctgcatga atttggagaa     240
gtcagggaag ccatgaaaat tcgtggcttc ttaaaatcgg tcggtaatgt tttgaggcat     300
ccaatggaca cgttggaata cgccattgtt ccgatggtgt tccgctcctt aaagatcgcg     360
gacgagttag cagcttctgc catagtcagg ggaattgaaa gcccctacaa gaaagaaagc     420
tactatgtca gccggatcgc tgcgctggat tactttttga ttgttgtcag cgtgggagct     480
gccgtgtgct gctgtctttt atag                                            504
```

<210> 19
<211> 1305
<212> DNA
<213> Unknownn

<220>
<223> isolated from gut, unidentified

<400> 19

```
atgttagcaa tcgtaggttt attaactatc ctggtcgtaa tgtttctgat tatgacaaaa      60
aaatgttcga ctctggtcgc actgattgca gttcccatga ttgcatgtgt tattgtgggt     120
cagggcgccg atatgggagg gtacataacg gccggtatca aaagtgtggc cgccaccgga     180
gtcatgttta tttttgcagt ggcctttttc ggtgtcatgg gtgatgtggg tgcatttgaa     240
atcgtagtga ataaaatact caggattatt gggaaagatc ctttgaaaat ctgtatcggc     300
acgctgatta tcacattgat gacccacctg gacggctccg gcgcaacgac atttttgatc     360
acaataccgg cgctgctgcc gatatacgat aaattgaaga tggatcggcg tgtgctggca     420
```

```
actatagtgg cggcaggagc aggaaccatg aatctcgtcc cttggggagg gccgacgatc      480
cgagcagcga cggcactgga ggtctcactg accgagcttt acaatcctat gattgtccct      540
cagctttgcg gagtcgccgc ctgcgtgaca gtggcggtga tgtttggcct gaaggaacgg      600
aaacgtttaa aagggactct ggaatctgtt tcggtagagc ctccgaaatt tgaggactta      660
ccggaggagg agagagtgaa acgccgtccc caccttgtct ggtttaacat tctgctcatt      720
atagttacaa ttgtgtcatt ggttatggag cttttgccgc cggccggctg ttttatggcg      780
gcgctgtgca tcgcaatgct ggttaactac cgtgatttaa aggatcaggg aaaacggatg      840
gacgagcatg cggtagcggc catgatgatg gcatccaccc tgtttggcgc aggctgcttt      900
accggtatcc tgggaggctg cggcatgctg gaagcgatgg cccagggact ctgtgatatt      960
ctcccggtag ccattatggg tcacattgcg attttggtgg cagttttctc catgcctctg     1020
tcgctgatgt tcgatccgga cagcttctac tatgcagtac ttccggtaat tgcagtggcg     1080
gccgaggtgg ccggtgttcc ggcattggca gtgggccgcg cggcgatatg cggacagatt     1140
actgttggat tccccatttc accactgact ccatccacct tccttctgac aggactaacg     1200
ggcgtggatc tcggggacca tcagaagcac agtttcgtgt ggctgtggct gatttccctg     1260
acgattgtgc tggttgccgt ggtgatgggc gtaattccgg tatag                     1305
```

<210> 20
<211> 708
<212> DNA
<213> Eubacterium ventriosum ATCC 27560

<220>
<223> isolated from gut, Eubacterium ventriosum ATCC 27560

<400> 20

```
gcagcttcaa actacgacct ttgtacaaca atccttagaa atgaatgggg atacgatggt        60
atcgtaatga ctgactggtg ggccaagatg aacgacgttg tagaaggtgg cgaagaatca       120
aatcaggata caagagatat ggttcgctca cagaacgacg tatatatggt tgtaaacaat       180
aacggcgcag aagttaactc aaacaacgac aacacagaga aatcaattaa agagggaaga       240
cttacaatcg gagaacttca gcgagctgca atcaacatct gcaacttcat tctttcagca       300
cctgttattg aaagagaatt agttgacaca gacgttgcaa aacattacga ttcagttcca       360
aatgatcagg ccaagtatga agtatttaac attgaaaaag acaataaggt aatgttcaat       420
agcggagcag aagcaacatt ggaagttgaa gacgaagggg aatacacaat tattgttaac       480
atctcatttg acaagtccaa cttatcacag tcaacagtaa acgttaatgc caacggcaca       540
acaatggtag taatccagac taatggaaca gacggcaact ggattacaca gaagctttgc       600
aaggttaaac ttgacaaggg tgtatacaac ttaaaacttg aagaagtatt agcaggaatc       660
aaagttaaat atattcagtt taagaagatt cctaagaaaa ataaataa                    708
```

## Description

### CROSS-REFERENCE TO RELATED APPLICATION

None

### FIELD

The present invention relates to biomarkers and methods for predicting the risk of a disease related to microbiota, in particular colorectal cancer (CRC) related diseases.

### BACKGROUND

Colorectal cancer (CRC) is the third most common form of cancer and the second leading cause of cancer-related death in the Western world (Schetter AJ, Harris CRC (2011) Alterations of microRNAs contribute to colon carcinogenesis. Semin Oncol 38:734-742). A lot of people are diagnosed with CRC and many patients die of this disease each year worldwide. Although current strategies, including surgery, radiotherapy, and chemotherapy, have a significant clinical value for CRC, the relapses and metastases of cancers after surgery have hampered the success of those treatment modalities. Early diagnosis of CRC will help to not only prevent mortality, but also reduce the costs for surgical intervention.

Current tests of CRC, such as flexible sigmoidoscopy and colonoscopy, are invasive and patients may find the procedures and bowel preparation to be uncomfortable or unpleasant.

The development of CRC is a multifactorial process influenced by genetic, physiological, and environmental factors. Regarding environmental factors, the lifestyle, particularly dietary intake, may affect the risk of CRC developing. Western diet, rich in animal fat and poor in fiber, is generally associated with an increased risk of CRC. Thus, it has been hypothesized that the connection between the diet and CRC, may be the influence that the diet has on the colon microbiota and bacterial metabolism, making both relevant factors in the etiology of the disease (McGarr SE, Ridlon JM, Hylemon PB (2005). Diet, anaerobic bacterial metabolism, and colon cancer. J Clin Gastroenterol. 39:98-109; Hatakka K, Holma R, El-Nezami H, Suomalainen T, Kuisma M, Saxelin M, Poussa T, Mykkänen H, Korpela R (2008). The influence of Lactobacillus rhamnosus LC705 together with Propionibacterium freudenreichii ssp. shermanii JS on potentially carcinogenic bacterial activity in human colon. Int J Food Microbiol. 128:406-410).

Castellarin et al. (2011) Genome Res. 22:299-306 discloses that *Fusobacterium nucleatum* infection is prevalent in human colorectal carcinoma.

Rubinstein et al. (2013) Cell Host & Microbe 14 :195-206 discloses that *Fusobacterium nucleatum* promotes colorectal carcinogenesis by modulating E-cadherin/β-catenin signaling via its FadA adhesion.

Zeller et al. (2014) Molecular Systems Biology 10:766 discloses the use of metagenomics sequencing of fecal sample to identify taxonomic markers that distinguished CRC patients from tumor-free controls in a study population.

Kostic et al. (2011) Genome Res. 22:292-298 discloses the association of *Fusobacterium* with colorectal carcinoma.

### SUMMARY

Embodiments of the present disclosure seek to solve at least one of the problems existing in the prior art to at least some extent.

The present invention is based on the following findings by the inventors:

Intestinal microbiota analysis of feces DNA has the potential to be used as a noninvasive test for finding specific biomarkers that may be used as a screening tool for early diagnosis of patients having CRC, thus leading to a longer survival and a better quality of life. To carry out analysis on gut microbial content in CRC patients, the inventors carried out a protocol for a Metagenome-Wide Association Study (MGWAS) (Qin, J. et al. A metagenome-wide association study of gut microbiota in type 2 diabetes. Nature 490, 55-60 (2012)) based on deep shotgun sequencing of the gut microbial DNA from 128 Chinese individuals (cohort I). The inventors identified and validated 140,455 CRC-associated gene markers. To exploit the potential ability of CRC classification by gut microbiota, the inventors developed a disease classifier system based on the 20 gene markers that are defined as an optimal gene set by a minimum redundancy - maximum relevance (mRMR) feature selection method. For intuitive evaluation of the risk of CRC disease based on these 20 gut microbial gene markers, the inventors calculated a healthy index (CRC index). The inventors' data provide insight into the characteristics of the gut metagenome related to CRC risk, a paradigm for future studies of the pathophysiological role of the gut metagenome in other relevant disorders, and the potential usefulness for a gut-microbiota-based approach for assessment of individuals at risk of such disorders.

It is believed that gene markers of intestinal microbiota are valuable for increasing cancer detection at earlier stages due to the following. First, the markers of the present invention are more specific and sensitive as compared with conventional cancer markers. Second, analysis of stool promises accuracy, safety, affordability, and patient compliance. And samples of stool are transportable. As compared with colonoscopy requiring bowel preparation, polymerase chain reaction (PCR)-based assays are comfortable and noninvasive, so people will participate in a given screening program more easily. Third, the markers of the present invention may also serve as tools for therapy monitoring in cancer patients to detect the response to therapy.

## BRIEF DISCRIPTION OF DRAWINGS

These and other aspects and advantages of the present disclosure will become apparent and more readily appreciated from the following descriptions taken in conjunction with the drawings, in which:

**Fig.1** shows distribution of *P*-value association statistics of all microbial genes in the study. The association analysis of *CRC p-value distribution identified a disproportionate over-representation of strongly associated markers at lower* P-values, with the majority of genes following the expected P-value distribution under the null hypothesis. This suggests that the significant markers likely represent true rather than spurious associations.

**Fig.2** shows species involved in gut microbial dysbiosis during colorectal cancer. Differential relative abundance of two CRC-associated and one control-associated microbial species consistently identified using three different methods: MLG, mOTU and IMG database.

**Fig.3** shows enrichment of *Solobacterium moorei* and *Peptostreptococcus stomatis* in CRC patient microbiomes.

**Fig.4** shows the Receive-Operator-Curve of CRC specific species marker selection using random forest method and three different species annotation methods. **A,** IMG species annotation using clean reads to IMG version 400. **B,** mOTU species using published methods (E. M. Gonçalves, E. M. Salomão, M. C. C. Gomes-Marcondes, Leucine modulates the effect of Walker factor, a proteolysis-inducing factor-like protein from Walker tumours, on gene expression and cellular activity in C2C12 myotubes. Cytokine 64, 343 (10//, 2013)), C, All significant genes clustered using MLG methods (M. R. Rubinstein et al., Fusobacterium nucleatum promotes colorectal carcinogenesis by modulating E-cadherin/beta-catenin signaling via its FadA adhesin. Cell Host Microbe 14, 195 (Aug 14, 2013)) and the species annotation using IMG version 400.

**Fig.5** shows stage specific abundance of three species that are enriched in stage II and later, using three species annotation methods: MLG, IMG and mOTU.

**Fig.6** shows species involved in gut microbial dysbiosis during colorectal cancer. Relative abundances of three enriched in CRC-associated microbiomes, during different stages of CRC (three different species annotation methods were used).

**Fig.7** shows minimum redundancy maximum relevance (mRMR) method to identify 20 gene markers that differentiate colorectal cancer cases from controls. Incremental search was performed using the mRMR method which generated a sequential number of subsets. For each subset, the error rate was estimated by a leave-one-out cross-validation (LOOCV) of a linear discrimination classifier. The optimum subset with the lowest error rate contained 20 gene markers.

**Fig.8** shows principal component analysis (PCA) based on profiles of 20 gene markers separates CRC cases and control individuals. First and second principal components associate with CRC status (PC1 and PC2 explain 31.9% and 13.3% of variance, respectively). Compare this with the analysis based on 2.1 million genes, where no separation can be observed.

**Fig.9** shows discovering gut microbial gene markers associated with CRC. CRC index calculated for CRC patients (black) and control individuals (gray) from this study, shown along patients and control individuals (gray) from earlier studies on type 2 diabetes and inflammatory bowel disease. The box depicts the interquartile ranges between the first and third quartiles, and the line inside denotes the median. CRC indices for CRC patient microbiomes are significantly different from the rest.

**Fig.10** shows ROC analysis of CRC index from 20 gene markers in Chinese cohort I,which shows excellent classification potential with an area under the curve of 0.99.

**Fig.11** shows CRC index using 20 gene markers in 128 samples.

**Fig.12** shows CRC index ,which classifies with an area under the receiver operating characteristic (ROC) curve of 0.97.

**Fig.13** shows correlation between quantification by the metagenomic approach versus quantitative polymerase chain reaction (qPCR) for four gene markers.

**Fig.14-1** shows that ROC analysis reveals moderate potential for classification using CRC index, with an area under the curve of 0.71.

**Fig.14-2** shows CRC index, which classifies with an area under the receiver operating characteristic (ROC) curve of 0.85.

**Fig.15** shows validating robust gene markers associated with CRC. Quantitative PCR abundance (in log10 scale, zero abundance plotted as -8) of two gene markers (m1704941: butyryl-CoA dehydrogenase from *F. nucleatum,* m1696299: RNA polymerase subunit beta, *rpoB,* from *P. micra*) were measured in cohort II consisting 47 cases and 109 healthy controls. **(a)** CRC index based on the two genes clearly separates CRC microbiomes from controls. **(b)** CRC index classifies with an area under the receiver operating characteristic (ROC) curve of 0.84. **(c,d)** The two marker genes show relatively higher incidence and abundance starting in CRC stage II and III compared to control and stage I microbiomes.

**Fig.16** shows CRC index (only using 1696299), which classifies with an area under the receiver operating characteristic (ROC) curve of 0.80.

**Fig.17** shows CRC index (only using 1704941), which classifies with an area under the receiver operating characteristic (ROC) curve of 0.69.

## DETAILED DESCRIPTION

Terms used herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

In one aspect, the present disclosure discloses a gene marker set for predicting the risk of colorectal cancer (CRC) in a subject comprising one or more of the genes as set forth in SEQ ID NOs: 1 to 20.

The present invention concerns a gene marker set for use in the prediction of the risk of colorectal cancer (CRC) in a subject comprising:

a) 20 genes with the sequences as set forth in SEQ ID NOs: 1 to 20;
b) 4 genes with the sequences as set forth in SEQ ID NOs: 1, 9, 13 and 16;
c) 2 genes with the sequences as set forth in SEQ ID NOs: 13 and 16; or
d) the gene with the sequence as set forth in SEQ ID NOs: 13, or *rpoB* gene from *P. micra* encoding RNA polymerase subunit β.

In another aspect, the present disclosure discloses the use of the gene marker set described herein for predicting the risk of colorectal cancer (CRC) in a subject, via the steps of:

1) collecting a sample *j* from the subject and extracting DNA from the sample;
2) determining the abundance information of each of gene marker in the gene marker set; and
3) calculating the index of sample *j* by the formula below:

$$I_j = \left[\frac{\sum_{i \in N} \log_{10}\left(A_{ij} + 10^{-20}\right)}{|N|} - \frac{\sum_{i \in M} \log_{10}\left(A_{ij} + 10^{-20}\right)}{|M|}\right]$$

$A_{ij}$ is the relative abundance of marker *i* in sample *j,* wherein i refers to each of the gene markers in said gene marker set;
*N* is a subset of all CRC-enriched markers in the gene marker set;
*M* is a subset of all control-enriched markers in the gene marker set;
and |*N*| and |*M*| are the sizes (number) of the biomarker respectively in these two subsets;

wherein an index greater than a cutoff indicates that the subject has or is at the risk of developing colorectal cancer (CRC).

In yet another aspect, the present disclosure discloses the use of the gene marker set disclosed herein for preparation of a kit for predicting the risk of colorectal cancer (CRC) in a subject, via the steps of:

1) collecting a sample *j* from the subject and extracting DNA from the sample;
2) determining the abundance information of each of gene marker in the gene marker set; and
3) calculating the index of sample *j* by the formula below:

$$I_j = \left[\frac{\sum_{i\in N}\log_{10}\left(A_{ij}+10^{-20}\right)}{|N|} - \frac{\sum_{i\in M}\log_{10}\left(A_{ij}+10^{-20}\right)}{|M|}\right]$$

$A_{ij}$ is the relative abundance of marker *i* in sample *j,* wherein i refers to each of the gene markers in said gene marker set;
*N* is a subset of all CRC-enriched markers in the gene marker set;
*M* is a subset of all control-enriched markers in the gene marker set;
and $|N|$ and $|M|$ are the sizes (number) of the biomarker respectively in these two subsets;

wherein an index greater than a cutoff indicates that the subject has or is at the risk of developing colorectal cancer (CRC).

In another aspect, the present disclosure discloses a method for diagnosing whether a subject has colorectal cancer or is at the risk of developing colorectal cancer, comprising:

1) collecting a feces sample *j* from the subject and extracting DNA from the sample;
2) determining the abundance information of each of the marker in a gene marker set comprising one or more of the genes as set forth in SEQ ID NOs: 1 to 20; and
3) calculating the index of sample *j* by the formula below:

$$I_j = \left[\frac{\sum_{i\in N}\log_{10}\left(A_{ij}+10^{-20}\right)}{|N|} - \frac{\sum_{i\in M}\log_{10}\left(A_{ij}+10^{-20}\right)}{|M|}\right]$$

$A_{ij}$ is the relative abundance of marker *i* in sample *j,* wherein i refers to each of the gene markers in said gene marker set;
*N* is a subset of all CRC-enriched markers in the gene marker set;
*M* is a subset of all control-enriched markers in the gene marker set;
and $|N|$ and $|M|$ are the sizes (number) of the biomarker respectively in these two subsets;

wherein an index greater than a cutoff indicates that the subject has or is at the risk of developing colorectal cancer (CRC).

In one specific embodiment, the abundance information is gene relative abundance of each of gene marker in the gene marker set which is determined by means of sequencing method.

In another specific embodiment, the abundance information is gene relative abundance of each of gene marker in the gene marker set which is determined by a qPCR method.

In yet another specific embodiment, the cutoff value is obtained by a Receiver Operator Characteristic (ROC) method, wherein the cutoff corresponds to when AUC (Area Under the Curve) reached at its maximum.

In a preferred embodiment, the gene marker set of the present invention consists of SEQ ID NOs: 1 to 20, more preferably, the gene marker set of the present invention consists of SEQ ID NOs: 1, 9, 13 and 16, most preferably, the gene marker set of the present invention consists of SEQ ID NOs: 13 and 16. In another preferred embodiment, the gene marker set consists of SEQ ID NO: 13.

In yet another aspect, the present disclosure discloses the use of a marker as set forth in SEQ ID NO: 13 or *rpoB* gene encoding RNA polymerase subunit β as a gene marker for predicting the risk of colorectal cancer (CRC) in a subject, wherein the enrichment of said gene marker in a sample of the subject relative to a control sample is indicative of the risk of colorectal cancer in the subject

The present invention is further exemplified in the following non-limiting Examples. Unless otherwise stated, parts and percentages are by weight and degrees are Celsius. As apparent to one of ordinary skill in the art, these Examples, while indicating preferred embodiments of the invention, are given by way of illustration only, and the agents were all commercially available.

## Example 1. Identifying 20 biomarker and use gut healthy index to evaluate their colorectal cancer risk

### 1.1 Sample collection

#### 1.1.1 Sample collection in China

Cohorts I (Table 1, used in Example 1, consisting of 74 colorectal cancer patients and 54 control subjects) and cohort II (Table 13, used in Example 3, consisting of 47 colorectal cancer patients and 109 control subjects): Stool samples were collected between 2002 and 2012 in the Prince of Wales Hospital, Hong Kong. The inclusion criteria of all the samples were: 1) not taking antibiotics or other medications, with no particular diets (diabetic, vegetarian, etc) and with normal lifestyle (without extra stress) for a minimum 3 months; 2) a minimum of 3 months after any medical intervention; 3) no history of colorectal surgery, any kind of cancer, or inflammatory or infectious diseases of the intestine. Subjects were asked to collect stool samples in standardized containers at home and store in their home freezer immediately. Frozen samples were then delivered to the hospital in insulating polystyrene foam containers and stored at -80°C immediately until further analysis.

#### 1.1.2 Sample collection in Denmark

Cohort III (Table 15, used in Example 3, consisting of 16 colorectal cancer patients and 24 control subjects): Stool samples were collected from individuals referred to colonoscopy due to symptoms associated with colorectal cancer or from patients who had been diagnosed with colorectal cancer and referred to large bowel resection for their primary cancer disease. All individuals were included at their visit to the out-patient clinic either before colonoscopy or before the operation and always before bowel evacuation. The individuals received a stool collection set including a tube without stabilizing buffer and were instructed to collect a stool sample at home one or two days before initiation of large bowel evacuation. Every included individual kept the sample refrigerated at -18°C and contacted a research nurse who collected the sample. At the laboratory stool samples were immediately snap frozen in liquid nitrogen and subsequently stored at -80°C under 24/7 electronic surveillance until analysis.

All included individuals thus underwent complete colonoscopy either as the primary examination of after the subsequent operation. Exclusion criteria were previous adenoma, previous colorectal cancer and previous or present other malignant diseases.

The collection of stool samples and the recording of data from the included individuals were performed according to the Helsinki II declaration. The protocol was approved by the Ethics Committee of the Capital Region of Denmark (H-3-2009-110) and the Danish Data Protection Agency (2008-41-2252).

Table 1 Baseline characteristics of colorectal cancer (CRC) cases and controls in cohort I. FBG: fasting blood glucose; ALT/GPT: alanine transaminase/glutamate pyruvated transaminase; BMI: body mass index; DM: diabetes mellitus type 2; HDL: high density lipoprotein; TG: triglyceride; eGFR: epidermal growth factor receptor; TCHO: total cholesterol; Cr: creatinine; LDL; low density lipoprotein; TNM: tumor node metastasis staging system.

| **Parameter** | **Controls (n=54)** | **Cases (n=74)** | **Statistical test for differences** | ***P-value*** | **q-value** |
|---|---|---|---|---|---|
| FBG | 5.238889 | 6.428125 | Wilcoxon | 0.0001313 | 0.001314 |
| HDL | 1.735714 | 1.315522 | Wilcoxon | 0.0001877 | 0.001314 |
| Fecal sampling before or after colonoscopy (before:after) | 30:24 | 21:52 | Chi-square | 0.0042174 | 0.019681 |
| Age | 61.75926 | 66.04054 | Wilcoxon | 0.0073739 | 0.025809 |
| TG | 1.054571 | 1.347164 | Wilcoxon | 0.0199168 | 0.055767 |
| ALT/GPT | 23.30556 | 20.36111 | Wilcoxon | 0.0506818 | 0.118258 |
| BMI | 23.46789 | 23.98188 | Wilcoxon | 0.1107815 | 0.221563 |
| TCHO | 5.041143 | 4.820896 | Wilcoxon | 0.299775 | 0.4928 |
| Cr | 73 | 79.25352 | Wilcoxon | 0.3257186 | 0.4928 |
| DM (%) | 16 (29.6%) | 29 (39.2%) | Chi-square | 0.352 | 0.4928 |

(continued)

| Parameter | Controls (n=54) | Cases (n=74) | Statistical test for differences | *P-value* | q-value |
|---|---|---|---|---|---|
| Duration between colonoscopy and fecal sample collection | 27.55294 | 20.54988 | Wilcoxon | 0.7586482 | 0.936833 |
| Gender (M:F) | 33:21 | 48:26 | Chi-square | 0.803 | 0.936833 |
| LDL | 2.915833 | 2.837879 | Wilcoxon | 0.9413451 | 0.989789 |
| eGFR | 74.24108 | 74.14778 | Wilcoxon | 0.9897886 | 0.989789 |
| Stage of CRC (1:2:3:4) | n.a | 18:22:26:8 | n.a | n.a | n.a |
| TNM (T1N0:T2N0:T3N0: T4N0:T2 N1:T3N1:T3N2: T3N+: T4N2:T3N1M1: T3N3M1:T4 N1M1:T4N2M1: M:M1: multiple liver met) | n.a | 12:6:21:1:3: 14:5:2:2:2:1: 1:1:1:1 | n.a | n.a | n.a |
| Lesion specific location (1:2:3: 4:6:7:8:9:NA) | n.a | 3:3:3:2:6:14: 5:29:9 | n.a | n.a | n.a |
| Lesion location (1:2:NA) | n.a | 11:54:9 | n.a | n.a | n.a |

### 1.2 DNA extraction

Chinese samples: Stool samples were thawed on ice and DNA extraction was performed using the Qiagen QIAamp DNA Stool Mini Kit (Qiagen) according to manufacturer's instructions. Extracts were treated with DNase-free RNase to eliminate RNA contamination. DNA quantity was determined using NanoDrop spectrophotometer, Qubit Fluorometer (with the Quant-iTTMdsDNA BR Assay Kit) and gel electrophoresis.

Danish samples: A frozen aliquot (200 mg) of each fecal sample was suspended in 250 μl of 4 M guanidine thiocyanate- 0.1 M Tris (pH 7.5) and 40 μl of 10% N-lauroyl sarcosine. Then, DNA extraction was conducted using bead beating method as previously described (J. J. Godon, E. Zumstein, P. Dabert, F. Habouzit, R. Moletta, Molecular microbial diversity of an anaerobic digestor as determined by small-subunit rDNA sequence analysis. Applied and environmental microbiology 63, 2802 (Jul, 1997), incorporated herein by reference). The DNA concentration and its molecular size were estimated by nanodrop (Thermo Scientific) and agarose gel electrophoresis.

### 1.3 DNA library construction and sequencing

DNA library construction was performed following the manufacturer's instruction (Illumina HiSeq 2000 platform). The inventors used the same workflow as described previously to perform cluster generation, template hybridization, isothermal amplification, linearization, blocking and denaturation, and hybridization of the sequencing primers (Qin, J. et al. A metagenome-wide association study of gut microbiota in type 2 diabetes. Nature 490, 55-60 (2012)).

The inventors constructed one paired-end (PE) library with insert size of 350bp for each sample, followed by a high-throughput sequencing to obtain around 30 million PE reads of length 2×100bp. High-quality reads were obtained by filtering low-quality reads with ambiguous 'N' bases, adapter contamination and human DNA contamination from the Illumina raw reads, and by trimming low-quality terminal bases of reads simultaneously. 751 million metagenomic reads (high quality reads) were generated (5.86 million reads per individual on average)

### 1.4 Species annotation of IMG genomes

For each IMG genome, using the NCBI taxonomy identifier provided by IMG, the inventors identified the corresponding NCBI taxonomic classification at species and genus levels using NCBI taxonomy dump files. The genomes without corresponding NCBI species names were left with its original IMG names, most of which were unclassified.

### 1.5 Data profile construction

#### 1.5.1 Gene, KEGG Ortholog (KO) and genus profiles

The inventors mapped the high-quality reads to the gene catalogue to a published reference gut gene catalogue established from European and Chinese adults((Qin et al. 2012, supra) (identity >= 90%), based on which the inventors derived the gene, KO, and genus profiles using the same method of the published T2D paper (Qin et al. 2012, supra).

#### 1.5.2 mOTU profile

Clean reads were aligned to mOTU reference (total 79268 sequences) with default parameters (S. Sunagawa et al., Metagenomic species profiling using universal phylogenetic marker genes. Nature methods 10, 1196 (Dec, 2013)).549 species level mOTUs were identified, including 307 annotated species and 242 mOTU linkage groups without representative genomes, which were putatively Firmicutes or Bacteroidetes.

#### 1.5.3 IMG-species and IMG-genus profiles.

Bacterial, archaeal and fungal sequences were extracted from IMG v400 reference database (V. M. Markowitz et al., IMG: the Integrated Microbial Genomes database and comparative analysis system. Nucleic acids research 40, D115 (Jan, 2012)) downloaded from http://ftp.jgi-psf.org. 522,093 sequences were obtained in total, and SOAP reference index was constructed based on 7 equal size chunks of the original file. Clean reads were aligned to reference using SOAP aligner (R. Li et al., SOAP2: an improved ultrafast tool for short read alignment. Bioinformatics 25, 1966 (Aug 1, 2009)) version 2.22, with parameters "-m 4 -s 32 -r 2 -n 100 -x 600 -v 8 -c 0.9 -p 3". Then, SOAP coverage software was used to calculate read coverage of each genome, normalized with genome length, and further normalized to relative abundance for each individual sample. The profile was generated based on uniquely mapped reads only.

### 1.6 Analysis of factors influencing gut microbiota gene profile

From the reference gene catalogue (Qin et al. 2012, supra), the inventors derived a subset of 2.1M (2,110,489) genes that appeared in at least 6 samples in all 128 Hong Kong samples, and generated 128 gene abundance profiles using these 2.1 million genes. The inventors used the permutational multivariate analysis of variance (PERMANOVA) test to assess the effect of different characteristics, including age, BMI, eGFR, TCHO, LDL, HDL, TG, gender, DM, CRC status and location, on gene profiles of 2.1M genes. The inventors performed the analysis using the method implemented in package "vegan" in R, and the permuted p-value was obtained by 10,000 times permutations. The inventors also corrected for multiple testing using "p.adjust" in R with Benjamini-Hochberg method to get the q-value for each gene.

When the inventors performed permutational multivariate analysis of variance (PERMANOVA) on 19 different covariates, only CRC status and CRC stage were significantly associated with these gene profiles ($q < 0.05$, Table 2). Thus the data suggest an altered gene composition in CRC patient microbiomes that cannot be explained by other recorded factors.

Table 2 PERMANOVA analysis of microbial gene profiles in cohort I. The analysis was conducted to test whether clinical parameters and CRC status have significant impact on the gut microbiota with $q<0.05$. BMI: body mass index; DM: diabetes mellitus type 2; FBG: fasting blood glucose; HDL: high density lipoprotein; TG: triglyceride; eGFR: epidermal growth factor receptor; TNM: tumor node metastasis staging system; TCHO: total cholesterol; Cr: creatinine; LDL; low density lipoprotein. ALT/GPT: alanine transaminase/glutamate pyruvated transaminase.

| Parameter | Df | SumsOfSqs | MeanSqs | F.Model | $R^2$ | Pr(>F) | q-value |
|---|---|---|---|---|---|---|---|
| CRC Status | 1 | 0.6792933 | 0.6792933 | 1.9596297 | 0.0153144 | 0.0004 | 0.0076 |
| Stage of CRC | 4 | 1.7697175 | 0.4424294 | 1.2778364 | 0.0398977 | 0.0058 | 0.0551 |
| Lesion location(1:2: NA) | 1 | 0.464298 | 0.464298 | 1.31427 | 0.020435 | 0.0536 | 0.2717 |
| BMI | 1 | 0.4600024 | 0.4600024 | 1.3200099 | 0.0104497 | 0.0572 | 0.2717 |
| DM | 1 | 0.4383585 | 0.4383585 | 1.257642 | 0.0098826 | 0.084 | 0.285 |
| FBG | 1 | 0.4319269 | 0.4319269 | 1.2300105 | 0.0123955 | 0.09 | 0.285 |

(continued)

| Parameter | Df | SumsOfSqs | MeanSqs | F.Model | $R^2$ | Pr(>F) | q-value |
|---|---|---|---|---|---|---|---|
| Lesion specific location(1:2:3:4:6:7:8:9:NA) | 1 | 0.421307 | 0.421307 | 1.190278 | 0.018543 | 0.1369 | 0.371586 |
| Age | 1 | 0.3972817 | 0.3972817 | 1.1387282 | 0.0089566 | 0.1923 | 0.456713 |
| HDL | 1 | 0.3641778 | 0.3641778 | 1.0352042 | 0.010246 | 0.3578 | 0.722 |
| eGFR | 1 | 0.3585266 | 0.3585266 | 1.0231375 | 0.0094715 | 0.38 | 0.722 |
| TG | 1 | 0.3522642 | 0.3522642 | 1.001382 | 0.0099145 | 0.4329 | 0.747736 |
| Duration between colonoscopy and fecal sample collection | 1 | 0.3397823 | 0.3397823 | 0.9722612 | 0.0077181 | 0.5036 | 0.761608 |
| Fecal sampling before or after colonoscopy | 1 | 0.3378151 | 0.3378151 | 0.9665887 | 0.0076734 | 0.5211 | 0.761608 |
| TNM | 15 | 5.3000663 | 0.3533378 | 0.9890377 | 0.2036857 | 0.5781 | 0.766587 |
| Cr | 1 | 0.3281613 | 0.3281613 | 0.9330291 | 0.0088077 | 0.6052 | 0.766587 |
| TCHO | 1 | 0.3127842 | 0.3127842 | 0.8878167 | 0.0088 | 0.7198 | 0.854763 |
| LDL | 1 | 0.2994855 | 0.2994855 | 0.8502487 | 0.0084308 | 0.8146 | 0.863233 |
| ALT/GPT | 1 | 0.2976508 | 0.2976508 | 0.847193 | 0.007929 | 0.8178 | 0.863233 |
| Gender | 1 | 0.2677377 | 0.2677377 | 0.7651615 | 0.0060361 | 0.9528 | 0.9528 |

### 1.7 CRC-associated genes identified by MGWAS

#### 1.7.1 Identification of colorectal cancer associated genes

The inventors performed a metagenome wide association study (MGWAS) to identify the genes contributing to the altered gene composition in CRC. To identify the association between the metagenomic profile and colorectal cancer, a two-tailed Wilcoxon rank-sum test was used in the 2.1M gene profiles. The inventors got 140,455 gene markers, which were enriched in either case or control with $P$<0.01(**Fig.1**).

#### 1.7.2 Estimating the false discovery rate (FDR)

Instead of a sequential p-value rejection method, the inventors applied the "qvalue" method proposed in a previous study (J. D. Storey, R. Tibshirani, Statistical significance for genomewide studies. Proceedings of the National Academy of Sciences of the United States of America 100, 9440 (Aug 5, 2003)) to estimate the FDR. In this analysis, the statistical hypothesis tests were performed on a large number of features of the 140,455 genes. The false discovery rate (FDR) was 11.03%.

### 1.8 Taxonomic alterations in CRC microbiomes

The inventors examined the taxonomic differences between control and CRC-associated microbiomes to identify microbial taxa contributing to the dysbiosis. For this, the inventors used taxonomic profiles derived from three different methods, as supporting evidence from multiple methods would strengthen an association. First, the inventors mapped metagenomic reads to 4650 microbial genomes in the IMG database (V. M. Markowitz et al., IMG: the Integrated Microbial Genomes database and comparative analysis system. Nucleic acids research 40, D115 (Jan, 2012)) (version 400) and estimated the abundance of microbial species included in that database (denoted IMG species). Second, the inventors estimated the abundance of species-level molecular operational taxonomic units (mOTUs) using universal phylogenetic marker genes (S. Sunagawa et al., Metagenomic species profiling using universal phylogenetic marker genes. Nature methods 10, 1196 (Dec, 2013)). Third, the inventors organized the 140,455 genes identified by MGWAS into metagenomic linkage groups (MLGs) that represent clusters of genes originating from the same genome (Qin et al. 2012, supra), annotated the MLGs at species level using IMG database whenever possible, grouped MLGs based on these species annotations, and then estimated the abundance of these species (denoted MLG species).

### 1.8.1 Identification of colorectal cancer associated MLG species

Based on the identified 140,455 colorectal cancer associated marker genes profile, the inventors constructed the colorectal cancer associated MLGs using the method described in the previous type 2 diabetes study (Qin et al. 2012, supra). All genes were aligned to the reference genomes of IMG database v400 to get genome level annotation. An MLG was assigned to a genome if >50% constitutive genes were annotated to that genome, otherwise it was termed as unclassified. Total 87 MLGs with gene number over than 100 were selected as colorectal cancer associated MLGs. These MLGs were grouped based on the species annotation of these genomes to construct MLG species.

To estimate the relative abundance of an MLG species, the inventors estimated the average abundance of the genes of the MLG species, after removing the 5% lowest and 5% highest abundant genes. Relative abundance of IMG species was estimated by summing the abundance of IMG genomes belonging to that species. Genus abundances were estimated by analogously summing species abundances.

### 1.8.2 CRC-associated species

Above analysis identified 28 IMG species, 21 mOTUs and 85 MLG species that were significantly associated with CRC status after stratifying by colonoscopy as a confounding factor (Wilcoxon rank-sum test, q<0.05; see Table 3). *Eubacterium ventriosum* was consistently enriched in the control microbiomes across all three methods (Wilcoxon rank-sum tests - IMG: q = 0.002; mOTU: q = 0.0049; MLG: q = $3.33x10^{-4}$). On the other hand, *Parvimonas micra* (q<$7.73x10^{-6}$), *Solobacterium moorei* (q<0.011) and *Fusobacterium nucleatum* (q<0.00279) were consistently enriched in CRC patient microbiomes across all three methods **(Fig.2, Fig.3),** while *Peptostreptococcus stomatis* (q < $7.73x10^{-6}$) was enriched according to two out of three methods. PERMANOVA analysis showed that only CRC status ($P \leq 0.013$ from all three methods) and colonoscopy ($P$ = 0.079 from two methods) explained the quantitative variation in the three CRC-enriched species. All other non-CRC specific factors could not explain the variation with statistical significance ($P$ > 0.18; Table 4). *P. stomatis* has recently been shown to significantly associated with CRC, and *S. moorei* has previously been associated with bacteremia. The results confirmed this association in a new cohort with different genetic and cultural origins. However, a highly significant enrichment of *P. micra* - an obligate anaerobic bacterium that can cause oral infections like *F. nucleatum* - in CRC-associated microbiomes is a novel finding. *P. micra* is involved in the etiology of periodontis, and it produces a wide range of proteolytic enzymes and uses peptones and amino acids as energy source. It is known to produce hydrogen sulphide, which promotes tumor growth and proliferation of colon cancer cells. *P. micra* may represent opportunities for non-invasive diagnostic biomarkers for CRC.

## 1.9 Species level analysis

In order to evaluate the predictive power of these taxonomic associations, the inventors used the random forest ensemble learning method (D. Knights, E. K. Costello, R. Knight, Supervised classification of human microbiota. FEMS microbiology reviews 35, 343 (Mar, 2011)) to identify key marker species in the species profiles from the three different methods. This analysis revealed that 17 IMG species, 7 species-level mOTUs and 27 MLG species were highly predictive of CRC status (Table 5), with predictive power of 0.86, 0.89 and 0.96 in ROC analysis, respectively **(Fig.4).** *P. micra* was identified as a key species from all three methods, while *F. nucleatum, P. stomatis* and *S. moorei* were identified from two out of three methods, providing further statistical support for their association with CRC status.

### 1.9.1 MLG species marker identification

Based on the constructed 87 MLGs with gene numbers over than 100, the inventors performed the Wilcoxon rank-sum test to each MLG with Benjamini-Hochberg adjustment, and 85 MLGs were selected out as colorectal associated MLGs with q<0.05. To identify MLG species markers, the inventors used "randomForest 4.5-36" package in R vision 2.10 based on the 85 colorectal cancer associated MLG species. Firstly, the inventors sorted all the 85 MLG species by the importance given by the "randomForest" method. MLG marker sets were constructed by creating incremental subsets of the top ranked MLG species, starting from 1 MLG species and ending at all 85 MLG species. For each MLG markers set, the inventors calculated the false predication ratio in the 128 Chinese cohorts (cohort I). Finally, the MLG species sets with lowest false prediction ratio were selected out as MLG species markers. Furthermore, the inventors drew the ROC curve using the probability of illness based on the selected MLG species markers.

### 1.9.2 IMG species and mOTU species markers identification

Based on the IMG species and mOTU species profiles, the inventors identified the colorectal cancer associated IMG species and mOTU species with q<0.05 (Wilcoxon rank-sum test with Benjamini-Hochberg adjustment). Subsequently, IMG species markers and mOTU species markers were selecting using the random forest approach as in MLG species markers selection.

### 1.9.3 MLG, IMG and mOTU species Stage enrichment analysis:

Encouraged by the consistent species associations with CRC status and to take advantage of the records of disease stages of the CRC patients **(Table 1),** the inventors explored the species profiles for specific signatures identifying early stages of CRC. The inventors hypothesized that such an effort might even reveal stage-specific associations that are difficult to identify in a global analysis. To identified which species were enriched in the four colorectal cancer progress or health control, the inventors did Kruskal test for the MLG species with gene number over 100, and all IMG species and mOTU species with q<0.05 (Wilcoxon rank-sum test with Benjamini-Hochberg adjustment) to get the species enrichment by the highest rank mean among four CRC stages and control. And the inventors also compared the significance between each two groups by pair-wise Wilcoxon Rank sum test.

In Chinese cohort I, several species showed significantly different abundances in different stages. Among these, the inventors did not identify any species enriched in stage I compared to all other stages and control samples. *Peptostreptococcus stomatis, Prevotella nigrescens* and *Clostridium symbiosum* were enriched in stage II or later compared to control samples, suggesting that they colonize the colon/rectum after the onset of CRC **(Fig.5).** However, *Fusobacterium nucleatum, Parvimonas micra,* and *Solobacterium moorei* were enriched in all four stages compared to controls and were most abundant in stage II **(Fig.6),** suggesting that they may play a role in both CRC etiology and pathogenesis, and implying them as potential biomarkers for early CRC.

### 1.10 CRC biomarker discovery

The inventors proceeded to identify potential biomarkers for CRC from the 140,455 genes identified by the MGWAS approach, using the minimum redundancy maximum relevance (mRMR) feature selection method (H. Peng, F. Long, C. Ding, Feature selection based on mutual information: criteria of max-dependency, max-relevance, and min-redundancy. IEEE transactions on pattern analysis and machine intelligence 27, 1226 (Aug, 2005)). To eliminate the confounding effects of colonoscopy, the inventors selected genes that were significant even after stratifying for colonoscopy, which resulted in 102,514 genes. However, since the computational complexity of mRMR method did not allow us to use all 102,514 genes, the inventors had to reduce the number of candidate genes. First, the inventors selected a stricter set of 24,960 genes with higher statistical significance ($P < 0.001$; FDR $\leq$ 5.23%). Then the inventors identified groups of genes that were highly correlated with each other (Kendall's $\tau > 0.9$) and chose the longest gene in each group, to generate a statistically non-redundant set of 11,128 significant genes. Finally, the inventors used the mRMR method and identified an optimal set of 20 genes that were strongly associated with CRC status **(Fig.7,** Table 6 and Table 7). PCA (principal component analysis) using these 20 genes showed good separation of CRC patients from controls **(Fig.8).** PERMANOVA analysis showed that only CRC status, stage and fasting blood glucose explained the variation in the 20 marker gene abundances with statistical significance ($P \leq 0.01$; see Table 8). Although the inventors cannot rule out other confounding factors, the results suggest that the 20 marker genes characterize differences between CRC and control microbiomes. The inventors calculated a simple CRC index based on un-weighted log relative abundance of these 20 markers, which clearly separated the CRC patient microbiomes from the control microbiomes, as well as from 490 fecal microbiomes from two previous studies on type 2 diabetes in Chinese individuals (Qin et al. 2012, supra) and inflammatory bowel disease in European individuals (J. Qin et al., A human gut microbial gene catalogue established by metagenomic sequencing. Nature 464, 59 (Mar 4, 2010)) **(Fig.9,** median CRC-index for patients and controls in this study were 7.31 and -5.56, respectively; Wilcoxon rank-sum test, $q < 6\times10^{-11}$ for all five comparisons, see Table 9). Classification of the 74 CRC patient microbiomes against the 54 control microbiomes using the CRC index exhibited an area under the receiver operating characteristic (ROC) curve of 0.99 (**Fig.10**), while the areas under the curve (AUC) for classifying type 2 diabetes and IBD patients against the corresponding controls were 0.658 and 0.738, respectively, suggesting that the patterns captured by the index are predominantly CRC-specific. At the cutoff 0.7383 in Fig.10, true positive rate (TPR) was 0.99, and false positive rate (FPR) was 0.07, indicating that the 20 gene markers could be used to accurately classify CRC individuals.

### 1.10.1 Minimum Redundancy Maximum Relevance (mRMR) feature selection framework

To establish a colorectal cancer classification only by gut metagenomic markers, the inventors adopted an mRMR method to perform a feature selection. The inventors used the "sideChannelAttack" package from R to perform an incremental search and found 128 sequential markers sets. For each sequential set, the inventors estimated the error rate by leave-one-out cross-validation (LOOCV) of a linear discrimination classifier. The optimal selection of marker sets was the one corresponding to the lowest error rate. In the present study, the inventors made the feature selection on a set of 102,514 colorectal cancer associated gene markers. Since this was computationally prohibitive to perform mRMR using all genes, the inventors derived a statistically non-redundant gene set. Firstly, the inventors pre-grouped the 102,514 colorectal cancer associated genes that are highly correlated with each other (Kendall correlation > 0.9). Then the inventors chose the longest gene as representative gene for the group, since longer genes have a higher chance of being functionally annotated, and will attract more reads during the mapping procedure. This generated a non-redundant set of 11,128 significant genes. Subsequently, the inventors applied the mRMR feature selection method to the 11,128 significant genes and identified an optimal set of 20 gene biomarkers that are strongly associated with colorectal cancer for colorectal cancer classification, which were shown on Table 6 and Table 7. The gene id is from the published reference gene catalogue as Qin et al. 2012, supra

Table 6 SEQ ID NO. of 20 gene markers

| Gene id | SEQ ID NO: |
|---|---|
| 2361423 | 1 |
| 1804565 | 2 |
| 3531210 | 3 |
| 181682 | 4 |
| 2736705 | 5 |
| 370640 | 6 |
| 2211919 | 7 |
| 4171064 | 8 |
| 3173495 | 9 |
| 3611706 | 10 |
| 482585 | 11 |
| 1559769 | 12 |
| 1696299 | 13 |
| 3976414 | 14 |
| 4256106 | 15 |
| 1704941 | 16 |
| 3319526 | 17 |
| 2040133 | 18 |
| 3246804 | 19 |
| 2206475 | 20 |

Table 7 The 20 gene markers identified by the mRMR feature selection method. Detailed information regarding their enrichment, occurrence in CRC cases and controls, statistical test of association, taxonomy and identity percentage are listed.

| Marker gene id | Enrichment | Wilcoxon rank-sum test | | Occurrence | | | | Identity ( %) | Taxonomy (Blastn to IMG v400) | Description (Blastp to KEGG v59) |
|---|---|---|---|---|---|---|---|---|---|---|
| | | | | Control (n=54) | | Case (n=74) | | | | |
| | | P-value | q-value | N | Rate(%) | N | Rate(%) | | | |
| 2361423 | Case | 2.31E-13 | 4.88E-07 | 11 | 20.37037037 | 62 | 83.78378378 | 93.87 | Peptostreptococcus anaerobius | transposase |
| 3173495 | Case | 6.24E-13 | 6.58E-07 | 10 | 18.51851852 | 61 | 82.43243243 | 93.98 | Peptostreptococcus anaerobius | transposase |
| 2040133 | Case | 7.51E-10 | 4.06E-04 | 14 | 25.92592593 | 62 | 83.78378378 | 99.4 | Clostridium symbiosum | cobalt/nickel transport system permease protein |
| 1696299 | Case | 7.70E-10 | 4.06E-04 | 2 | 3.703703704 | 43 | 58.10810811 | 99.78 | Parvimonas micra | DNA-directed RNA polymerase subunit beta |
| 482585 | Case | 7.41E-09 | 1.05E-03 | 16 | 29.62962963 | 58 | 78.37837838 | NA | NA | RNA-directed DNA polymerase |
| 2211919 | Control | 4.98E-08 | 2.20E-03 | 49 | 90.74074074 | 47 | 63.51351351 | 80.99 | Coprobacillus sp. 8_2_54BFAA | NA |
| 4171064 | Control | 7.50E-08 | 2.61E-03 | 40 | 74.07407407 | 18 | 24.32432432 | 94.94 | Faecalibacterium prausnitzii | cytidine deaminase |
| 1704941 | Case | 7.53E-08 | 2.61E-03 | 2 | 3.703703704 | 39 | 52.7027027 | 99.13 | Fusobacterium nucleatum | butyryl-CoAdehydrogenase |
| 3319526 | Control | 1.08E-07 | 2.79E-03 | 32 | 59.25925926 | 10 | 13.51351351 | 90.01 | Faecalibacterium prausnitzii | NA |
| 3246804 | Case | 1.80E-07 | 3.24E-03 | 1 | 1.851851852 | 35 | 47.2972973 | NA | NA | citrate-Mg2+:H+ or citrate-Ca2+: H+ symporter, CitMHS family |
| 3976414 | Control | 4.42E-07 | 4.07E-03 | 30 | 55.55555556 | 9 | 12.16216216 | 87.12 | Faecalibacterium prausnitzii | adenosylcobinamide-phosphate synthase CobD |
| 4256106 | Control | 7.39E-07 | 4.53E-03 | 28 | 51.85185185 | 9 | 12.16216216 | NA | NA | integrase/recombinase XerD |
| 3531210 | Control | 1.44E-06 | 5.63E-03 | 13 | 24.07407407 | 0 | 0 | NA | NA | GDP-L-fucose synthase |

(continued)

| Marker gene id | Enrichment | Wilcoxon rank-sum test | | Occurrence | | | | Identity (%) | Taxonomy (Blastn to IMG v400) | Description (Blastp to KEGG v59) |
|---|---|---|---|---|---|---|---|---|---|---|
| | | | | Control (n=54) | | Case (n=74) | | | | |
| | | P-value | q-value | N | Rate(%) | N | Rate(%) | | | |
| 3611706 | Control | 1.68E-06 | 5.82E-03 | 15 | 27.77777778 | 0 | 0 | NA | NA | anti-repressor protein |
| 2206475 | Control | 1.81E-06 | 5.95E-03 | 28 | 51.85185185 | 9 | 12.16216216 | 98.59 | Eubacterium ventriosum | beta-glucosidase |
| 181682 | Control | 1.95E-06 | 6.09E-03 | 34 | 62.96296296 | 15 | 20.27027027 | 99.25 | Roseburia intestinalis | NA |
| 1804565 | Control | 2.03E-06 | 6.16E-03 | 22 | 40.74074074 | 4 | 5.405405405 | NA | NA | branched-chain amino acid transport system ATP-binding protein |
| 2736705 | Case | 5.71E-06 | 8.55E-03 | 2 | 3.703703704 | 32 | 43.24324324 | 99.68 | Clostridium hathewayi | NA |
| 1559769 | Control | 1.03E-05 | 1.04E-02 | 27 | 50 | 7 | 9.459459459 | 88.65 | Coprococcus catus | polar amino acid transport system substrate-binding protein |
| 370640 | Control | 2.64E-05 | 1.47E-02 | 14 | 25.92592593 | 0 | 0 | 99.4 | Bacteroides clarus | NA |

### 1.10.2 Definition of CRC index

To exploit the potential ability of disease classification by gut microbiota, the inventors developed a disease classifier system based on the gene markers that the inventors defined. For intuitive evaluation of the risk of disease based on these gut microbial gene markers, the inventors calculated a gut healthy index (CRC index).

To evaluate the effect of the gut metagenome on colorectal cancer, the inventors defined and calculated a CRC index for each individual on the basis of the selected 20 gut metagenomic markers by mRMR method. For each individual sample, the CRC index of sample j that denoted by $I_j$ was calculated by the formula below:

$$I_j = \left[\frac{\sum_{i \in N} \log_{10}(A_{ij} + 10^{-20})}{|N|} - \frac{\sum_{i \in M} \log_{10}(A_{ij} + 10^{-20})}{|M|}\right]$$

$A_{ij}$ is the relative abundance of marker *i* in sample *j,* wherein i refers to each of the gene markers in said gene marker set;.

*N* is a subset of all CRC-enriched markers in these selected gut metagenomic markers (namely, a subset of all patient-enriched markers in selected biomarkers related to the abnormal condition).

*M* is a subset of all control-enriched markers in these selected gut metagenomic markers (namely, a subset of all control-enriched markers in selected biomarkers related to the abnormal condition).

wherein the subset of CRC-enriched markers and the subset of control-enriched markers are shown in Table 7.

And |*N*| and |*M*| are the sizes (number) of these two sets, wherein |*N*| is 8 and |*M*| is 12.

Larger the CRC index, higher the risk of disease. Smaller the CRC index, more healthy the people. The inventors can build an optimal CRC index cutoff based on a large cohort. If the test sample CRC index is larger than the cutoff, then the person is in higher disease risk. And if the test sample CRC index is smaller than the cutoff then he is more healthy at low risk of disease. The optimal CRC index cutoff can be determined by a ROC method when AUC (Area Under the Curve) reached at its maximum.

### 1.10.3 Receiver Operator Characteristic (ROC) analysis

The inventors applied the ROC analysis to assess the performance of the colorectal cancer classification based on metagenomic markers. Based on the 20 gut metagenomic markers selected above, the inventors calculated the CRC index for each sample. The inventors then used the "Daim" package in R to draw the ROC curve.

### 1.10.4 CRC index validation

After establishing CRC-index, the inventors calculated the CRC-index in Chinese cohort I consisting 128 individuals **(Fig.11,** Table 10), and 490 individuals from two previous studies on type 2 diabetes in Chinese individuals (Qin et al. 2012, supra) and inflammatory bowel disease in European individuals (J. Qin et al., A human gut microbial gene catalogue established by metagenomic sequencing. Nature 464, 59 (Mar 4, 2010)). The ability of the CRC index to distinguish CRC patients from the rest was compared using Wilcoxon rank-sum test with Benjamini-Hochberg adjustment for Chinese CRC cohorts, T2D cohorts and IBD cohorts.

Table 9 CRC index estimated in CRC, T2D and IBD patient and healthy cohorts.

| Cohort/group | Median CRC index | Comparison with CRC patients | |
|---|---|---|---|
| | | ***P*-value** | **q-value** |
| CRC patients | 7.30636 | NA | NA |
| CRC controls | -5.558923 | 3.91E-21 | 4.89E-21 |
| T2D patients | 0.2512602 | 1.71E-26 | 2.85E-26 |
| T2D controls | -1.47849 | 2.00E-30 | 1.00E-29 |
| IBD patients | -1.789305 | 6.00E-11 | 6.00E-11 |
| IBD controls | -4.505388 | 1.27E-28 | 3.16E-28 |

Table 10. 128 samples' calculated CRC index (CRC patients and non-CRC controls)

| Sample ID | CRC index | Sample Type (control: non-CRC controls; case: CRC patients) | Sample ID | CRC index | Sample Type (control: non-CRC controls; case: CRC patients) |
|---|---|---|---|---|---|
| A10A | 14.19383056 | case | 502A | -7.79722274 | control |
| M2-PK002A | 9.202164571 | case | 512A | -5.197373955 | control |
| M2-PK003A | 6.596330348 | case | 515A | -3.850212619 | control |
| M2-PK018A | 3.738441263 | case | 516A | -4.579206562 | control |
| M2-PK019A | -1.552560207 | case | 517A | -8.730876643 | control |
| M2-PK021A | 8.625310562 | case | 519A | -9.119326221 | control |
| M2-PK022A | 7.95771066 | case | 530A | -0.043165542 | control |
| M2-PK023A | 10.1028154 | case | 534A | -6.70898767 | control |
| M2-PK024A | 1.859507913 | case | 536A | -7.14849116 | control |
| M2-PK026A | 7.099184942 | case | M2-PK504A | -2.336003525 | control |
| M2-PK027A | 2.148283112 | case | M2-PK514A | -5.530573705 | control |
| M2-PK029A | 9.69680859 | case | M2-PK520B | -8.58680181 | control |
| M2-PK030A | 4.184660423 | case | M2-PK522A | 1.950778822 | control |
| M2-PK032A | 7.335875158 | case | M2-PK523A | -5.741405612 | control |
| M2-PK037A | 4.550762928 | case | M2-PK524A | -9.130416872 | control |
| M2-PK038A | 4.415834566 | case | M2-PK531B | -5.546653702 | control |
| M2-PK041A | 5.977334578 | case | M2-PK532A | -5.569068576 | control |
| M2-PK042A | 5.69903073 | case | M2-PK533A | -8.160297407 | control |
| M2-PK043A | 6.724224328 | case | M2-PK543A | -9.056715419 | control |
| M2-PK045A | 8.489338918 | case | M2-PK548A | -2.427151597 | control |
| M2-PK046A | 10.09963159 | case | M2-PK556A | -3.070447693 | control |

(continued)

| Sample ID | CRC index | Sample Type (control: non-CRC controls; case: CRC patients) | Sample ID | CRC index | Sample Type (control: non-CRC controls; case: CRC patients) |
|---|---|---|---|---|---|
| M2-PK047A | 10.14131194 | case | M2-PK558A | -9.019379986 | control |
| M2-PK051A | 5.617848197 | case | M2-PK602A | -6.53273818 | control |
| M2-PK052A | 5.068450036 | case | M2-PK615A | 4.307491874 | control |
| M2-PK055A | 5.384040448 | case | M2-PK617A | -8.879301252 | control |
| M2-PK056B | 7.334688588 | case | M2-PK619A | -7.886681204 | control |
| M2-PK059A | 4.029993543 | case | M2-PK630A | -4.061470866 | control |
| M2-PK063A | 2.616294256 | case | M2-PK644A | 2.698033772 | control |
| M2-PK064A | 4.066149942 | case | M2-PK647A | -7.856026984 | control |
| M2-PK065A | 7.27803084 | case | M2-PK649A | 0.069506921 | control |
| M2-PK066A | 1.903461677 | case | M2-PK653A | -3.996231994 | control |
| M2-PK067A | 12.49515261 | case | M2-PK656A | -7.593429394 | control |
| M2-PK069B | 2.618969008 | case | M2-PK659A | -8.137346224 | control |
| M2-PK083B | 9.738788076 | case | M2-PK663A | -5.011731262 | control |
| M2-PK084A | 9.579990733 | case | M2-PK699A | -9.958709759 | control |
| M2-PK085A | 6.235288073 | case | M2-PK701A | -1.227789483 | control |
| MSC103A | 8.783772395 | case | M2-PK705A | -9.885773253 | control |
| MSC119A | 5.052422612 | case | M2-PK708A | -6.244142013 | control |
| MSC120A | 6.146086863 | case | M2-PK710A | -6.85601043 | control |
| MSC1A | 11.30413029 | case | M2-PK712A | -8.088632665 | control |
| MSC45A | 11.28949284 | case | M2-PK723A | -5.657226022 | control |
| MSC4A | 0.994547041 | case | M2-PK725A | -7.825464882 | control |

(continued)

| Sample ID | CRC index | Sample Type (control: non-CRC controls; case: CRC patients) | Sample ID | CRC index | Sample Type (control: non-CRC controls; case: CRC patients) |
|---|---|---|---|---|---|
| MSC54A | 14.03552 | case | M2-PK729A | -4.852280607 | control |
| MSC5A | 5.016184446 | case | M2-PK730A | -4.225433172 | control |
| MSC63A | 7.803312153 | case | M2-PK732A | -4.457650321 | control |
| MSC6A | 8.49644053 | case | M2-PK750A | -6.750508944 | control |
| MSC76A | 9.478934126 | case | M2-PK751A | -4.358053381 | control |
| MSC78A | 6.3069609 | case | M2-PK797A | -4.519277041 | control |
| MSC79A | 9.020635328 | case | M2-PK801A | -6.91026768 | control |
| MSC81A | 10.37665297 | case | 509A | -0.206336818 | control |
| M118A | 9.089603466 | case | A60A | 3.357117487 | control |
| M123A | 9.050925877 | case | 506A | -1.540530227 | control |
| M2-Pk-001A | 7.861052045 | case | A21A | -5.548776543 | control |
| M2-Pk-005A | 5.101439907 | case | A51A | -1.747206239 | control |
| M2-Pk-009A | 8.540631454 | case | | | |
| M2-Pk-017A | 6.455139538 | case | | | |
| M84A | 5.404378934 | case | | | |
| M89A | 0.738273449 | case | | | |
| M2-Pk-007A | 8.814262687 | case | | | |
| M2-Pk-010A | 8.420509186 | case | | | |
| M122A | 8.656723607 | case | | | |
| M2-Pk-004A | 3.71514381 | case | | | |
| M2-Pk-008A | 6.55825389 | case | | | |
| M2-Pk-011A | 8.699227128 | case | | | |
| M2-Pk-015A | 7.647130585 | case | | | |
| M113A | 8.972062997 | case | | | |

(continued)

| Sample ID | CRC index | Sample Type (control: non-CRC controls; case: CRC patients) | Sample ID | CRC index | Sample Type (control: non-CRC controls; case: CRC patients) |
|---|---|---|---|---|---|
| M116A | 11.54385563 | case | | | |
| M117A | 2.164298748 | case | | | |
| M2-Pk-006A | 11.64439561 | case | | | |
| M2-Pk-012A | 4.307525456 | case | | | |
| M2-Pk-014A | 10.68683722 | case | | | |
| M2-Pk-016A | 10.22191839 | case | | | |
| M115A | 6.747992005 | case | | | |
| M2-Pk-013A | 6.77362866 | case | | | |

**Example 2. Validating the 20 biomarkers**

The inventors validated the discriminatory power of the CRC classifier using another new independent study group, including 15 CRC patients and 15 non- CRC controls that were also collected in the Prince of Wales Hospital.

For each sample, DNA was extracted and a DNA library was constructed followed by high throughput sequencing as described in Example 1. The inventors calculated the gene abundance profile for these samples using the same method as described in Qin et al. 2012, *supra.* Then the gene relative abundance of each of the markers as set forth in SEQ ID NOs: 1-20 was determined. Then the index of each sample was calculated by the formula below:

$$I_j = \left[\frac{\sum_{i\in N}\log_{10}\left(A_{ij}+10^{-20}\right)}{|N|} - \frac{\sum_{i\in M}\log_{10}\left(A_{ij}+10^{-20}\right)}{|M|}\right]$$

$A_{ij}$ is the relative abundance of marker *i* in sample *j,* wherein i refers to each of the gene markers as set forth in SEQ ID NOs 1-20;

*N* is a subset of all CRC-enriched (case) markers in these 20 selected gut metagenomic markers (namely, a subset of all patient-enriched markers in selected biomarkers related to the abnormal condition);

*M* is a subset of all control-enriched markers in these 20 selected gut metagenomic markers (namely, a subset of all control-enriched markers in selected biomarkers related to the abnormal condition);

wherein the subset of CRC-enriched markers and the subset of control-enriched markers are shown in Table 7;

And |*N*| and |*M*| are the sizes (number) of these two sets, wherein |*N*| is 8 and |*M*| is 12.

Table 11 shows the calculated index of each sample and Table 12 shows the relevant gene relative abundance of a representative sample V1 and V30. In this assessment analysis, the areas under the curve (AUC) for classifying was 0.9733 **(Fig.12).** At the cutoff 0.9945, true positive rate (TPR) was 1, and false positive rate (FPR) was 0.2, validating that the 20 gene markers could be used to accurately classify CRC individuals.

Table 11. 30 samples' calculated CRC index

| Sample ID | Sample Type (control:non-CRC controls; case:CRC patients) | CRC index | | Sample ID | Sample Type (control:non-CRC controls; case:CRC patients) | CRC index |
|---|---|---|---|---|---|---|
| V1 | control | -7.7973 | | V6 | case | 8.6252 |

(continued)

| Sample ID | Sample Type (control:non-CRC controls; case:CRC patients) | CRC index | | Sample ID | Sample Type (control:non-CRC controls; case:CRC patients) | CRC index |
|---|---|---|---|---|---|---|
| V20 | control | -5.1973 | | V17 | case | 0.9944 |
| V13 | control | -3.8501 | | V28 | case | 3.7150 |
| V4 | control | -4.5791 | | V19 | case | 6.5582 |
| V5 | control | -8.7308 | | V2 | case | 8.6991 |
| V16 | control | -9.1192 | | V11 | case | 7.6470 |
| V27 | control | -0.0431 | | V22 | case | 8.9720 |
| V8 | control | -6.7091 | | V23 | case | 11.5438 |
| V9 | control | -7.1484 | | V24 | case | 2.1642 |
| V10 | control | -2.3361 | | V15 | case | 11.6443 |
| V21 | control | -5.5305 | | V26 | case | 4.3076 |
| V12 | control | -8.5867 | | V7 | case | 10.6867 |
| V3 | control | 1.9507 | | V18 | case | 10.2218 |
| V14 | control | 4.3074 | | V29 | case | 6.7481 |
| V25 | control | 3.3570 | | V30 | case | 6.7738 |

Table 12. Gene relative abundance of Sample V1 and V30

| Gene id | Enrichment | SEQ ID NO: | Sample V1(Calculation of gene relative abundance) | Sample V30 (Calculation of gene relative abundance) |
|---|---|---|---|---|
| 2361423 | case | 1 | 0 | 1.97E-07 |
| 3173495 | case | 9 | 0 | 1.24E-07 |
| 2040133 | case | 18 | 0 | 6.37E-07 |
| 1696299 | case | 13 | 0 | 0 |
| 482585 | case | 11 | 0 | 9.96E-07 |
| 1704941 | case | 16 | 0 | 0 |
| 3246804 | case | 19 | 0 | 0 |
| 2736705 | case | 5 | 0 | 0 |
| 2211919 | control | 7 | 7.08E-07 | 0 |
| 4171064 | control | 8 | 4.20E-07 | 0 |
| 3319526 | control | 17 | 3.11E-07 | 0 |
| 3976414 | control | 14 | 4.62E-08 | 0 |
| 4256106 | control | 15 | 0 | 0 |
| 3531210 | control | 3 | 1.82E-07 | 0 |
| 3611706 | control | 10 | 0 | 0 |
| 2206475 | control | 20 | 0 | 0 |
| 181682 | control | 4 | 4.48E-07 | 0 |
| 1804565 | control | 2 | 0 | 0 |

(continued)

| Gene id | Enrichment | SEQ ID NO: | Sample V1(Calculation of gene relative abundance) | Sample V30 (Calculation of gene relative abundance) |
|---|---|---|---|---|
| 1559769 | control | 12 | 1.06E-07 | 0 |
| 370640 | control | 6 | 0 | 0 |

Thus the inventors have identified and validated 20 markers set by a minimum redundancy - maximum relevance (mRMR) feature selection method based on 140,455 CRC-associated markers. And the inventors have built a gut healthy index to evaluate the risk of CRC disease based on these 20 gut microbial gene markers.

**Example 3 Validation of gene markers by qPCR**

Abundances of four randomly selected gene markers, including two enriched in control (m181682 (SEQ ID NO:4) and m370640 (SEQ ID NO:6)) and two enriched in patient (m482585 (SEQ ID NO: 11) and m1704941 (SEQ ID NO: 16)), were further evaluated in 96 stool samples of the sequenced cohort (51 cases and 45 controls, a subset of cohort I) and cohort II of 156 samples (47 cases and 109 controls) using TaqMan probe-based qPCR. Primers and probes were designed using Primer Express v3.0 (Applied Biosystems, Foster City, CA, USA). The qPCR was performed on an ABI7500 Real-Time PCR System using the TaqMan® Universal PCR Master Mixreagent (Applied Biosystems). Universal 16S rDNA was used as internal control and abundance of gene markers were expressed as relative levels to 16S rDNA.

**3.1 Evaluating CRC biomarkers using targeted quantitative PCR**

The biomarkers were derived using the admittedly expensive deep metagenome sequencing approach. Translating them into diagnostic biomarkers would require reliable measurement by simple, affordable and targeted methods such as quantitative PCR (qPCR). To verify this, the inventors randomly selected two case-enriched and two control-enriched gene markers and measured their abundances by qPCR in a subset of 96 samples selected from cohort I (51 cases and 45 controls). Quantification of each of the four genes by the two platforms (metagenomic sequencing and qPCR) showed strong correlations (Spearman $r$=0.81-0.95, **Fig.13),** suggesting that the gene markers could also be reliably measured using qPCR. Next, in order to validate the markers in previously unseen samples, the inventors measured the abundance of these four gene markers using qPCR in 156 fecal samples (47 cases and 109 controls) from an independent Chinese cohort (cohort II; see Table 13). The two control-enriched genes did not show significant associations ($P > 0.31$; Table 14). On the other hand, the CRC-enriched gene markers (m1704941, butyryl-CoA dehydrogenase from *F. nucleatum;* m482585, RNA-directed DNA polymerase from an unknown microbe) significantly associated with CRC status after stratifying by colonoscopy ($P = 0.0015$ and $P = 0.045$, respectively, see Table 14). However, only the gene from *F. nucleatum* remained significant after a Mantel-Haenszel test adjusted for colonoscopy (odds ratio=18.5, $P = 0.0051$). The CRC index based on the abundances of the four genes only moderately classified CRC microbiomes from control microbiomes (AUC=0.73), perhaps suggesting that choosing randomly from the list of 20 biomarkers was not an effective strategy. Nevertheless, the gene from *F. nucleatum* was present only in 4 out of 109 control microbiomes, suggesting a potential for developing specific diagnostic tests for CRC using fecal samples.

**3.2 Accurate qPCR biomarkers identified by validation in an independent metagenomic cohort**

To identify robust biomarkers that can have a more general applicability, the inventors evaluated all 20 gene markers using fecal metagenomes from a cohort with different genetic background and lifestyle: 16 CRC patients and 24 control individuals from Denmark (cohort III). These were symptomatic individuals referred to colonoscopy and all samples were blinded before DNA extraction and analyses (see Table 15). When mapped to the 4.3 million gut microbial genes, the 40 Danish microbiomes exhibited significantly higher gene richness and gene alpha diversity, both in cases (Wilcoxon rank-sum tests, gene count: $P = 1.94\times10^{-5}$; Shannon's index: $P = 5.85\times10^{-5}$) and controls (gene count: $P = 0.0017$; Shannon's index: $P = 9.34\times10^{-4}$; Table 16), agreeing with a recent study and suggesting differences in gut microbial community structure between the Chinese and Danish populations (J. Li et al., An integrated catalog of reference genes in the human gut microbiome. Nature biotechnology 32, 834 (Aug, 2014)). Among the 102,514 genes associated with CRC status in Chinese cohort I, only 1,498 genes could be validated in the Danish microbiomes. However, CRC-enriched genes were shared significantly more between the two populations than control-enriched genes (1,452 out of 35,735 CRC-enriched vs. 46 out of 66,779 in control-enriched; two-tailed chi-squared test, chi-squared=2576.57, $P$ <

0.0001). Over half (53.6%) of the 1,452 CRC-enriched genes were from just three species: *Parvimonas micra* (389 genes), *Solobacterium moorei* (204 genes) and *Clostridium symbiosum* (177 genes) (see Table 17). At the species level, *P. micra* was enriched in CRC microbiomes using all three methods, while *P. stomatis, G morbillorum,* and *S. moorei* were enriched according to two methods (Wilcoxon rank-sum test, $q < 0.05$; Table 18). Notably, all the species that were validated by at least one method were CRC-enriched. These results suggest that changes in the colorectal environment during CRC development and progression may facilitate the growth of similar species across the two populations, potentially leading to the reduced microbial diversity observed in the CRC patients, in line with earlier observations by others (J. Ahn et al., Human gut microbiome and risk for colorectal cancer. Journal of the National Cancer Institute 105, 1907 (Dec 18, 2013)). The CRC index using 20 gene markers discovered in Chinese cohort I marginally differentiated the Danish patient microbiomes from the control ones (Wilcoxon rank-sum test, $P = 0.029$) and exhibited moderate classification potential (area under ROC curve 0.71, **Fig.14-1).** Only four out of the 20 genes (two from *P. anaerobius* and one each from *P. micra* and *F. nucleatum*) were associated with CRC status in the Danish cohort III (Wilcoxon rank-sum test, $q \leq 0.06$; all CRC-enriched; see Table 19). Among the factors the inventors had recorded, only CRC status could explain the variation in these four genes (PERMANOVA $P \leq 0.0001$; see Table 20), suggesting that these signatures are CRC-specific. CRC index using these four genes could classify CRC patients accurately with area under ROC curve of 0.85 **(Fig.14-2,** Table 21). At the cutoff -16.68, true positive rate (TPR) was 0.75, and false positive rate (FPR) was 0.08333. This higher AUC validated that the 4 gene markers could be used to classify CRC individuals. Two of the four genes were transposases from *Peptostreptococcus anaerobius.* The third gene (m1704941, butyryl-CoA dehydrogenase from *F. nucleatum*) was incidentally among the two genes successfully validated using qPCR in Chinese cohort II. The fourth gene from *P. micra* was the highly conserved *rpoB* gene (namely m1696299 (SEQ ID NO: 13, with identity of 99.78%) encoding RNA polymerase subunit $\beta$, often used as a phylogenetic marker (F. D. Ciccarelli et al., Toward automatic reconstruction of a highly resolved tree of life. Science 311, 1283 (Mar 3, 2006)).

For each sample, DNA was extracted and a DNA library was constructed followed by high throughput sequencing as described in Example 1. The inventors calculated the gene abundance profile for these samples using the same method as described in Qin et al. 2012, *supra.* Then the gene relative abundance of each of the markers as set forth in SEQ ID NOs: 1, SEQ ID NO: 9, SEQ ID NO: 13 and SEQ ID NO:16 was determined. Then the index of each sample was calculated by the formula below:

$$I_j = \left[\frac{\sum_{i\in N} \log_{10}\left(A_{ij} + 10^{-20}\right)}{|N|}\right]$$

$A_{ij}$ is the relative abundance of marker *i* in sample *j,* wherein i refers to each of the gene markers as set forth in said gene marker set;.

*N* is a subset of all CRC-enriched (case) markers in these 4 selected gut metagenomic markers (namely, a subset of all patient-enriched markers in selected biomarkers related to the abnormal condition).

wherein the subset of CRC-enriched markers are the marker as set forth in SEQ ID NOs: 1, SEQ ID NO: 9, SEQ ID NO: 13 and SEQ ID NO: 16;

$|N|$ is the sizes (number) of the biomarkers in the subset, wherein $|N|$ is 4.

wherein an index greater than a cutoff indicates that the subject has or is at the risk of developing colorectal cancer.

Table 21. 40 samples' gene relative abundance and calculated CRC index

| sample ID | Sample Type (control: non-CRC controls; case:CRC patients) | 1696299 (SEQ ID NO: 13) | 1704941 (SEQ ID NO: 16) | 2361423 (SEQ ID NO:1) | 3173495 (SEQ ID NO:9) | CRC index |
|---|---|---|---|---|---|---|
| MG-N-9 | control | 0 | 0 | 0 | 0 | -20 |
| MG-N-7 | control | 0 | 0 | 0 | 0 | -20 |
| MG-N-37 | control | 0 | 0 | 0 | 0 | -20 |
| MG-N-34 | control | 0 | 0 | 0 | 0 | -20 |

(continued)

| sample ID | Sample Type (control: non-CRC controls; case:CRC patients) | 1696299 (SEQ ID NO: 13) | 1704941 (SEQ ID NO: 16) | 2361423 (SEQ ID NO:1) | 3173495 (SEQ ID NO:9) | CRC index |
|---|---|---|---|---|---|---|
| MG-N-32 | control | 0 | 0 | 0 | 0 | -20 |
| MG-N-30 | control | 0 | 0 | 0 | 0 | -20 |
| MG-N-25 | control | 0 | 0 | 0 | 0 | -20 |
| MG-N-24 | control | 0 | 0 | 4.80E-08 | 0 | -16.82968969 |
| MG-N-50 | control | 0 | 0 | 4.80E-08 | 0 | -16.82968969 |
| MG-N-49 | control | 0 | 0 | 0 | 0 | -20 |
| MG-N-45 | control | 0 | 0 | 0 | 0 | -20 |
| MG-N-43 | control | 4.62E-08 | 0 | 0 | 0 | -16.83383951 |
| MG-N-42 | control | 0 | 0 | 0 | 0 | -20 |
| MG-N-41 | control | 0 | 0 | 0 | 0 | -20 |
| MG-N-40 | control | 0 | 0 | 0 | 0 | -20 |
| MG-N-38 | control | 0 | 0 | 4.56E-08 | 0 | -16.83525879 |
| MG-N-31 | control | 0 | 0 | 0 | 0 | -20 |
| MG-N-29 | control | 0 | 0 | 0 | 0 | -20 |
| MG-N-28 | control | 0 | 0 | 0 | 0 | -20 |
| MG-N-27 | control | 0 | 0 | 4.80E-08 | 9.07E-08 | -13.59028787 |
| MG-N-26 | control | 0 | 0 | 0 | 0 | -20 |
| MG-N-21 | control | 0 | 0 | 0 | 0 | -20 |
| MG-N-16 | control | 0 | 1.40E-06 | 0 | 0 | -16.46346799 |
| MG-N-12 | control | 0 | 0 | 0 | 0 | -20 |
| MG-C-48 | case | 4.49E-08 | 0 | 5.11E-07 | 5.80E-07 | -9.968976191 |

(continued)

| sample ID | Sample Type (control: non-CRC controls; case:CRC patients) | 1696299 (SEQ ID NO: 13) | 1704941 (SEQ ID NO: 16) | 2361423 (SEQ ID NO:1) | 3173495 (SEQ ID NO:9) | CRC index |
|---|---|---|---|---|---|---|
| MG-C-47 | case | 0 | 0 | 2.80E-07 | 1.51E-07 | -13.34346626 |
| MG-C-46 | case | 3.23E-08 | 4.37E-08 | 0 | 0 | -13.71257901 |
| MG-C-44 | case | 3.87E-08 | 0 | 1.28E-06 | 8.32E-07 | -9.846238935 |
| MG-C-39 | case | 7.47E-07 | 5.85E-07 | 1.56E-05 | 1.17E-05 | -5.524553268 |
| MG-C-19 | case | 0 | 0 | 4.36E-08 | 0 | -16.84012838 |
| MG-C-35 | case | 0 | 0 | 0 | 0 | -20 |
| MG-C-33 | case | 0 | 0 | 2.55E-07 | 0 | -16.64836495 |
| MG-C-23 | case | 1. 13E-07 | 5.09E-08 | 3.82E-06 | 3.48E-06 | -6.279140292 |
| MG-C-22 | case | 0 | 1.85E-07 | 0 | 0 | -16.68320707 |
| MG-C-20 | case | 0 | 0 | 0 | 0 | -20 |
| MG-C-18 | case | 3.87E-08 | 0 | 1.32E-07 | 2.50E-07 | -10.22344377 |
| MG-C-17 | case | 0 | 0 | 0 | 0 | -20 |
| MG-C-15 | case | 3.14E-08 | 8.49E-08 | 1.07E-07 | 6.75E-08 | -7.178868778 |
| MG-C-14 | case | 9.10E-06 | 0 | 1.08E-05 | 1.22E-05 | -8.730293755 |
| MG-C-13 | case | 2.68E-07 | 5.89E-07 | 8.78E-07 | 1.15E-06 | -6.199389389 |

The inventors decided to evaluate the diagnostic potential of the two non-transposase genes in the independent Chinese cohort II using qPCR. As these were originally discovered in Chinese cohort I and validated in Danish cohort III, cohort II serves as a suitable independent validation cohort of these genes, notably in a different platform. The inventors performed additional qPCR measurements of *rpoB* from *P. micra,* which showed a significant enrichment in CRC patient microbiomes in cohort II (Wilcoxon rank-sum test, stratified by colonoscopy, $P = 8.97 \times 10^{-8}$). Mantel-Haenszel odds ratio adjusted for colonoscopy was 20.17 (95% confidence interval 4.59-88.6, $P = 3.36 \times 10^{-7}$). Combined qPCR measurements (primers in Table 22) of the two genes (1696299 (SEQ ID NO:13) and 1704941(SEQ ID NO:16)) clearly separated case from control samples in Chinese cohort II (Wilcoxon rank-sum test stratified by colonoscopy, $P = 1.404 \times 10^{-8}$, **Fig.15a).** Their combined abundance accurately classified CRC samples in Chinese cohort II with an improved area under the ROC curve of 0.84 (cutoff -13.38, true-positive rate=0.723, false-positive rate=0.073; **Fig.15b,** Table 23), validating that the 2 gene markers could be used to classify CRC individuals. The accuracy was slightly better than that in a recent study (AUC=0.836, true-positive rate=0.58, false-positive rate=0.08), even though they used a combination of abundances of 22 species using metagenomic sequencing (G. Zeller et al., Potential of fecal microbiota for early-stage detection of colorectal cancer. Molecular systems biology 10, 766 (2014)). The Mantel-Haenszel odds ratio (adjusted for colonoscopy) for detecting at least one of the two markers by qPCR in CRC patients was 22.99 (*P*

$=5.79x10^{-8}$, 95% confidence interval 5.83-90.8). When stratifying the cohort into early stage (stages I-II) and late stage (stages III-IV) cancer patients, the classification potential and the odds ratio were still significant (see Table 24). Abundance of these two genes was significantly higher compared to control samples starting from stage II of CRC **(Fig.15c-d),** agreeing with the results from species abundances, and providing proof-of-principle that fecal metagenomes may harbor non-invasive biomarkers for the identification of early stage CRC.

For each sample, DNA was extracted as described in Example 1. The inventors performed qPCR as described above. Then the gene relative abundance of each of the markers as set forth in SEQ ID NO: 13 and SEQ ID NO:16 was determined. Then the index of each sample was calculated by the formula below:

$$I_j = \left[\frac{\sum_{i \in N} \log_{10}(A_{ij} + 10^{-20})}{|N|}\right]$$

$A_{ij}$ is the relative abundance of marker *i* in sample *j,* wherein i refers to each of the gene markers as set forth in said gene marker set;.

*N* is a subset of all CRC-enriched (case) markers in these 2 selected gut metagenomic markers (namely, a subset of all patient-enriched markers in selected biomarkers related to the abnormal condition).

wherein the subset of CRC-enriched markers are the marker as set forth in SEQ ID NO: 13 and SEQ ID NO:16;

|*N*| is the sizes (number) of the biomarkers in the subset, wherein |*N*| is 2.

wherein an index greater than a cutoff indicates that the subject has or is at the risk of developing colorectal cancer.

The inventors also used one of the two gene markers to calculate CRC index respectively (Table 23). The area under the ROC curve was 0.80 only using 1696299 (cutoff -6.762, true-positive rate=0.6383, false-positive rate=0.05505, **Fig.16**) and the area under the ROC curve was 0.69 only using 1704941 **(Fig.17).** The results showed that gene marker 1696299 (SEQ ID NO: 13) was the robust biomarkers, which also could be used to classify CRC individuals uniquely.

The inventors have demonstrated, for the first time, the potential for CRC diagnosis through affordable targeted detection methods for microbial biomarkers in fecal samples. Two recent studies reported on potential CRC diagnosis using metagenomic sequencing of the fecal microbiome, with the same accuracy as ours (in terms of area under the receiver-operating curve). While the 16S ribosomal RNA gene based study used 5 operational taxonomic units to classify CRC from healthy samples in a cohort notably without any cross-validation (J. P. Zackular, M. A. Rogers, M. T. t. Ruffin, P. D. Schloss, The human gut microbiome as a screening tool for colorectal cancer. Cancer prevention research 7, 1112 (Nov, 2014)), the metagenomic shotgun study used 22 species-level taxonomic units to accurately classify CRC patients notably in an independent cohort (G. Zeller et al., Potential of fecal microbiota for early-stage detection of colorectal cancer. Molecular systems biology 10, 766 (2014)). The inventors have shown that using just two gene markers, discovered in 128 Chinese individuals and validated in 40 Danish individuals, the inventors could accurately classify CRC patients from control individuals in an independent qPCR validation cohort of 156 Chinese individuals. The significant improvement in the classification potential (from AUC=0.73 to AUC=0.84) by using a gene (*rpoB* gene from *P. micra*) validated in the Danish cohort reiterates the importance of validating newly discovered biomarkers in independent cohorts with different genetic and environmental background.

Table 22. Sequence Information for the primers and probes for the selected 2 gene markers

| | | |
|---|---|---|
| >1696299 | Forward | AAGAATGGAGAGAGTTGTTAGAGAAAGAA |
| | Reverse | TTGTGATAATTGTGAAGAACCGAAGA |
| | Probe | AACTCAAGATCCAGACCTTGCTACGCCTCA |
| >1704941 | Forward | TTGTAAGTGCTGGTAAAGGGATTG |
| | Reverse | CATTCCTACATAACGGTCAAGAGGTA |
| | Probe | AGCTTCTATTGGTTCTTCTCGTCCAGTGGC |

Table 23 156 samples' qPCR gene relative abundance and calculated CRC index

| sample ID | Sample Type (control:non-CRC controls; case:CRC patients) | 1696299 (SEQ ID NO: 13) | 1704941 (SEQ ID NO: 16) | CRC index (using1696299 and 1704941) | CRC index (only using 1696299) | CRC index (only using 1704941) |
|---|---|---|---|---|---|---|
| M2-PK537 | control | 0 | 0 | -20 | -20 | -20 |
| M2-PK528 | control | 0 | 0 | -20 | -20 | -20 |
| M2-PK505 | control | 0 | 0 | -20 | -20 | -20 |
| M2-PK526 | control | 0 | 0 | -20 | -20 | -20 |
| M2-PK507 | control | 0 | 0 | -20 | -20 | -20 |
| M2-PK501 | control | 0 | 0 | -20 | -20 | -20 |
| M2-PK510 | control | 0 | 0 | -20 | -20 | -20 |
| M2-PK518 | control | 0.000211515 | 0 | -11.83732941 | -3.674658828 | -20 |
| A001 | control | 0 | 0 | -20 | -20 | -20 |
| A004 | control | 0 | 0 | -20 | -20 | -20 |
| A006 | control | 0 | 0 | -20 | -20 | -20 |
| A007 | control | 0 | 0 | -20 | -20 | -20 |
| A036 | control | 0 | 0 | -20 | -20 | -20 |
| A037 | control | 0 | 0 | -20 | -20 | -20 |
| A041 | control | 0 | 0 | -20 | -20 | -20 |
| A047 | control | 0 | 0 | -20 | -20 | -20 |
| A050 | control | 0 | 0 | -20 | -20 | -20 |
| A059 | control | 0 | 0 | -20 | -20 | -20 |
| A073 | control | 0 | 0 | -20 | -20 | -20 |
| A081 | control | 0 | 0 | -20 | -20 | -20 |
| A083 | control | 0 | 0 | -20 | -20 | -20 |
| A084 | control | 0 | 0 | -20 | -20 | -20 |
| A089 | control | 0 | 0 | -20 | -20 | -20 |
| M2-PK_ 685 | control | 0 | 0 | -20 | -20 | -20 |
| M2-PK_ 755 | control | 0 | 0 | -20 | -20 | -20 |

(continued)

| sample ID | Sample Type (control:non-CRC controls; case:CRC patients) | 1696299 (SEQ ID NO: 13) | 1704941 (SEQ ID NO: 16) | CRC index (using1696299 and 1704941) | CRC index (only using 1696299) | CRC index (only using 1704941) |
|---|---|---|---|---|---|---|
| M2-PK_767 | control | 0 | 0 | -20 | -20 | -20 |
| M2-PK_782 | control | 0 | 0 | -20 | -20 | -20 |
| M2-PK_815 | control | 0 | 0 | -20 | -20 | -20 |
| M2-PK_863 | control | 0 | 0 | -20 | -20 | -20 |
| M2-PK_870 | control | 0 | 0 | -20 | -20 | -20 |
| M2-PK_884 | control | 0 | 0 | -20 | -20 | -20 |
| M2-PK_885 | control | 0 | 0 | -20 | -20 | -20 |
| M2-PK_891 | control | 0 | 0 | -20 | -20 | -20 |
| M2-PK_896 | control | 0 | 0 | -20 | -20 | -20 |
| M2-PK_897 | control | 0 | 0 | -20 | -20 | -20 |
| M2-PK_908 | control | 0 | 0 | -20 | -20 | -20 |
| M2-PK_911 | control | 0 | 0 | -20 | -20 | -20 |
| M2-PK_923 | control | 0 | 0 | -20 | -20 | -20 |
| M2-PK_925 | control | 0 | 0 | -20 | -20 | -20 |

(continued)

| sample ID | Sample Type (control:non-CRC controls; case:CRC patients) | 1696299 (SEQ ID NO: 13) | 1704941 (SEQ ID NO: 16) | CRC index (using1696299 and 1704941) | CRC index (only using 1696299) | CRC index (only using 1704941) |
|---|---|---|---|---|---|---|
| M2-PK_926 | control | 0 | 0 | -20 | -20 | -20 |
| M2-PK_931 | control | 0 | 0 | -20 | -20 | -20 |
| M2-PK_937 | control | 0 | 0 | -20 | -20 | -20 |
| M2-PK_939 | control | 0 | 0 | -20 | -20 | -20 |
| M2-PK_940 | control | 0 | 0 | -20 | -20 | -20 |
| M2-PK_947 | control | 0 | 0 | -20 | -20 | -20 |
| M2-PK_948 | control | 0 | 0 | -20 | -20 | -20 |
| M2-PK_952 | control | 0 | 0 | -20 | -20 | -20 |
| M2-PK_954 | control | 0 | 0 | -20 | -20 | -20 |
| M2-PK_956 | control | 0 | 0 | -20 | -20 | -20 |
| M2-PK_960 | control | 1.03E-05 | 0 | -12.49358139 | -4.987162775 | -20 |
| M2-PK_989 | control | 0 | 0 | -20 | -20 | -20 |
| M2-PK_1007 | control | 0 | 0 | -20 | -20 | -20 |
| M2-PK_1010 | control | 0 | 0 | -20 | -20 | -20 |

(continued)

| sample ID | Sample Type (control:non-CRC controls; case:CRC patients) | 1696299 (SEQ ID NO: 13) | 1704941 (SEQ ID NO: 16) | CRC index (using1696299 and 1704941) | CRC index (only using 1696299) | CRC index (only using 1704941) |
|---|---|---|---|---|---|---|
| M2-PK_1016 | control | 0 | 0 | -20 | -20 | -20 |
| M2-PK_1025 | control | 0 | 0 | -20 | -20 | -20 |
| M2-PK_1028 | control | 0 | 0 | -20 | -20 | -20 |
| M2-PK_1029 | control | 0 | 0 | -20 | -20 | -20 |
| M2-PK_1030 | control | 0 | 0 | -20 | -20 | -20 |
| M2-PK_1032 | control | 0 | 0 | -20 | -20 | -20 |
| M2-PK_1033 | control | 0 | 2.10E-05 | -12.33889035 | -20 | -4.677780705 |
| M2-PK_1037 | control | 0 | 0 | -20 | -20 | -20 |
| M2-PK_1038 | control | 0 | 0 | -20 | -20 | -20 |
| M2-PK_1041 | control | 0.00179637 | 0.000250648 | -3.173269981 | -2.745604206 | -3.600935756 |
| M2-PK_1044 | control | 0 | 0 | -20 | -20 | -20 |
| M2-PK_1047 | control | 0 | 0 | -20 | -20 | -20 |
| M2-PK_1058 | control | 0 | 0 | -20 | -20 | -20 |
| M2-PK_1062 | control | 0 | 0 | -20 | -20 | -20 |

(continued)

| sample ID | Sample Type (control:non-CRC controls; case:CRC patients) | 1696299 (SEQ ID NO: 13) | 1704941 (SEQ ID NO: 16) | CRC index (using1696299 and 1704941) | CRC index (only using 1696299) | CRC index (only using 1704941) |
|---|---|---|---|---|---|---|
| M2-PK_1065 | control | 0 | 0 | -20 | -20 | -20 |
| M2-PK_1068 | control | 0 | 0 | -20 | -20 | -20 |
| M2-PK_1070 | control | 0 | 0 | -20 | -20 | -20 |
| M2-PK_1074 | control | 0 | 0 | -20 | -20 | -20 |
| M2-PK_1079 | control | 0.000315363 | 0 | -11.75059463 | -3.501189262 | -20 |
| M2-PK_1081 | control | 0 | 0 | -20 | -20 | -20 |
| M2-PK_1084 | control | 0 | 0 | -20 | -20 | -20 |
| M2-PK_1088 | control | 0 | 0 | -20 | -20 | -20 |
| M2-PK_1092 | control | 0 | 0 | -20 | -20 | -20 |
| M2-PK_1101 | control | 1.88E-06 | 0 | -12.86292108 | -5.725842151 | -20 |
| M2-PK_1110 | control | 0 | 0 | -20 | -20 | -20 |
| M2-PK_1113 | control | 0 | 0 | -20 | -20 | -20 |
| M2-PK_1116 | control | 0 | 0 | -20 | -20 | -20 |
| M2-PK_1119 | control | 0 | 0 | -20 | -20 | -20 |

(continued)

| sample ID | Sample Type (control:non-CRC controls; case:CRC patients) | 1696299 (SEQ ID NO: 13) | 1704941 (SEQ ID NO: 16) | CRC index (using1696299 and 1704941) | CRC index (only using 1696299) | CRC index (only using 1704941) |
|---|---|---|---|---|---|---|
| M2-PK_ 1135 | control | 0.000177345 | 1.02E-06 | -4.87129044 | -3.751181051 | -5.991399828 |
| M2-PK_ 1145 | control | 0 | 0 | -20 | -20 | -20 |
| M2-PK_ 1150 | control | 0 | 0 | -20 | -20 | -20 |
| M2-PK_ 1231 | control | 0 | 0 | -20 | -20 | -20 |
| M2-PK_ 1243 | control | 0 | 0 | -20 | -20 | -20 |
| M2-PK_ 1244 | control | 0 | 0 | -20 | -20 | -20 |
| M2-PK_ 1245 | control | 0 | 0 | -20 | -20 | -20 |
| M2-PK_ 1255 | control | 0 | 0 | -20 | -20 | -20 |
| M2-PK_ 1271 | control | 0 | 0 | -20 | -20 | -20 |
| M2-PK_ 1301 | control | 0 | 0 | -20 | -20 | -20 |
| M2-PK_ 1305 | control | 0 | 0 | -20 | -20 | -20 |
| M2-PK_ 1312 | control | 0 | 0 | -20 | -20 | -20 |
| M2-PK_ 1318 | control | 0 | 0 | -20 | -20 | -20 |
| M2-PK_ 1337 | control | 0 | 0 | -20 | -20 | -20 |

(continued)

| sample ID | Sample Type (control:non-CRC controls; case:CRC patients) | 1696299 (SEQ ID NO: 13) | 1704941 (SEQ ID NO: 16) | CRC index (using1696299 and 1704941) | CRC index (only using 1696299) | CRC index (only using 1704941) |
|---|---|---|---|---|---|---|
| M2-PK_ 1347 | control | 0 | 0 | -20 | -20 | -20 |
| M2-PK_ 1361 | control | 0 | 0 | -20 | -20 | -20 |
| M2-PK_ 1366 | control | 0 | 0 | -20 | -20 | -20 |
| M2-PK_ 1568 | control | 0 | 0 | -20 | -20 | -20 |
| M2-PK_ 1369 | control | 0 | 0 | -20 | -20 | -20 |
| M2-PK_ 1376 | control | 0 | 0 | -20 | -20 | -20 |
| M2-PK_ 1383 | control | 0 | 0.000689816 | -11.58063337 | -20 | -3.161266737 |
| M2-PK_ 1385 | control | 0 | 0 | -20 | -20 | -20 |
| M2-PK_ 1390 | control | 0 | 0 | -20 | -20 | -20 |
| M2-PK_ 1412 | control | 0 | 0 | -20 | -20 | -20 |
| M2-PK_ 1417 | control | 0 | 0 | -20 | -20 | -20 |
| M2-PK_ 1423 | control | 0 | 0 | -20 | -20 | -20 |
| M2-PK_ 1443 | control | 0 | 0 | -20 | -20 | -20 |
| M2-PK_ 1452 | control | 0 | 0 | -20 | -20 | -20 |
| A009 | case | 0.006203293 | 0 | -11.10368885 | -2.207377705 | -20 |

(continued)

| sample ID | Sample Type (control:non-CRC controls; case:CRC patients) | 1696299 (SEQ ID NO: 13) | 1704941 (SEQ ID NO: 16) | CRC index (using1696299 and 1704941) | CRC index (only using 1696299) | CRC index (only using 1704941) |
|---|---|---|---|---|---|---|
| A017 | case | 0.002625577 | 0.087144293 | -1.820268145 | -2.580775241 | -1.059761049 |
| A019 | case | 0 | 0.005819658 | -11.11755127 | -20 | -2.235102536 |
| A020 | case | 0.001675491 | 0 | -11.38792895 | -2.775857901 | -20 |
| A071 | case | 0 | 0 | -20 | -20 | -20 |
| A080 | case | 1.60E-06 | 0 | -12.89794001 | -5.795880017 | -20 |
| A094 | case | 1.73E-07 | 0 | -13.38097695 | -6.761953897 | -20 |
| A096 | case | 0.000316655 | 0 | -11.74970683 | -3.49941365 | -20 |
| A100 | case | 0 | 0 | -20 | -20 | -20 |
| A103 | case | 0.049138581 | 0 | -10.65428869 | -1.308577389 | -20 |
| A104 | case | 0.009579061 | 0 | -11.00933853 | -2.018677061 | -20 |
| M2-PK_86 | case | 0.000802784 | 0 | -11.54770065 | -3.095401292 | -20 |
| M2-PK_89 | case | 0 | 0 | -20 | -20 | -20 |
| M2-PK_93 | case | 0.008910363 | 8.53E-05 | -3.059577786 | -2.050104603 | -4.069050969 |
| M2-PK_95 | case | 0.044982261 | 5.55E-05 | -2.801332868 | -1.346958719 | -4.255707017 |
| M2-PK_97 | case | 0.066693964 | 2.89E-05 | -2.857507813 | -1.175913469 | -4.539102157 |
| M2-PK_98 | case | 0.063669666 | 0.006985843 | -1.675924304 | -1.196067428 | -2.155781179 |
| M2-PK_99 | case | 0 | 0 | -20 | -20 | -20 |
| M2-PK_101 | case | 0 | 0 | -20 | -20 | -20 |
| M2-PK_102 | case | 0 | 0 | -20 | -20 | -20 |
| M2-PK_103 | case | 9.80E-06 | 9.72E-05 | -4.51055383 | -5.008773924 | -4.012333735 |
| M2-PK_104 | case | 0.01946802 | 0.002622757 | -2.145960081 | -1.710678216 | -2.581241945 |
| M2-PK_105 | case | 0.322093176 | 0.001461738 | -1.66357447 | -0.492018476 | -2.835130463 |

(continued)

| sample ID | Sample Type (control:non-CRC controls; case:CRC patients) | 1696299 (SEQ ID NO: 13) | 1704941 (SEQ ID NO: 16) | CRC index (using1696299 and 1704941) | CRC index (only using 1696299) | CRC index (only using 1704941) |
|---|---|---|---|---|---|---|
| M2-PK_109 | case | 1.61E-05 | 0 | -12.39658706 | -4.793174124 | -20 |
| M2-PK_112 | case | 0 | 7.85E-05 | -12.05256517 | -20 | -4.105130343 |
| M2-PK_114 | case | 0.003779209 | 0 | -11.21129954 | -2.42259909 | -20 |
| M2-PK_115 | case | 0.009892837 | 0.000711697 | -2.576192006 | -2.004679147 | -3.147704865 |
| M2-PK_116 | case | 0.011960736 | 0.000418046 | -2.650509012 | -1.922242095 | -3.378775928 |
| M2-PK_119 | case | 0.007125466 | 0.023680609 | -1.88639693 | -2.147186728 | -1.625607133 |
| M2-PK_121 | case | 0 | 0 | -20 | -20 | -20 |
| M2-PK_122 | case | 0.000169321 | 0 | -11.88564459 | -3.771289175 | -20 |
| M2-PK_126 | case | 0 | 0.0005857 | -11.61616239 | -20 | -3.232324776 |
| M2-PK_127 | case | 0.012612517 | 6.71E-05 | -3.036237858 | -1.899198235 | -4.17327748 |
| M2-PK_130 | case | 0 | 5.37E-05 | -12.13501286 | -20 | -4.270025714 |
| M2-PK_199 | case | 0.000296681 | 0.002785496 | -3.041403863 | -3.527710266 | -2.555097461 |
| M2-PK_200 | case | 0.014980712 | 0.000248796 | -2.714312076 | -1.824467545 | -3.604156606 |
| M2-PK_202 | case | 0 | 0 | -20 | -20 | -20 |

(continued)

| sample ID | Sample Type (control:non-CRC controls; case:CRC patients) | 1696299 (SEQ ID NO: 13) | 1704941 (SEQ ID NO: 16) | CRC index (using1696299 and 1704941) | CRC index (only using 1696299) | CRC index (only using 1704941) |
|---|---|---|---|---|---|---|
| M2-PK_203 | case | 0 | 0 | -20 | -20 | -20 |
| M2-PK_743 | case | 0 | 0 | -20 | -20 | -20 |
| M2-PK_1378 | case | 0 | 0 | -20 | -20 | -20 |
| MSC_38 | case | 0 | 0 | -20 | -20 | -20 |
| MSC_83 | case | 0.013275868 | 4.92E-06 | -3.592485986 | -1.876937074 | -5.308034897 |
| MSC_86 | case | 0.002163301 | 0 | -11.33244152 | -2.664883049 | -20 |
| MSC_87 | case | 2.18E-05 | 0 | -12.33077175 | -4.661543506 | -20 |
| MSC_90 | case | 9.22E-05 | 0 | -12.01763454 | -4.035269079 | -20 |
| MSC_91 | case | 9.01E-07 | 0 | -13.0226376 | -6.045275209 | -20 |
| MSC_97 | case | 0 | 0 | -20 | -20 | -20 |

Table 3 IMG, mOTU and MLG species associated with CRC with q-value < 0.05. 85 MLG species were formed after grouping 106 MLGs with more than 100 genes using species annotation when available.

| **28 IMG species** | | | | | |
|---|---|---|---|---|---|
| | **Control rank mean** | **Case rank mean** | **Enrichment (0:case/ 1:control)** | **P-value** | **q-value** |
| *Peptostreptococcus stomatis* | 37.25926 | 84.37838 | 0 | 5.11E-12 | 1.32E-08 |
| *Parvimonas micra* | 38.43519 | 83.52027 | 0 | 4.21E-11 | 5.43E-08 |
| *Parvimonas sp. oral taxon 393* | 39.81481 | 82.51351 | 0 | 2.79E-10 | 2.40E-07 |
| *Parvimonas sp. oral taxon 110* | 43.52778 | 79.80405 | 0 | 6.17E-08 | 3.98E-05 |
| *Gemella morbillorum* | 43.87037 | 79.55405 | 0 | 1.53E-07 | 7.88E-05 |
| *Fusobacterium nucleatum* | 45.09259 | 78.66216 | 0 | 3.86E-07 | 1.56E-04 |
| *Leptotrichia buccalis* | 45.60185 | 78.29054 | 0 | 4.44E-07 | 1.56E-04 |
| *Fusobacterium sp. oral taxon 370* | 45.02778 | 78.70946 | 0 | 4.83E-07 | 1.56E-04 |
| *Burkholderia mallei* | 45.19444 | 78.58784 | 0 | 7.93E-07 | 2.27E-04 |

(continued)

| 28 IMG species | | | | | |
|---|---|---|---|---|---|
| | **Control rank mean** | **Case rank mean** | **Enrichment (0:case/ 1:control)** | **P-value** | **q-value** |
| *Prevotella intermedia* | 46.47222 | 77.65541 | 0 | 1.92E-06 | 4.95E-04 |
| *Streptococcus pseudoporcinus* | 47.5 | 76.90541 | 0 | 4.03E-06 | 8.99E-04 |
| *Streptococcus dysgalactiae* | 47.06481 | 77 .22297 | 0 | 4.18E-06 | 8.99E-04 |
| *Beggiatoa sp. PS* | 46.53704 | 77.60811 | 0 | 5.03E-06 | 9.97E-04 |
| *Malassezia globosa* | 46.35185 | 77.74324 | 0 | 8.71E-06 | 1.60E-03 |
| *Paracoccus denitrificans* | 47.48148 | 76.91892 | 0 | 1.18E-05 | 2.02E-03 |
| *Eubacterium ventriosum* | 80.98148 | 52.47297 | 1 | 1.27E-05 | 2.05E-03 |
| *Streptococcus constellatus* | 48.2037 | 76.39189 | 0 | 1.66E-05 | 2.52E-03 |
| *Filifactor alocis* | 49.06481 | 75.76351 | 0 | 3.94E-05 | 5.65E-03 |
| *Peptoniphilus indolicus* | 51.2963 | 74.13514 | 0 | 4.53E-05 | 6.14E-03 |
| *Crenothrix polyspora* | 48.76852 | 75.97973 | 0 | 5.14E-05 | 6.63E-03 |
| *Peptostreptococcus anaerobius* | 50.14815 | 74.97297 | 0 | 5.88E-05 | 7.22E-03 |
| *Streptococcus equi* | 50.58333 | 74.65541 | 0 | 6.91E-05 | 8.10E-03 |
| *Solobacterium moorei* | 47.66667 | 76.78378 | 0 | 8.79E-05 | 9.85E-03 |
| *Sulfurovum sp. SCGC AAA036-O23* | 52.12037 | 73.53378 | 0 | 1.28E-04 | 1.37E-02 |
| *Streptobacillus moniliformis* | 52.35185 | 73.36486 | 0 | 1.44E-04 | 1.49E-02 |
| *Eubacteriaceae bacterium ACC19a* | 51.87037 | 73.71622 | 0 | 1.93E-04 | 1.92E-02 |
| *Fusobacterium necrophorum* | 52.37037 | 73.35135 | 0 | 3.72E-04 | 3.55E-02 |
| *Adhaeribacter aquaticus* | 77.06481 | 55.33108 | 1 | 4.79E-04 | 4.41E-02 |
| **21 mOTU species** | | | | | |
| | Control rank mean | Case rank mean | Enrichment (0:case/1: control) | P-value | q-value |
| *Parvimonas micra* | 46.2963 | 77.78378 | 0 | 2.31E-08 | 7.73E-06 |
| *Peptostreptococcus stomatis* | 46.25 | 77.81757 | 0 | 2.81E-08 | 7.73E-06 |
| motu_linkage_group_731 | 50.42593 | 74.77027 | 0 | 2.91E-07 | 5.33E-05 |
| *Gemella morbillorum* | 47.93519 | 76.58784 | 0 | 8.63E-07 | 1.18E-04 |
| motu_linkage_group_407 | 81.13889 | 52.35811 | 1 | 8.51E-06 | 9.34E-04 |
| motu_linkage_group_490 | 80.46296 | 52.85135 | 1 | 3.04E-05 | 2.78E-03 |
| *Fusobacterium nucleatum* | 54.62037 | 71.70946 | 0 | 3.56E-05 | 2.79E-03 |
| *Clostridium symbiosum* | 48.66667 | 76.05405 | 0 | 4.50E-05 | 2.99E-03 |
| motu_linkage_group_443 | 79.66667 | 53.43243 | 1 | 4.91E-05 | 2.99E-03 |
| motu_linkage_group_316 | 79.61111 | 53.47297 | 1 | 7.03E-05 | 3.86E-03 |
| *Eubacterium ventriosum* | 78.09259 | 54.58108 | 1 | 9.82E-05 | 4.90E-03 |

(continued)

| 21 mOTU species | | | | | |
|---|---|---|---|---|---|
| | Control rank mean | Case rank mean | Enrichment (0:case/1: control) | P-value | q-value |
| *Solobacterium moorei* | 51.22222 | 74.18919 | 0 | 2.49E-04 | 1.14E-02 |
| *Bacteroides fragilis* | 51.09259 | 74.28378 | 0 | 3.75E-04 | 1.58E-02 |
| *unclassified Fusobacterium* | 54.22222 | 72 | 0 | 4.20E-04 | 1.59E-02 |
| *Clostridiales bacterium 1_7_47FAA* | 51.27778 | 74.14865 | 0 | 4.34E-04 | 1.59E-02 |
| *Clostridium ramosum* | 50.92593 | 74.40541 | 0 | 5.21E-04 | 1.75E-02 |
| motu_linkage_group_611 | 77.2963 | 55.16216 | 1 | 5.50E-04 | 1.75E-02 |
| *Prevotella nigrescens* | 58.09259 | 69.17568 | 0 | 5.72E-04 | 1.75E-02 |
| motu_linkage_group_624 | 51.01852 | 74.33784 | 0 | 1.33E-03 | 3.69E-02 |
| motu_linkage_group_510 | 77.84259 | 54.76351 | 1 | 1.35E-03 | 3.69E-02 |
| *Clostridium bolteae* | 51.81481 | 73.75676 | 0 | 1.41E-03 | 3.69E-02 |
| **85 MLG species** | | | | | |
| | Control rank mean | Case rank mean | Enrichment (0:case/1: control) | P-value | q-value |
| *Parvimonas micra* | 38.40741 | 83.54054 | 0 | 5.56E-12 | 4.84E-10 |
| *Fusobacterium nucleatum* | 40.32407 | 82.14189 | 0 | 1.72E-10 | 7.48E-09 |
| *Solobacterium moorei* | 42.2037 | 80.77027 | 0 | 4.01E-08 | 1.16E-06 |
| *Clostridium symbiosum* | 46.31481 | 77.77027 | 0 | 2.67E-06 | 5.80E-05 |
| Con 10180 | 82.03704 | 51.7027 | 1 | 6.06E-06 | 1.05E-04 |
| CRC 2881 | 51.25926 | 74.16216 | 0 | 7.57E-06 | 1.10E-04 |
| CRC 2794 | 51.03704 | 74.32432 | 0 | 1.04E-05 | 1.30E-04 |
| *Coprococcus sp. ART55/1* | 80.85185 | 52.56757 | 1 | 2.09E-05 | 2.05E-04 |
| *Clostridium hathewayi* | 46.77778 | 77.43243 | 0 | 2.12E-05 | 2.05E-04 |
| *Clostridiales bacterium 1_7_47FAA* | 48.16667 | 76.41892 | 0 | 2.49E-05 | 2.17E-04 |
| CRC 4136 | 50.99074 | 74.35811 | 0 | 2.97E-05 | 2.32E-04 |
| *butyrate-producing bacterium SS3/4* | 80.57407 | 52.77027 | 1 | 3.19E-05 | 2.32E-04 |
| *Haemophilus parainfluenzae* | 80.49074 | 52.83108 | 1 | 4.18E-05 | 2.69E-04 |
| Con 154 | 80.35185 | 52.93243 | 1 | 4.45E-05 | 2.69E-04 |
| *Clostridium clostridioforme* | 50.2037 | 74.93243 | 0 | 4.64E-05 | 2.69E-04 |
| *Bacteroides fragilis* | 49.09259 | 75.74324 | 0 | 5.56E-05 | 3.02E-04 |
| Con 1979 | 79.94444 | 53.22973 | 1 | 6.03E-05 | 3.09E-04 |
| *Eubacterium ventriosum* | 78.62963 | 54.18919 | 1 | 6.88E-05 | 3.33E-04 |
| Con 7958 | 75.27778 | 56.63514 | 1 | 7.40E-05 | 3.33E-04 |
| Con 5770 | 79.39815 | 53.62838 | 1 | 7.66E-05 | 3.33E-04 |
| *Clostridium sp. HGF2* | 48.27778 | 76.33784 | 0 | 8.28E-05 | 3.43E-04 |

(continued)

| 85 MLG species | | | | | |
|---|---|---|---|---|---|
| | Control rank mean | Case rank mean | Enrichment (0:case/1: control) | P-value | q-value |
| CRC 6481 | 52.09259 | 73.55405 | 0 | 9.87E-05 | 3.90E-04 |
| *Cloacibacillus evryensis* | 52.73148 | 73.08784 | 0 | 1.13E-04 | 4.23E-04 |
| Con 1987 | 79.42593 | 53.60811 | 1 | 1.17E-04 | 4.23E-04 |
| Con 4595 | 77.21296 | 55.22297 | 1 | 1.38E-04 | 4.81E-04 |
| Con 1617 | 76.12963 | 56.01351 | 1 | 1.50E-04 | 5.03E-04 |
| Con 1371 | 78.46296 | 54.31081 | 1 | 2.05E-04 | 6.60E-04 |
| *Lachnospiraceae bacterium 5_1_57FAA* | 49.96296 | 75.10811 | 0 | 2.49E-04 | 7.73E-04 |
| *Eubacterium biforme* | 74.68519 | 57.06757 | 1 | 3.00E-04 | 8.70E-04 |
| *Faecalibacterium prausnitzii* | 78.25926 | 54.45946 | 1 | 3.00E-04 | 8.70E-04 |
| Con 4699 | 78.78704 | 54.07432 | 1 | 3.13E-04 | 8.79E-04 |
| *Desulfovibrio sp. 6_1_46AFAA* | 53.33333 | 72.64865 | 0 | 3.70E-04 | 9.87E-04 |
| Con 1529 | 75.05556 | 56.7973 | 1 | 3.74E-04 | 9.87E-04 |
| *Ruminococcus torques* | 76.92593 | 55.43243 | 1 | 5.28E-04 | 1.35E-03 |
| *Coprobacillus sp. 3_3_56FAA* | 50.53704 | 74.68919 | 0 | 6.01E-04 | 1.46E-03 |
| *Streptococcus equinus* | 54.52778 | 71.77703 | 0 | 6.02E-04 | 1.46E-03 |
| *Synergistes sp. 3_1_syn1* | 54.37963 | 71.88514 | 0 | 6.89E-04 | 1.62E-03 |
| *Lachnospiraceae bacterium 8_1_57FAA* | 51.88889 | 73.7027 | 0 | 7.91E-04 | 1.81E-03 |
| *Klebsiella pneumoniae* | 74.7037 | 57.05405 | 1 | 8.33E-04 | 1.86E-03 |
| *Eubacterium eligens* | 79.53704 | 53.52703 | 1 | 9.07E-04 | 1.97E-03 |
| *Clostridium bolteae* | 51.39815 | 74.06081 | 0 | 9.27E-04 | 1.97E-03 |
| Con 1513 | 76.59259 | 55.67568 | 1 | 1.02E-03 | 2.11E-03 |
| *Clostridium citroniae* | 51.71296 | 73.83108 | 0 | 1.08E-03 | 2.19E-03 |
| *Fusobacterium varium* | 54.57407 | 71.74324 | 0 | 1.15E-03 | 2.28E-03 |
| *Bacteroides clarus* | 75.55556 | 56.43243 | 1 | 1.29E-03 | 2.50E-03 |
| *Ruminococcus obeum* | 77.53704 | 54.98649 | 1 | 1.34E-03 | 2.54E-03 |
| Con 2606 | 77.5 | 55.01351 | 1 | 1.42E-03 | 2.59E-03 |
| *Lachnospiraceae bacterium 3_1_46FAA* | 52.53704 | 73.22973 | 0 | 1.44E-03 | 2.59E-03 |
| CRC 2867 | 52.31481 | 73.39189 | 0 | 1.46E-03 | 2.59E-03 |
| Con 6037 | 77.5463 | 54.97973 | 1 | 1.56E-03 | 2.71E-03 |
| *Clostridium sp. L2-50* | 76.37963 | 55.83108 | 1 | 1.61E-03 | 2.75E-03 |
| Con 1867 | 76.38889 | 55.82432 | 1 | 2.13E-03 | 3.57E-03 |
| *Roseburia intestinalis* | 76.99074 | 55.38514 | 1 | 2.20E-03 | 3.58E-03 |
| *Subdoligranulum sp. 4_3_54A2FAA* | 51.56481 | 73.93919 | 0 | 2.24E-03 | 3.58E-03 |

(continued)

| 85 MLG species | | | | | |
|---|---|---|---|---|---|
| | Control rank mean | Case rank mean | Enrichment (0:case/1: control) | P-value | q-value |
| Con 1197 | 75.42593 | 56.52703 | 1 | 2.26E-03 | 3.58E-03 |
| CRC 4069 | 53.7963 | 72.31081 | 0 | 2.56E-03 | 3.96E-03 |
| Con 8757 | 77.17593 | 55.25 | 1 | 2.60E-03 | 3.96E-03 |
| Con 5752 | 73.65741 | 57.81757 | 1 | 2.71E-03 | 4.07E-03 |
| Con 4295 | 74.98148 | 56.85135 | 1 | 2.95E-03 | 4.34E-03 |
| *Eubacterium rectale* | 75.90741 | 56.17568 | 1 | 3.21E-03 | 4.60E-03 |
| Con 2494 | 74.35185 | 57.31081 | 1 | 3.22E-03 | 4.60E-03 |
| Con 7367 | 76.23148 | 55.93919 | 1 | 3.63E-03 | 5.09E-03 |
| Con 4829 | 76.7963 | 55.52703 | 1 | 3.88E-03 | 5.35E-03 |
| Con 356 | 75.94444 | 56.14865 | 1 | 3.95E-03 | 5.37E-03 |
| *Dorea_formicigenerans* | 52.98148 | 72.90541 | 0 | 4.36E-03 | 5.84E-03 |
| Con 10559 | 76.59259 | 55.67568 | 1 | 4.52E-03 | 5.91E-03 |
| Con 563 | 72.7037 | 58.51351 | 1 | 4.55E-03 | 5.91E-03 |
| Con 4909 | 75.72222 | 56.31081 | 1 | 4.79E-03 | 6.12E-03 |
| Con 6128 | 76.22222 | 55.94595 | 1 | 4.86E-03 | 6.13E-03 |
| Con 2503 | 74.14815 | 57.45946 | 1 | 6.02E-03 | 7.46E-03 |
| CRC 3579 | 54.05556 | 72.12162 | 0 | 6.09E-03 | 7.46E-03 |
| Con 2703 | 74.55556 | 57.16216 | 1 | 7.67E-03 | 9.15E-03 |
| Con 6068 | 75.74074 | 56.2973 | 1 | 7.67E-03 | 9.15E-03 |
| Con 1604 | 71.92593 | 59.08108 | 1 | 8.96E-03 | 1.05E-02 |
| Con 5615 | 76.07407 | 56.05405 | 1 | 9.70E-03 | 1.12E-02 |
| *Lachnospiraceae bacterium 3_1_57FAA_CT1* | 54.07407 | 72.10811 | 0 | 1.04E-02 | 1.19E-02 |
| Con 569 | 73.41667 | 57.99324 | 1 | 1.30E-02 | 1.46E-02 |
| Con 631 | 70.01852 | 60.47297 | 1 | 1.31E-02 | 1.46E-02 |
| Con 1241 | 76.27778 | 55.90541 | 1 | 1.46E-02 | 1.61E-02 |
| *Alistipes indistinctus* | 54.50926 | 71.79054 | 0 | 1.59E-02 | 1.72E-02 |
| Con 8420 | 72.64815 | 58.55405 | 1 | 2.32E-02 | 2.48E-02 |
| *Burkholderiales bacterium 1_1_47* | 72.37963 | 58.75 | 1 | 2.34E-02 | 2.48E-02 |
| Con 7993 | 73.74074 | 57.75676 | 1 | 3.01E-02 | 3.16E-02 |
| Con 425 | 73.19444 | 58.15541 | 1 | 3.87E-02 | 4.01E-02 |
| Con 561 | 70.5 | 60.12162 | 1 | 4.81E-02 | 4.92E-02 |

Table 4 PERMANOVA analysis of variation in three CRC-enriched species measured by three different methods in cohort I. CRC status and colonoscopy explain the variation in these three species.

| Parameter | Df | mOTU species | | | | | IMG species | | | | | MLG species | | | | |
|---|---|---|---|---|---|---|---|---|---|---|---|---|---|---|---|---|
| | | SumsOf Sqs | MeanSqs | F. Model | $R^2$ | Pr(>F) | SumsOf Sqs | MeanSqs | F.Model | $R^2$ | Pr(>F) | SumsOf Sqs | MeanSqs | F.Model | $R^2$ | Pr(>F) |
| CRC Status | 1 | 5.85E-05 | 5.85E-05 | 5.1835238 | 0.0395135 | **0.0076** | 2.42E-04 | 2.42E-04 | 4.2189512 | 0.0323989 | 0.0127 | 7.02E-03 | 7.02E-03 | 5.9492807 | 0.0450876 | **0.0072** |
| Duration between colonoscopy and fecal sample collection | 1 | 4.05E-05 | 4.05E-05 | 3.5159771 | 0.0273583 | **0.0523** | 1.57E-04 | 1.57E-04 | 2.6787139 | 0.0209801 | 0.0777 | 4.25E-03 | 4.25E-03 | 3.5265637 | 0.0274384 | **0.0569** |
| Fecal sampling before or after colonoscopy | 1 | 3.21E-05 | 3.21E-05 | 2.7722393 | 0.0216967 | **0.0799** | 1.12E-04 | 1.12E-04 | 1.8992995 | 0.0149670 | 0.163 | 3.54E-03 | 3.54E-03 | 2.9217093 | 0.0228398 | **0.0799** |
| Stage of CRC | 4 | 8.38E-05 | 2.09E-05 | 1.8432688 | 0.0565537 | 0.1262 | 4.44E-04 | 1.11E-04 | 1.9437773 | 0.0594540 | 0.1157 | 1.27E-02 | 3.17E-03 | 2.7293564 | 0.0815236 | **0.0354** |
| Lesion location | 1 | 3.02E-05 | 3.02E-05 | 1.5272855 | 0.0236688 | 0.1846 | 1.28E-04 | 1.28E-04 | 1.2152307 | 0.0189243 | 0.1988 | 2.27E-03 | 2.27E-03 | 1.0493068 | 0.0163828 | 0.3215 |
| LDL | 1 | 2.03E-05 | 2.03E-05 | 1.4217908 | 0.0140186 | 0.2414 | 2.52E-05 | 2.52E-05 | 0.3436566 | 0.0034248 | 0.5793 | 6.77E-04 | 6.77E-04 | 0.4524804 | 0.0045044 | 0.5249 |
| eGFR | 1 | 5.78E-06 | 5.78E-06 | 0.4256440 | 0.0039622 | 0.5138 | 4.77E-06 | 4.77E-06 | 0.0692402 | 0.0006467 | 0.8438 | 3.31E-04 | 3.31E-04 | 0.2318740 | 0.0021624 | 0.6453 |
| TCHO | 1 | 1.24E-05 | 1.24E-05 | 0.8618039 | 0.0085444 | 0.3454 | 7.84E-06 | 7.84E-06 | 0.1067080 | 0.0010659 | 0.7915 | 2.81E-04 | 2.81E-04 | 0.1872153 | 0.0018687 | 0.6821 |
| Lesion specific location | 1 | 4.15E-06 | 4.15E-06 | 0.2052181 | 0.0032469 | 0.6648 | 1.41E-06 | 1.41E-06 | 0.0131386 | 0.0002085 | 0.9754 | 8.14E-05 | 8.14E-05 | 0.0370280 | 0.0005874 | 0.9353 |
| HDL | 1 | 3.24E-07 | 3.24E-07 | 0.0222985 | 0.0002229 | 0.9401 | 4.69E-06 | 4.69E-06 | 0.0638119 | 0.0006377 | 0.8687 | 3.50E-05 | 3.50E-05 | 0.0232691 | 0.0002326 | 0.955 |

(continued)

| Parameter | Df | mOTU species | | | | | IMG species | | | | | MLG species | | | | |
|---|---|---|---|---|---|---|---|---|---|---|---|---|---|---|---|---|
| | | SumsOf Sqs | MeanSqs | F. Model | $R^2$ | Pr(>F) | SumsOf Sqs | MeanSqs | F.Model | $R^2$ | Pr(>F) | SumsOf Sqs | MeanSqs | F.Model | $R^2$ | Pr(>F) |
| Age | 1 | 1.75E-07 | 1.75E-07 | 0.0148715 | 0.0001180 | 0.9652 | 3.05E-06 | 3.05E-06 | 0.0515304 | 0.0004088 | 0.8841 | 3.47E-05 | 3.47E-05 | 0.0280829 | 0.0002228 | 0.9507 |
| FBG | 1 | 4.03E-06 | 4.03E-06 | 0.2850014 | 0.0028997 | 0.5725 | 1.73E-05 | 1.73E-05 | 0.2322323 | 0.0023641 | 0.6205 | 1.70E-03 | 1.70E-03 | 1.1175736 | 0.0112752 | 0.2544 |
| BMI | 1 | 1.41E-06 | 1.41E-06 | 0.1195008 | 0.0009551 | 0.749 | 1.07E-05 | 1.07E-05 | 0.1801544 | 0.0014392 | 0.6958 | 8.11E-05 | 8.11E-05 | 0.0651803 | 0.0005212 | 0.8618 |
| Cr | 1 | 2.32E-06 | 2.32E-06 | 0.1668589 | 0.0015866 | 0.6698 | 3.16E-06 | 3.16E-06 | 0.0449746 | 0.0004281 | 0.8759 | 1.61E-04 | 1.61E-04 | 0.1103230 | 0.0010496 | 0.7615 |
| ALT/GPT | 1 | 8.01E-07 | 8.01E-07 | 0.0625344 | 0.0005896 | 0.8156 | 6.22E-06 | 6.22E-06 | 0.0929296 | 0.0008759 | 0.7813 | 5.69E-04 | 5.69E-04 | 0.4106836 | 0.0038594 | 0.4907 |
| TNM | 15 | 5.83E-05 | 3.89E-06 | 0.1815751 | 0.0448528 | 0.9841 | 3.68E-04 | 2.46E-05 | 0.2193220 | 0.0536766 | 0.9134 | 1.15E-02 | 7.68E-04 | 0.3435946 | 0.0816089 | 0.8323 |
| TG | 1 | 3.80E-07 | 3.80E-07 | 0.0261886 | 0.0002618 | 0.9144 | 6.05E-07 | 6.05E-07 | 0.0082320 | 0.0000823 | 0.9827 | 1.39E-04 | 1.39E-04 | 0.0922060 | 0.0009212 | 0.7912 |
| Gender | 1 | 1.07E-06 | 1.07E-06 | 0.0908585 | 0.0007206 | 0.8475 | 9.10E-06 | 9.10E-06 | 0.1537437 | 0.0012187 | 0.8233 | 1.65E-04 | 1.65E-04 | 0.1336220 | 0.0010594 | 0.7801 |
| DM | 1 | 5.19E-07 | 5.19E-07 | 0.0441774 | 0.0003505 | 0.9158 | 4.74E-06 | 4.74E-06 | 0.0800697 | 0.0006351 | 0.8975 | 2.34E-04 | 2.34E-04 | 0.1895356 | 0.0015020 | 0.7209 |

Table 5 IMG, mOTU and MLG species markers. IMG, mOTU and MLG species markers identified using random forest method among species associated with CRC (Table 4). Marker species were listed by their importance reported by the method.

| 17 IMG species markers | | | | | |
|---|---|---|---|---|---|
| | Control rank mean | Case rank mean | Enrichment(0:case/ 1:control) | P-value | q-value |
| *Peptostreptococcus stomatis* | 37.25926 | 84.37838 | 0 | 5.11E-12 | 1.32E-08 |
| *Parvimonas micra* | 38.43519 | 83.52027 | 0 | 4.21E-11 | 5.43E-08 |
| *Parvimonas sp. oral taxon 393* | 39.81481 | 82.51351 | 0 | 2.79E-10 | 2.40E-07 |
| *Parvimonas sp. oral taxon 110* | 43.52778 | 79.80405 | 0 | 6.17E-08 | 3.98E-05 |
| *Gemella morbillorum* | 43.87037 | 79.55405 | 0 | 1.53E-07 | 7.88E-05 |
| *Fusobacterium nucleatum* | 45.09259 | 78.66216 | 0 | 3.86E-07 | 1.56E-04 |
| *Leptotrichia buccalis* | 45.60185 | 78.29054 | 0 | 4.44E-07 | 1.56E-04 |
| *Fusobacterium sp. oral taxon 370* | 45.02778 | 78.70946 | 0 | 4.83E-07 | 1.56E-04 |
| *Burkholderia mallei* | 45.19444 | 78.58784 | 0 | 7.93E-07 | 2.27E-04 |
| *Prevotella intermedia* | 46.47222 | 77.65541 | 0 | 1.92E-06 | 4.95E-04 |
| *Streptococcus dysgalactiae* | 47.06481 | 77.22297 | 0 | 4.18E-06 | 8.99E-04 |
| *Beggiatoa sp. PS* | 46.53704 | 77.60811 | 0 | 5.03E-06 | 9.97E-04 |
| *Malassezia globosa* | 46.35185 | 77.74324 | 0 | 8.71E-06 | 1.60E-03 |
| *Paracoccus denitrificans* | 47.48148 | 76.91892 | 0 | 1.18E-05 | 2.02E-03 |
| *Eubacterium ventriosum* | 80.98148 | 52.47297 | 1 | 1.27E-05 | 2.05E-03 |
| *Filifactor alocis* | 49.06481 | 75.76351 | 0 | 3.94E-05 | 5.65E-03 |
| *Solobacterium moorei* | 47.66667 | 76.78378 | 0 | 8.79E-05 | 9.85E-03 |
| **7 mOTU species markers** | | | | | |
| | **Control rank mean** | **Case rank mean** | **Enrichment(0: case/l:control)** | **P-value** | **q-value** |
| *Peptostreptococcus stomatis* | 37.25926 | 84.37838 | 0 | 5.11E-12 | 1.32E-08 |
| *Parvimonas micra* | 38.43519 | 83.52027 | 0 | 4.21E-11 | 5.43E-08 |
| *Parvimonas sp. oral taxon 393* | 39.81481 | 82.51351 | 0 | 2.79E-10 | 2.40E-07 |
| *Parvimonas sp. oral taxon 110* | 43.52778 | 79.80405 | 0 | 6.17E-08 | 3.98E-05 |
| *Gemella morbillorum* | 43.87037 | 79.55405 | 0 | 1.53E-07 | 7.88E-05 |
| *Fusobacterium nucleatum* | 45.09259 | 78.66216 | 0 | 3.86E-07 | 1.56E-04 |

(continued)

| 7 mOTU species markers | | | | | |
|---|---|---|---|---|---|
| | **Control rank mean** | **Case rank mean** | **Enrichment(0: case/l:control)** | **P-value** | **q-value** |
| *Leptotrichia buccalis* | 45.60185 | 78.29054 | 0 | 4.44E-07 | 1.56E-04 |
| **27 MLG species markers** | | | | | |
| | Control rank mean | Case rank mean | Enrichment(0:case/ 1:control) | P-value | q-value |
| *Parvimonas micra* | 38.40741 | 83.54054 | 0 | 5.56E-12 | 4.84E-10 |
| *Fusobacterium nucleatum* | 40.32407 | 82.14189 | 0 | 1.72E-10 | 7.48E-09 |
| *Solobacterium moorei* | 42.2037 | 80.77027 | 0 | 4.01E-08 | 1.16E-06 |
| *Clostridium symbiosum* | 46.31481 | 77.77027 | 0 | 2.67E-06 | 5.80E-05 |
| Con_10180 | 82.03704 | 51.7027 | 1 | 6.06E-06 | 1.05E-04 |
| CRC_2881 | 51.25926 | 74.16216 | 0 | 7.57E-06 | 1. 10E-04 |
| *Coprococcus sp. ART55/1* | 80.85185 | 52.56757 | 1 | 2.09E-05 | 2.05E-04 |
| *Clostridium hathewayi* | 46.77778 | 77.43243 | 0 | 2.12E-05 | 2.05E-04 |
| *Clostridiales bacterium 1_7_47FAA* | 48.16667 | 76.41892 | 0 | 2.49E-05 | 2.17E-04 |
| CRC_4136 | 50.99074 | 74.35811 | 0 | 2.97E-05 | 2.32E-04 |
| *butyrate-producing bacterium SS3/4* | 80.57407 | 52.77027 | 1 | 3.19E-05 | 2.32E-04 |
| *Haemophilus parainfluenzae* | 80.49074 | 52.83108 | 1 | 4.18E-05 | 2.69E-04 |
| Con_154 | 80.35185 | 52.93243 | 1 | 4.45E-05 | 2.69E-04 |
| *Bacteroides fragilis* | 49.09259 | 75.74324 | 0 | 5.56E-05 | 3.02E-04 |
| Con_1979 | 79.94444 | 53.22973 | 1 | 6.03E-05 | 3.09E-04 |
| Con_7958 | 75.27778 | 56.63514 | 1 | 7.40E-05 | 3.33E-04 |
| Con_5770 | 79.39815 | 53.62838 | 1 | 7.66E-05 | 3.33E-04 |
| CRC_6481 | 52.09259 | 73.55405 | 0 | 9.87E-05 | 3.90E-04 |
| Con_1987 | 79.42593 | 53.60811 | 1 | 1.17E-04 | 4.23E-04 |
| Con_4595 | 77.21296 | 55.22297 | 1 | 1.38E-04 | 4.81E-04 |
| *Eubacterium biforme* | 74.68519 | 57.06757 | 1 | 3.00E-04 | 8.70E-04 |
| *Desulfovibrio sp. 6_1_46AFAA* | 53.33333 | 72.64865 | 0 | 3.70E-04 | 9.87E-04 |
| *Clostridium citroniae* | 51.71296 | 73.83108 | 0 | 1.08E-03 | 2.19E-03 |
| *Fusobacterium varium* | 54.57407 | 71.74324 | 0 | 1.15E-03 | 2.28E-03 |
| *Roseburia intestinalis* | 76.99074 | 55.38514 | 1 | 2.20E-03 | 3.58E-03 |
| *Dorea formicigenerans* | 52.98148 | 72.90541 | 0 | 4.36E-03 | 5.84E-03 |
| CRC_3579 | 54.05556 | 72.12162 | 0 | 6.09E-03 | 7.46E-03 |

Table 8 PERMANOVA analysis of variation in 20 CRC-associated gene markers in cohort I. CRC status explains the variation in these gene profiles, while CRC stage moderately explains the variation.

| Parameter | Df | SumsOfSqs | MeanSqs | F.Model | $R^2$ | Pr(>F) | q-value |
|---|---|---|---|---|---|---|---|
| CRC Status | 1 | 5.5793661 | 5.5793661 | 16.626711 | 0.116575 | 0.0001 | 0.00095 |
| Stage of CRC | 4 | 6.7812635 | 1.6953159 | 5.0761083 | 0.1416874 | 0.0001 | 0.00095 |
| FBG | 1 | 0.8119553 | 0.8119553 | 2.154786 | 0.0215146 | 0.0073 | 0.046233 |
| Fecal sampling before or after colonoscopy | 1 | 0.5473702 | 0.5473702 | 1.4588296 | 0.011536 | 0.0978 | 0.46455 |
| Lesion location | 1 | 0.500106 | 0.500106 | 1.4185104 | 0.0220202 | 0.1329 | 0.486163 |
| Lesion specific location | 7 | 2.7831853 | 0.3975979 | 1.1372468 | 0.1225468 | 0.1889 | 0.486163 |
| HDL | 1 | 0.4718905 | 0.4718905 | 1.2480119 | 0.0123263 | 0.203 | 0.486163 |
| ALT/GPT | 1 | 0.4650084 | 0.4650084 | 1.2366953 | 0.0115324 | 0.2047 | 0.486163 |
| Duration between colonoscopy and fecal sample collection | 1 | 0.4170429 | 0.4170429 | 1.1084063 | 0.0087893 | 0.3116 | 0.657822 |
| Age | 1 | 0.3976816 | 0.3976816 | 1.0557238 | 0.0083091 | 0.3669 | 0.676838 |
| TCHO | 1 | 0.3768657 | 0.3768657 | 0.9942006 | 0.0098441 | 0.4287 | 0.676838 |
| DM | 1 | 0.3653642 | 0.3653642 | 0.9692711 | 0.0076339 | 0.4617 | 0.676838 |
| BMI | 1 | 0.3660728 | 0.3660728 | 0.9708139 | 0.0077067 | 0.4631 | 0.676838 |
| Cr | 1 | 0.3412225 | 0.3412225 | 0.8963725 | 0.0084646 | 0.5617 | 0.719847 |
| TNM | 15 | 5.2686733 | 0.3512449 | 0.9797038 | 0.2021521 | 0.5683 | 0.719847 |
| LDL | 1 | 0.308397 | 0.308397 | 0.8136124 | 0.0080705 | 0.6624 | 0.741782 |
| Gender | 1 | 0.3092058 | 0.3092058 | 0.8193202 | 0.0064605 | 0.6637 | 0.741782 |
| TG | 1 | 0.291975 | 0.291975 | 0.7695216 | 0.0076365 | 0.7334 | 0.774144 |
| eGFR | 1 | 0.2043621 | 0.2043621 | 0.539403 | 0.0050159 | 0.9496 | 0.9496 |

Table 13 Baseline characteristics of the Chinese cohort (cohort II) consisting 47 CRC patients and 109 control individuals

| Parameter | Controls (n=109) | Cases (n=47) | Statistical test for differences | *P-value* | q-value |
|---|---|---|---|---|---|
| Fecal sampling before or after colonoscopy (before:after) | 101:8 | 9:38 | Chi-square | 1.466E-19 | 1.906E-18 |
| Duration between colonoscopy and fecal sample collection | -58.25688 | 25.02128 | Wilcoxon | 4.064E-14 | 2.642E-13 |
| Age | 58 | 67.95745 | Wilcoxon | 3.146E-06 | 1.363E-05 |
| TCHO | 5.036364 | 4.357143 | Wilcoxon | 0.0769 | 0.1824 |
| Gender (M:F) | 40:69 | 25:22 | Chi-square | 0.0818 | 0.1824 |
| FBG | 5.181818 | 6.128571 | Wilcoxon | 0.0842 | 0.1824 |
| ALT/GPT | 25.2 | 18.28571 | Wilcoxon | 0.1043 | 0.1936 |

(continued)

| Parameter | Controls (n=109) | Cases (n=47) | Statistical test for differences | *P-value* | q-value |
|---|---|---|---|---|---|
| Duration of frozen storage of fecal samples | 673.2844 | 1106.34 | Wilcoxon | 0.2086 | 0.3390 |
| HDL | 1.596 | 1.414286 | Wilcoxon | 0.2822 | 0.4076 |
| TG | 1.047273 | 1.028571 | Wilcoxon | 0.4680 | 0.6085 |
| Cr | 82.06667 | 77.57143 | Wilcoxon | 0.5484 | 0.6481 |
| LDL | 2.95 | 2.728571 | Wilcoxon | 0.6241 | 0.6761 |
| BMI | 23.29 | 22.69 | Wilcoxon | 0.7098 | 0.7098 |
| TNM (T1N0:T3N0:T1N1:T3N1:T3N2: T4N1: T2N1M1:T3N1M1:T3N2M1: UT4:Mx) | n.a | 4:23:1:9: 4:1:1:1:1: 1: 1 | n.a | n.a | n.a |
| Stage of CRC (1:2:3:4) | n.a | 4:24:15:4 | n.a | n.a | n.a |
| Lesion specific location (2:3:4:6:7:8:9: NA) | n.a | 3:3:3:2:7: 4:7:18 | n.a | n.a | n.a |
| Lesion location (1:2:NA) | n.a | 9:20:18 | n.a | n.a | n.a |

Table 14 two case-enriched and two control-enriched gene markers

| Marker ID | Gene description | Enrichment | Wilcoxon rank-sum test P-value | Wilcoxon rank-sum test stratified for colonoscopy | Mantel Haentzel Odds Ratio, adjusted for colonoscopy (95% CI) | Mantel Haentzel test P-value |
|---|---|---|---|---|---|---|
| 1704941 | butyryl-CoA dehydrogenase | case | 1.97E-09 | 1.52E-03 | 18.54(2.62-131) | 0.00509 |
| 482585 | RNA-directed DNA polymerase | case | 2.34E-03 | 4.55E-02 | 1.815 (0.653-5.05) | 0.38 |
| 181682 | gene with unknown function from Roseburia intestinalis | control | 2.15E-01 | 3.13E-01 | 1.495(0.456-4.9) | 0.714 |
| 370640 | gene with unknown function from Bacteroides clarus | control | 3.11E-01 | 6.30E-01 | 1.647 (0.395-6.88) | 0.778 |

Table 15 Baseline characteristics of the Danish cohort (cohort III) consisting 16 CRC patients and 24 control individuals.

| Parameter | Control (n=24) | Case(n=16) | *P-value* | q-value |
|---|---|---|---|---|
| Duration between colonoscopy and fecal sample collection | 0.79 | -83.5 | 0.075 | 0.23382 |
| Gender (M:F) | 7:17 | 10:06 | 0.07794 | 0.23382 |
| Age | 61.625 | 66.6875 | 0.4308219 | 0.759376 |
| Fecal sampling before or after colonoscopy (before:after) | 22:02 | 13:03 | 0.6255852 | 0.759376 |

(continued)

| Parameter | Control (n=24) | Case(n=16) | *P-value* | q-value |
|---|---|---|---|---|
| BMI | 25.90667 | 25.90667 | 0.6328136 | 0.759376 |
| DM (YES:NO) | 3:21 | 1:15 | 0.9143266 | 0.914327 |
| Cancer location (Adenocarcinom:Ascendens: Coecum:Rectum: Sigmoideum: Trans versum) | n.a | 1:1:1:9:3:1 | n.a | n.a |
| Cancer location (Distal:Proximal) | n.a | 13:03 | n.a | n.a |
| TNM (T1N0M0V0:T3N0M0V0:T3N0M0V1: T3N1M0V0:T3N2M0V0:T4N0M0: T4N2M0V 1: T4NxMx) | n.a | 1:6:3:1:2:1:1: 1 | n.a | n.a |
| Stage of CRC (1:2:3:4) | n.a | 1:9:5:1 | n.a | n.a |

Table 16 Community structure differences between Chinese and Danish cohorts. All comparisons were performed using Wilcoxon rank-sum test.

| | **Gene count P-value** | | | | **Shannon index P-value** | | | |
|---|---|---|---|---|---|---|---|---|
| | Denmark_Case | Denmark_Control | China_Case | China_Control | Denmark_Case | Denmark_Control | China_Case | China_Control |
| Denmark_Case | | 0.25991847 | 1. 94E-05 | 0.000294527 | | 0.772788361 | 5.84639E-05 | 4.02E-04 |
| Denmark_Control | | | 7.86E-05 | 0.001729823 | | | 2.25586E-05 | 9.34E-04 |
| China_Case | | | | 0.212812929 | | | | 0.178412749 |

Table 17 Species annotation of the 1498 overlapped genes in Chinese CRC case and Denmark CRC case. A large fraction was annotated to Parvimonas micra. Annotated species with more than 10 genes are listed here.

| Species | Gene numbers |
|---|---|
| Parvimonas micra | 389 |
| Solobacterium moorei | 204 |
| Clostridium symbiosum | 177 |
| Clostridium sp. 7_3_54FAA | 108 |
| Parvimonas sp. oral taxon 110 | 93 |
| Parvimonas sp. oral taxon 393 | 93 |
| Fusobacterium nucleatum | 64 |
| Peptostreptococcus stomatis | 23 |
| Clostridium hathewayi | 17 |
| Clostridium citroniae | 14 |
| Akkermansia muciniphila | 11 |
| [Clostridium] difficile | 11 |
| Peptostreptococcus anaerobius | 10 |

Table 18 List of CRC-associated species predicted from Chinese CRC cohort and validated in Danish CRC cohort with q<0.05

| ***IMG species validated in Danish cohort*** | | | | | |
|---|---|---|---|---|---|
| | Control rank mean | Case rank mean | Enrichment(0:case/1: control) | P-value | q-value |
| Parvimonas_sp._oral_ taxon_110 | 14.54166667 | 29.4375 | 0 | 9.06E-05 | 0.000808962 |
| Parvimonas_sp._oral_ taxon_393 | 14.66666667 | 29.25 | 0 | 0.000127394 | 0.000808962 |
| Parvimonas_micra | 14.70833333 | 29.1875 | 0 | 0.00015168 | 0.000808962 |
| Gemella_morbillorum | 15.70833333 | 27.6875 | 0 | 0.001465743 | 0.005862972 |
| Peptostreptococcus_ stomatis | 16.16666667 | 27 | 0 | 0.003409134 | 0.010909228 |
| Fusobacterium_sp._ oral_taxon_37 0 | 16.58333333 | 26.375 | 0 | 0.010235287 | 0.024739601 |
| Fusobacterium_ nucleatum | 16.70833333 | 26.1875 | 0 | 0.010823576 | 0.024739601 |
| Malassezia_globosa | 17 | 25.75 | 0 | 0.023703729 | 0.047407459 |
| **mOTU species validated in Danish cohort** | | | | | |
| | Control rank mean | Case rank mean | Enrichment(0:case/1: control) | P-value | q-value |
| Peptostreptococcus_ stomatis | 16.5 | 26.5 | 0 | 0.000139835 | 0.000978842 |
| Parvimonas_micra | 16.70833333 | 26.1875 | 0 | 0.000749378 | 0.002622823 |
| Gemella_morbillorum | 18 | 24.25 | 0 | 0.004603221 | 0.010740848 |

(continued)

| MLG species validated in Danish cohort | | | | | |
|---|---|---|---|---|---|
| | **Control rank mean** | **Case rank mean** | **Enrichment (1: Control;0:Case)** | **P-value** | **q-value** |
| *Parvimonas micra* | 15.20833333 | 28.4375 | 0 | 9.13E-05 | 0.002329351 |
| *Solobacterium moorei* | 16.22916667 | 26.90625 | 0 | 0.000172545 | 0.002329351 |

Table 19 List of four gene markers predicted from Chinese cohort I that show significant associations in Danish cohort III with q<0.05.

| Gene | Chinese cohort I | | | Danish cohort III | | | Blastn on IMG v400 | Blastp on KEGG v59 | |
|---|---|---|---|---|---|---|---|---|---|
| Marker ID | P-value | q-value | Enrich | P-value | q-value | Enrich | Species taxonomy | KEGG ID | Gene annotation |
| 2361423 | 2.31148E-13 | 4.87836E-07 | case | 0.000237993 | 0.001765177 | case | *Peptostreptococcus anaerobius* | K07485 | transposase |
| 3173495 | 6.23501E-13 | 6.57946E-07 | case | 0.000222993 | 0.001765177 | case | *Peptostreptococcus anaerobius* | K07485 | transposase |
| 1696299 | 7.69646E-10 | 0.000406082 | case | 0.000264777 | 0.001765177 | case | *Parvimonas micra* | K03043 | DNA-directed RNA polymerase subunit beta |
| 1704941 | 7.53342E-08 | 0.002606428 | case | 0.00669301 | 0.03346505 | case | *Fusobacterium nucleatum* | K00248 | butyryl-CoA dehydrogenase |

Table 20 PERMANOVA analysis of variation in four gene markers validated in cohort III (No. of permutations = 9999). CRC status explains the variation in these gene profiles.

| phenotype | Df | SumsOf Sqs | Mean Sqs | F.Model | $R^2$ | Pr (>F) |
|---|---|---|---|---|---|---|
| CRC Status | 1 | 8.11E-11 | 8.11E-11 | 4.8910108 | 0.1140335 | 0.0001 |
| Stage of CRC | 4 | 1.15E-10 | 2.86E-11 | 1.6816488 | 0.1612064 | 0.1375 |
| Duration between colonoscopy and fecal sample collection | 1 | 2.03E-11 | 2.03E-11 | 1.1199259 | 0.028628 | 0.2265 |
| Cancer location (Distal:Proximal) | 1 | 5.20E-11 | 5.20E-11 | 1.2648699 | 0.0828615 | 0.2383 |
| Cancer location(Adenocarcinom: Ascendens:Coecum:Rectum: Sigmoideum:Transversum) | 5 | 3.12E-10 | 6.24E-11 | 1.9756046 | 0.4969319 | 0.2998 |
| Age | 1 | 1.48E-11 | 1.48E-11 | 0.8097989 | 0.0208658 | 0.3989 |
| DM | 1 | 5.61E-12 | 5.61E-12 | 0.3020817 | 0.0078868 | 0.5654 |
| Gender | 1 | 6.48E-12 | 6.48E-12 | 0.3495622 | 0.0091152 | 0.571 |
| BMI | 1 | 7.51E-12 | 7.51E-12 | 0.4060178 | 0.0105717 | 0.5869 |
| DNA purification date | 1 | 3.66E-12 | 3.66E-12 | 0.1966498 | 0.0051484 | 0.6696 |
| Fecal sampling before or after colonoscopy | 1 | 6.95E-12 | 6.95E-12 | 0.3749813 | 0.0097715 | 0.6878 |
| TNM | 7 | 1.57E-10 | 2.25E-11 | 0.3823119 | 0.2506686 | 0.7061 |

Table 24 Classification accuracy of the two marker genes, stratified into early (I-II) and late (III-IV) stage cancer.

| Marker ID | Group | Enrichment | Wilcox rank-sum test, P-value | Wilcoxon rank-sum test stratified for colonoscopy, P-value | Mantel Haenszel Odds Ratio adjusted for colonoscopy (95% CI) | Mantel-Haenszel test P-value |
|---|---|---|---|---|---|---|
| 1696299 | Stage I, II | case | 6.51E-14 | 3.35E-06 | 21.5(3.18-146) | 1.38E-05 |
| 1704941 | | case | 4.15E-07 | 0.008654411 | 27.77(1.64-469) | 0.0322 |
| 1696299 or 1704941 | | | | | 33.37(4.49-248) | 1.68E-06 |
| 1696299 | Stage III,IV | case | 1.51E-11 | 0.00027574 | 15.44(3.06-77.9) | 0.00109 |
| 1704941 | | case | 4.40E-09 | 0.002700628 | 25.34(2.91-221) | 0.00842 |
| 1696299 or 1704941 | | | | | 15.77(3.52-70.6) | 0.000653 |

## Claims

**1.** A gene marker set for use in the prediction of the risk of colorectal cancer (CRC) in a subject comprising:

a) 20 genes with the sequences as set forth in SEQ ID NOs: 1 to 20;
b) 4 genes with the sequences as set forth in SEQ ID NOs: 1, 9, 13 and 16;
c) 2 genes with the sequences as set forth in SEQ ID NOs: 13 and 16; or
d) the gene with the sequence as set forth in SEQ ID NOs: 13, or *rpoB* gene from *P. micra* encoding RNA polymerase subunit β.

**2.** The gene marker set of claim 1 for use in the prediction of the risk of colorectal cancer (CRC) in a subject via the steps of:

1) collecting a sample *j* from the subject and extracting DNA from the sample;
2) determining the abundance information of each of gene marker in the gene marker set; and
3) calculating the index of sample *j* by the formula below:

$$I_j = \left[\frac{\sum_{i \in N} \log_{10}\left(A_{ij} + 10^{-20}\right)}{|N|} - \frac{\sum_{i \in M} \log_{10}\left(A_{ij} + 10^{-20}\right)}{|M|}\right]$$

$A_{ij}$ is the relative abundance of marker *i* in sample *j,* wherein i refers to each of the gene markers in said gene marker set;
*N* is a subset of all CRC-enriched markers in the gene marker set;
*M* is a subset of all control-enriched markers in the gene marker set;
and |*N*| and |*M*| are the sizes (number) of the biomarker respectively in these two subsets;

wherein an index greater than a cutoff indicates that the subject has or is at the risk of developing colorectal cancer (CRC).

**3.** The gene marker set for its use of claim 2, wherein the abundance information is gene relative abundance of each of gene marker in the gene marker set which is determined by means of sequencing method.

**4.** The gene marker set for its use of claim 2, wherein the abundance information is gene relative abundance of each of gene marker in the gene marker set which is determined by a qPCR method.

**5.** The gene marker set for its use of any one of claims 2-4, wherein the cutoff value is obtained by a Receiver Operator Characteristic (ROC) method, wherein the cutoff corresponds to when AUC (Area Under the Curve) reached at its maximum.

## Patentansprüche

**1.** Genmarkersatz zur Verwendung bei der Prognose des Kolorektalkrebsrisikos (KKR) bei einem Individuum, der umfasst:

a) 20 Gene mit den in SEQ ID NOs: 1 bis 20 angeführten Sequenzen;
b) 4 Gene mit den in SEQ ID NOs: 1, 9, 13 und 16 angeführten Sequenzen;
c) 2 Gene mit den in SEQ ID NOs: 13 und 16 angeführten Sequenzen; oder
d) das Gen mit der in SEQ ID NO: 13 angeführten Sequenz oder *rpoB*-Gen von *P. micra,* das für die RNA-Polymerasesubeinheit β codiert.

**2.** Genmarkersatz nach Anspruch 1 zur Verwendung bei der Prognose des Kolorektalkrebsrisikos (KKR) bei einem Individuum durch die Schritte:

1) Entnehmen einer Probe *j* aus dem Individuum und Extrahieren von DNA aus der Probe;
2) Bestimmen der Abundanzinformation jedes Genmarkers in dem Genmarkersatz; und
3) Berechnen des Index der Probe *j* mit der nachstehenden Formel:

$$I_j = \left[\frac{\sum_{i\in N}\log_{10}\left(A_{ij}+10^{-20}\right)}{|N|} - \frac{\sum_{i\in M}\log_{10}\left(A_{ij}+10^{-20}\right)}{|M|}\right]$$

wobei $A_{ij}$ die relative Abundanz eines Markers *i* in der Probe *j* ist, wobei sich i auf jeden der Genmarker in dem Genmarkersatz bezieht;
*N* eine Teilmenge aller mit KKR angereicherten Marker in dem Genmarkersatz ist;
*M* eine Teilmenge aller mit Kontrolle angereicherten Marker in dem Genmarkersatz ist;
und $|N|$ und $|M|$ die jeweiligen Mengen (Anzahl) des Biomarkers in diesen zwei Teilmengen sind;

wobei ein Index größer als ein Cutoff indiziert, dass das Individuum an Kolorektalkrebs (KKR) leidet oder bei diesem das Risiko dessen Entwicklung besteht.

**3.** Genmarkersatz zu seiner Verwendung nach Anspruch 2, wobei die Abundanzinformation eine genrelative Abundanz jedes Genmarkers in dem Genmarkersatz ist, wie mithilfe eines Sequenzierverfahrens bestimmt.

**4.** Genmarkersatz zur seiner Verwendung nach Anspruch 2, wobei die Abundanzinformation eine genrelative Abundanz jedes Genmarkers in dem Genmarkersatz ist, wie mithilfe eines qPCR-Verfahrens bestimmt.

**5.** Genmarkersatz zu seiner Verwendung nach einem der Ansprüche 2 bis 4, wobei der Cutoff-Wert mithilfe eines Receiver-Operator-Characteristic-(ROC-)Verfahrens erhalten wird, wobei der Cutoff dem Zeitpunkt entspricht, an dem eine AUC (Fläche unter der Kurve) maximal ist.

## Revendications

**1.** Ensemble de marqueurs géniques pour une utilisation dans la prédiction du risque de cancer colorectal (CCR) chez un sujet comprenant :

a) 20 gènes avec les séquences décrites dans les SEQ ID NO : 1 à 20 ;
b) 4 gènes avec les séquences décrites dans les SEQ ID NO : 1, 9, 13 et 16 ;
c) 2 gènes avec les séquences décrites dans les SEQ ID NO : 13 et 16 ; ou

d) le gène avec la séquence décrite dans la SEQ ID NO : 13, ou le gène *rpoB* de *P. micra* codant pour la sous-unité β de l'ARN polymérase.

**2.** Ensemble de marqueurs géniques selon la revendication 1 pour une utilisation dans la prédiction du risque de cancer colorectal (CCR) chez un sujet par les étapes de :

1) collecte d'un échantillon *j* à partir du sujet et extraction de l'ADN de l'échantillon ;
2) détermination des informations d'abondance de chacun des marqueurs géniques dans l'ensemble de marqueurs géniques ; et
3) calcul de l'indice de l'échantillon *j* par la formule ci-dessous :

$$I_j = \left[\frac{\sum_{i \in N} \log_{10}\left(A_{ij} + 10^{-20}\right)}{|N|} - \frac{\sum_{i \in M} \log_{10}\left(A_{ij} + 10^{-20}\right)}{|M|}\right]$$

$A_{ij}$ est l'abondance relative du marqueur *i* dans l'échantillon *j*, où i fait référence à chacun des marqueurs géniques dans ledit ensemble de marqueurs géniques ;
*N* est un sous-ensemble de tous les marqueurs enrichis avec le CCR dans l'ensemble de marqueurs géniques ;
*M* est un sous-ensemble de tous les marqueurs enrichis avec le contrôle dans l'ensemble de marqueurs géniques ;
et |*N*| et |*M*| sont les tailles (nombre) du biomarqueur respectivement dans ces deux sous-ensembles ;

dans lequel un indice supérieur à un seuil indique que le sujet a ou risque de développer un cancer colorectal (CCR).

**3.** Ensemble de marqueurs géniques pour son utilisation selon la revendication 2, dans lequel les informations d'abondance sont une abondance par rapport au gène de chacun des marqueurs géniques dans l'ensemble de marqueurs géniques qui est déterminée par le biais d'une méthode de séquençage.

**4.** Ensemble de marqueurs géniques pour son utilisation selon la revendication 2, dans lequel les informations d'abondance sont une abondance par rapport au gène de chacun des marqueurs géniques dans l'ensemble de marqueurs géniques qui est déterminée par une méthode qPCR.

**5.** Ensemble de marqueurs géniques pour une utilisation selon l'une quelconque des revendications 2 à 4, dans lequel la valeur de seuil est obtenue par une méthode de caractéristiques de fonctionnement du récepteur (ROC), dans lequel le seuil correspond à lorsque l'ASC (aire sous la courbe) a atteint son maximum.

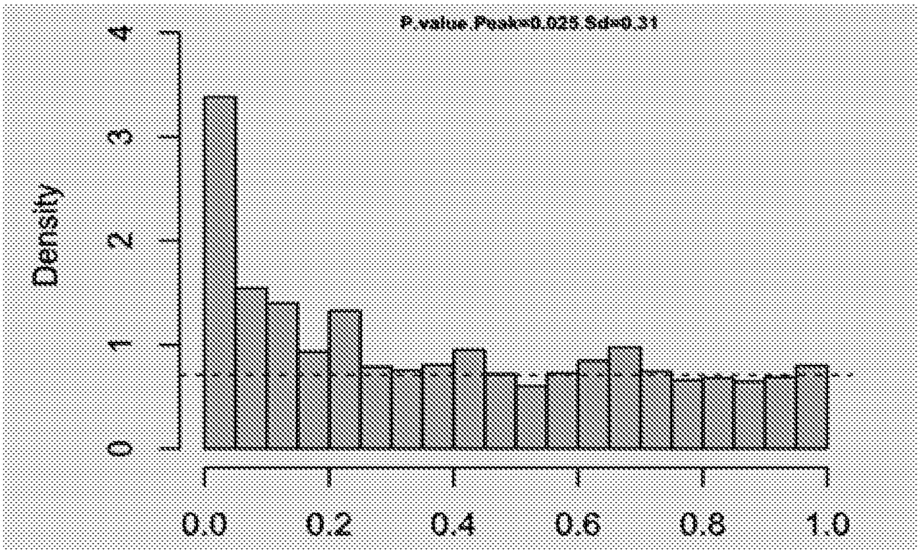
P.value.Peak=0.025.Sd=0.31
Density
0
1
2
3
4
0.0
0.2
0.4
0.6
0.8
1.0

Fig.1

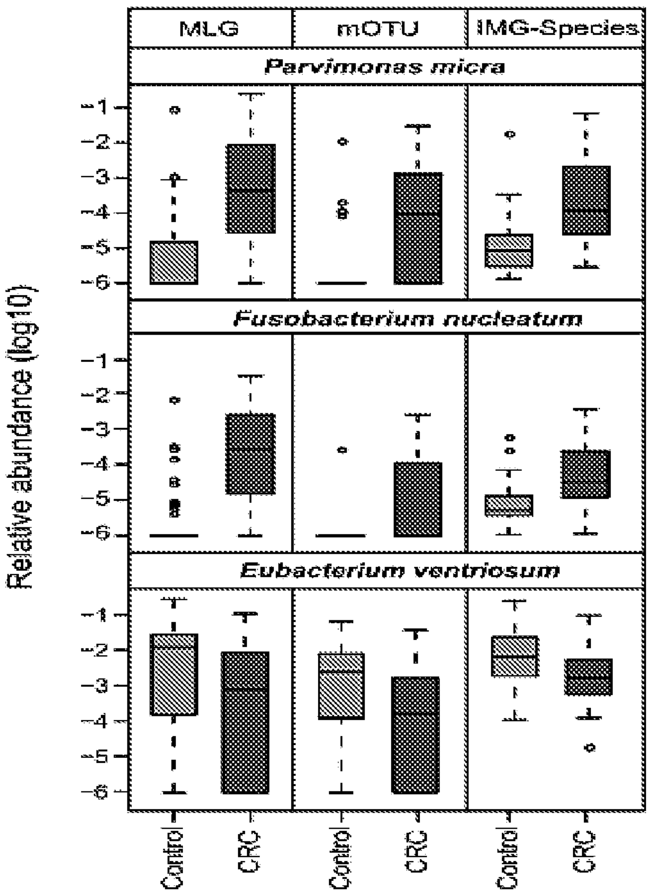
MLG
mOTU
IMG-Species
Parvimonas micra
Fusobacterium nucleatum
Eubacterium ventriosum
Relative abundance (log10)
-1
-2
-3
-4
-5
-6
Control
CRC

Fig.2

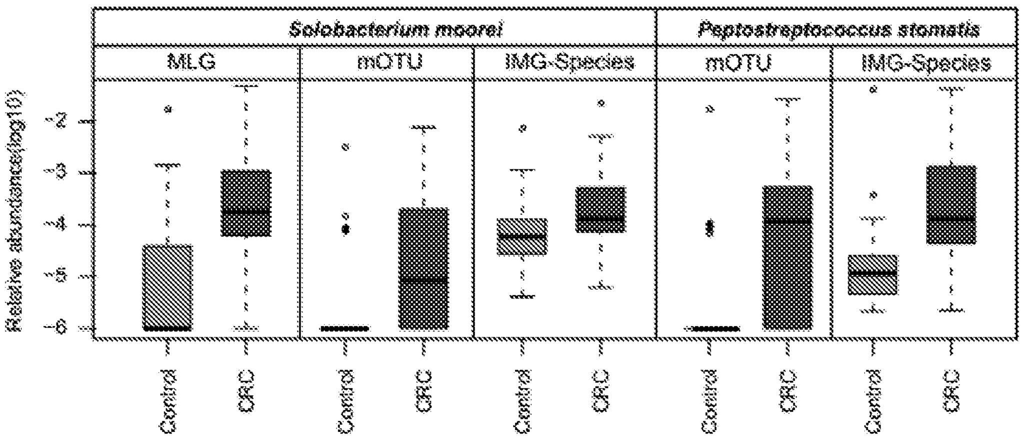
Solobacterium moorei
Peptostreptococcus stomatis
MLG
mOTU
IMG-Species
mOTU
IMG-Species
Relative abundance(log10)
-2
-3
-4
-5
-6
Control
CRC
Control
CRC
Control
CRC
Control
CRC
Control
CRC

Fig.3

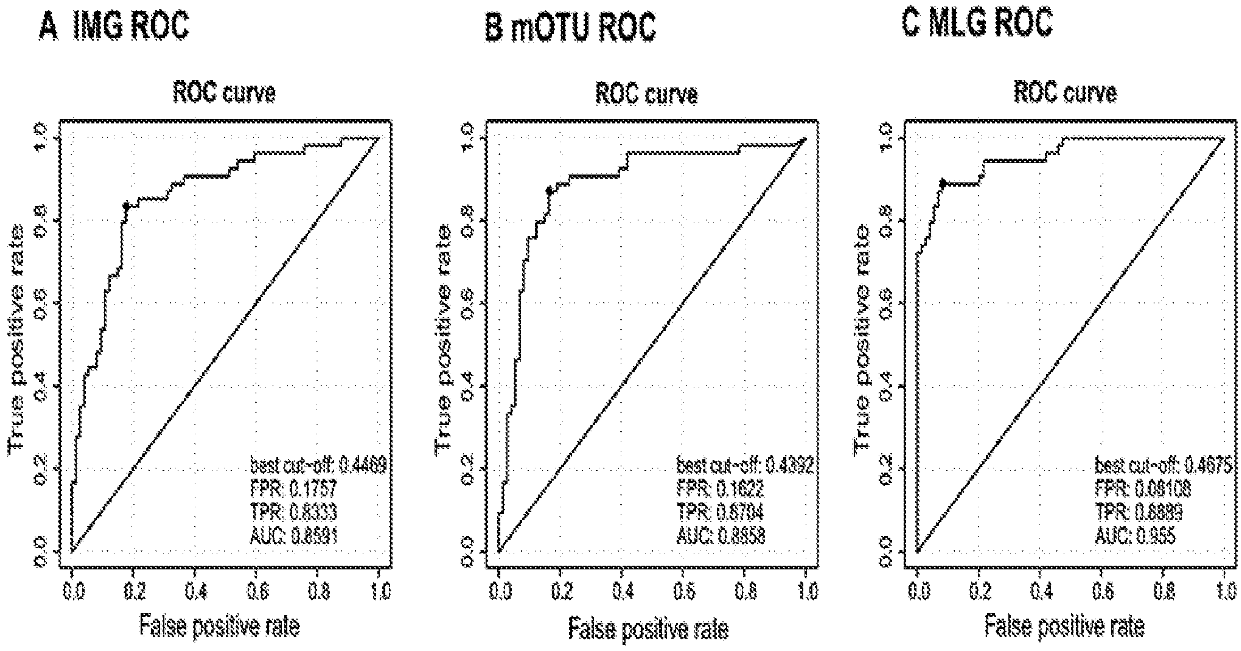
A IMG ROC
ROC curve
True positive rate
False positive rate
best cut-off: 0.4469
FPR: 0.1757
TPR: 0.8333
AUC: 0.8591
B mOTU ROC
ROC curve
True positive rate
False positive rate
best cut-off: 0.4392
FPR: 0.1622
TPR: 0.8704
AUC: 0.8858
C MLG ROC
ROC curve
True positive rate
False positive rate
best cut-off: 0.4675
FPR: 0.08108
TPR: 0.8889
AUC: 0.955
0.0
0.2
0.4
0.6
0.8
1.0

Fig.4

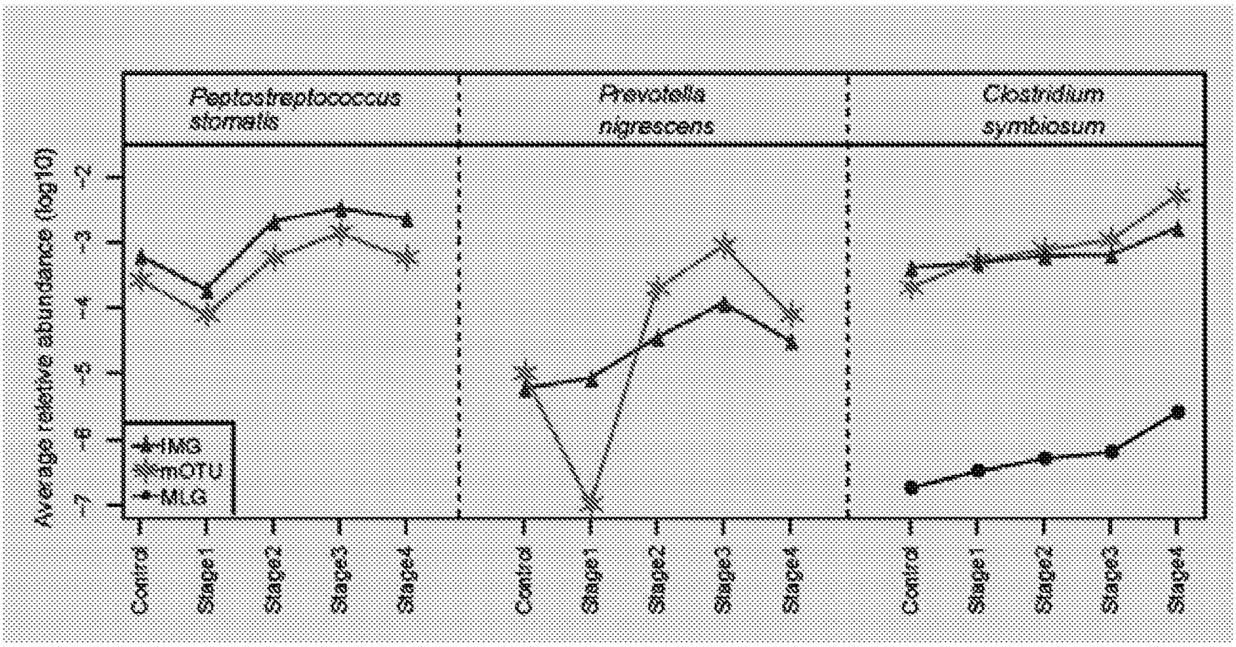
Peptostreptococcus stomatis
Prevotella nigrescens
Clostridium symbiosum
Average relative abundance (log10)
IMG
mOTU
MLG
Control
Stage1
Stage2
Stage3
Stage4

Fig.5

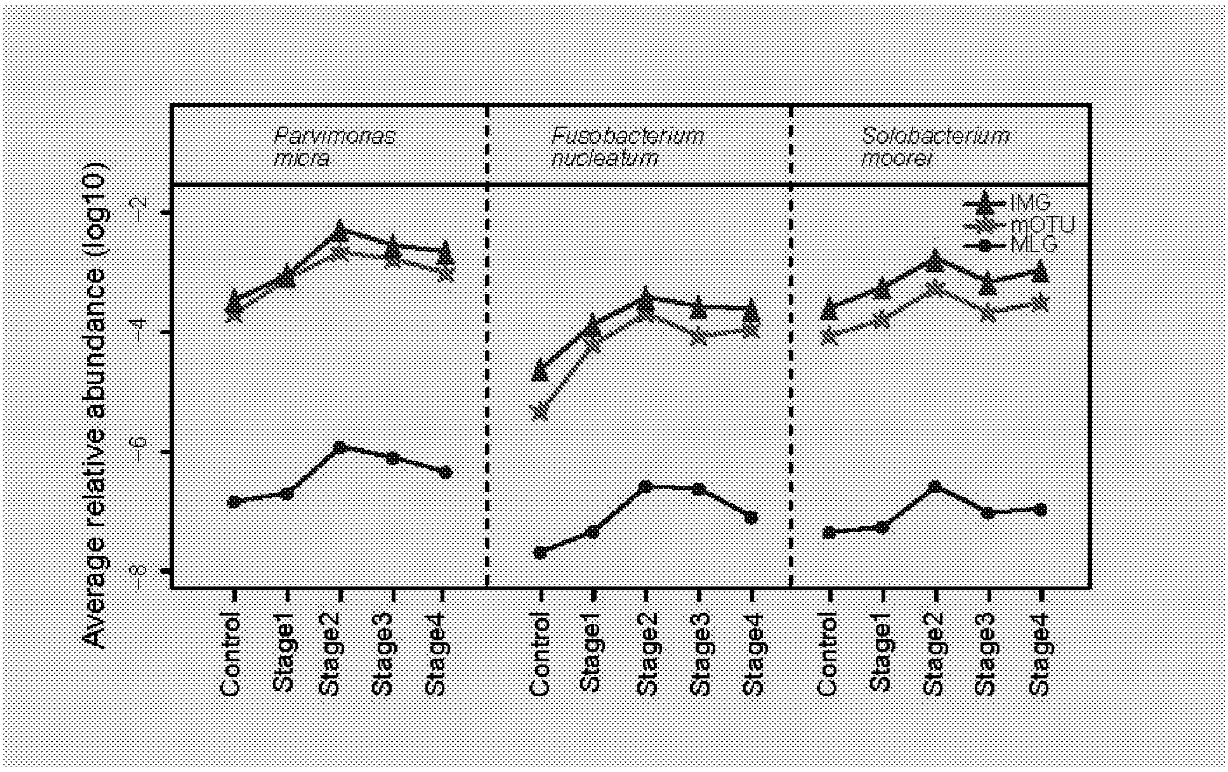
Parvimonas micra
Fusobacterium nucleatum
Solobacterium moorei
Average relative abundance (log10)
IMG
mOTU
MLG
Control
Stage1
Stage2
Stage3
Stage4

Fig.6

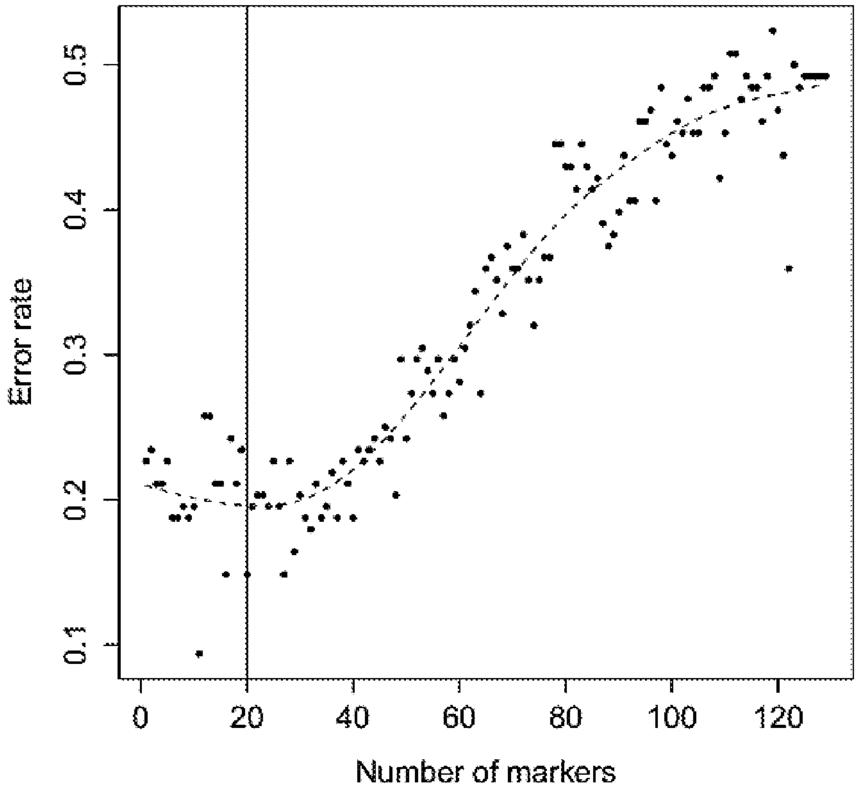
Error rate
0.1
0.2
0.3
0.4
0.5
0
20
40
60
80
100
120
Number of markers

Fig.7

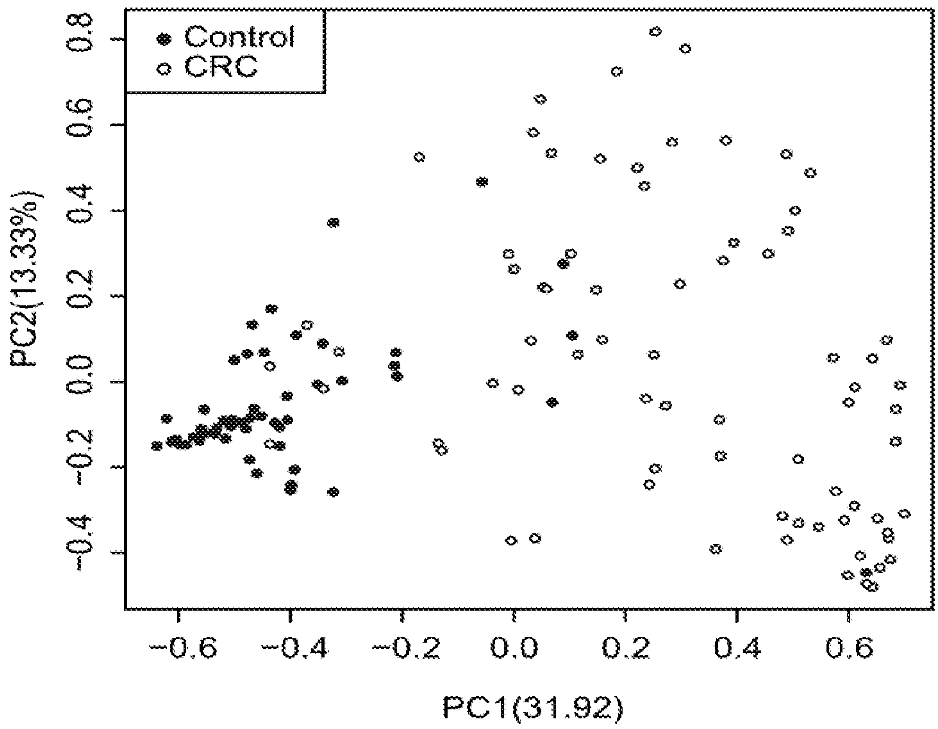
Control
CRC
PC2(13.33%)
−0.4
−0.2
0.0
0.2
0.4
0.6
0.8
−0.6
−0.4
−0.2
0.0
0.2
0.4
0.6
PC1(31.92)

Fig.8

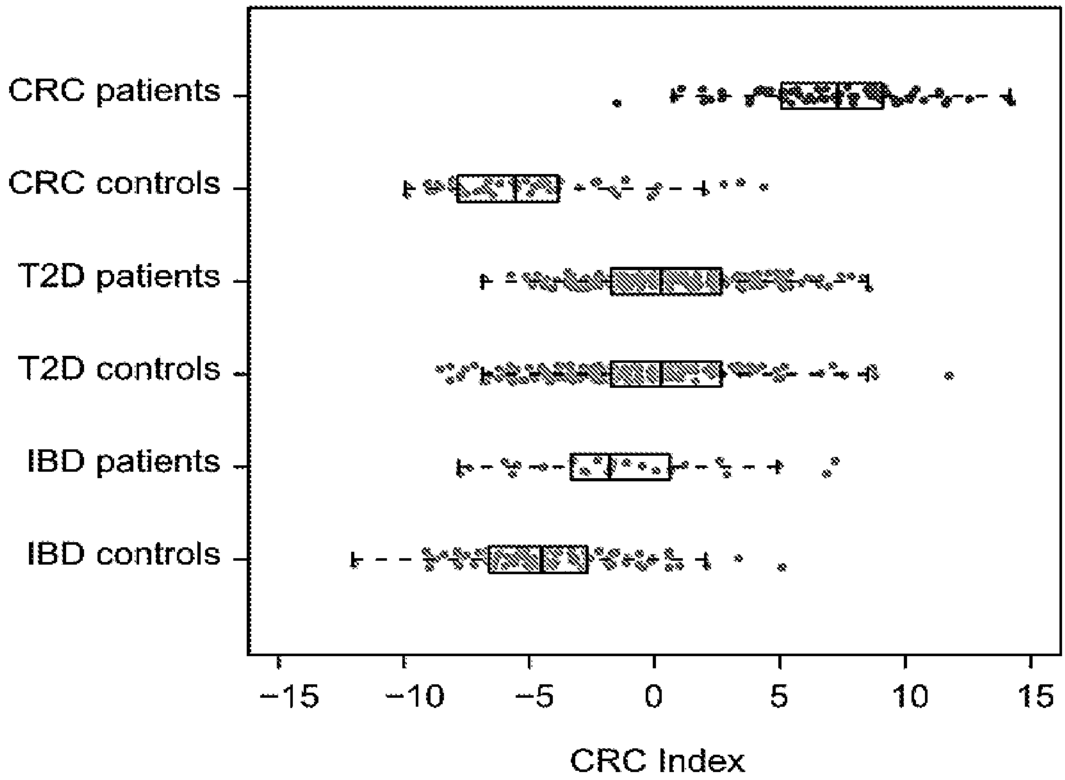
CRC patients
CRC controls
T2D patients
T2D controls
IBD patients
IBD controls
−15
−10
−5
0
5
10
15
CRC Index

Fig.9

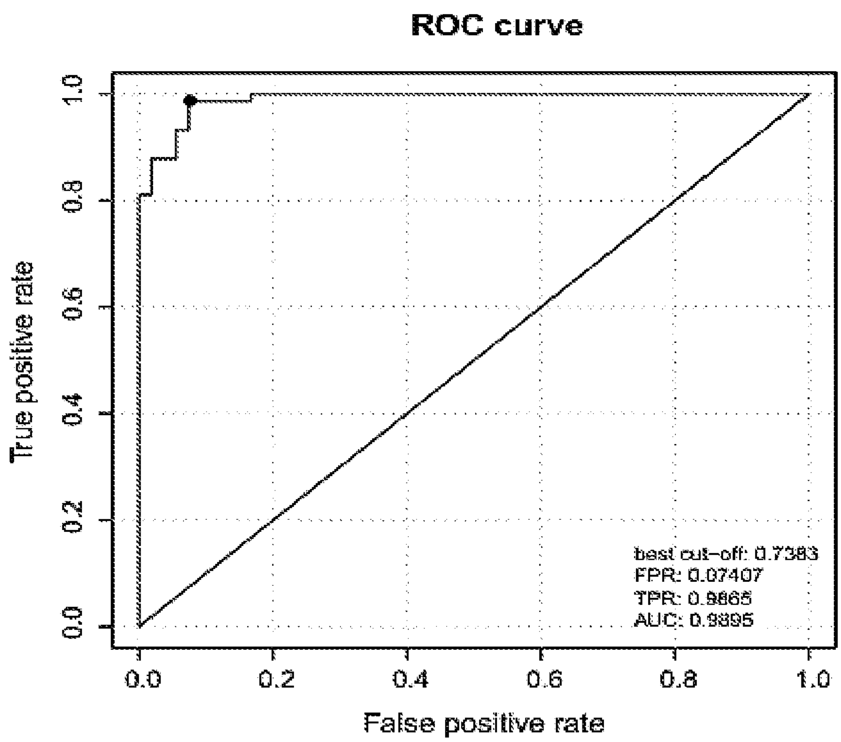
ROC curve
True positive rate
0.0
0.2
0.4
0.6
0.8
1.0
False positive rate
best cut−off: 0.7383
FPR: 0.07407
TPR: 0.9865
AUC: 0.9895

Fig.10

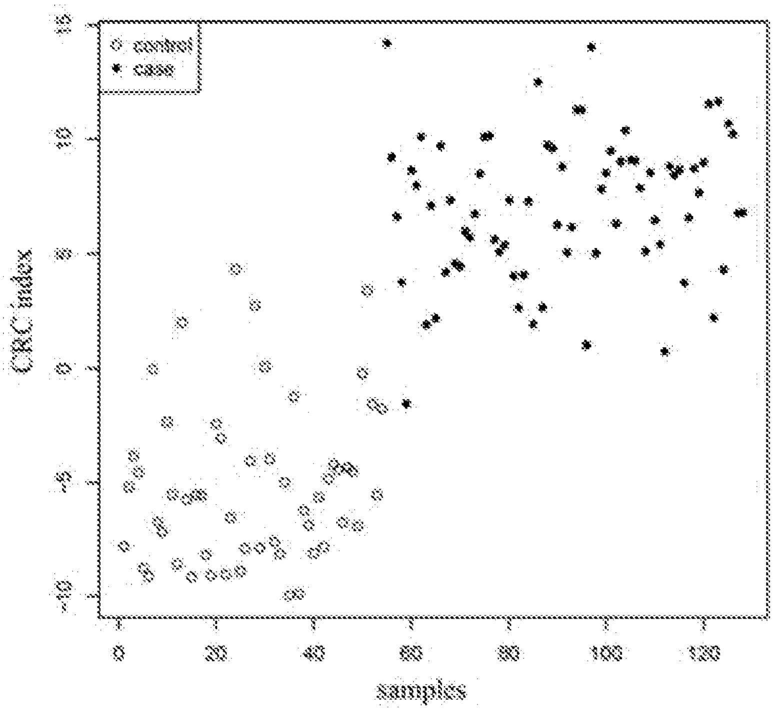

control
case
CRC index
samples


Fig.11

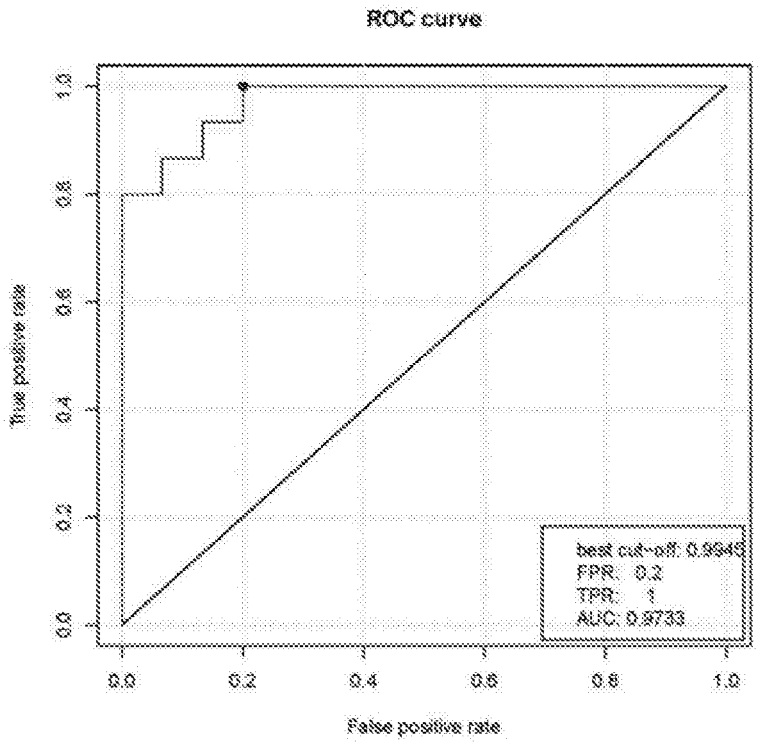

ROC curve
True positive rate
False positive rate
best cut-off: 0.9945
FPR: 0.2
TPR: 1
AUC: 0.9733


Fig.12

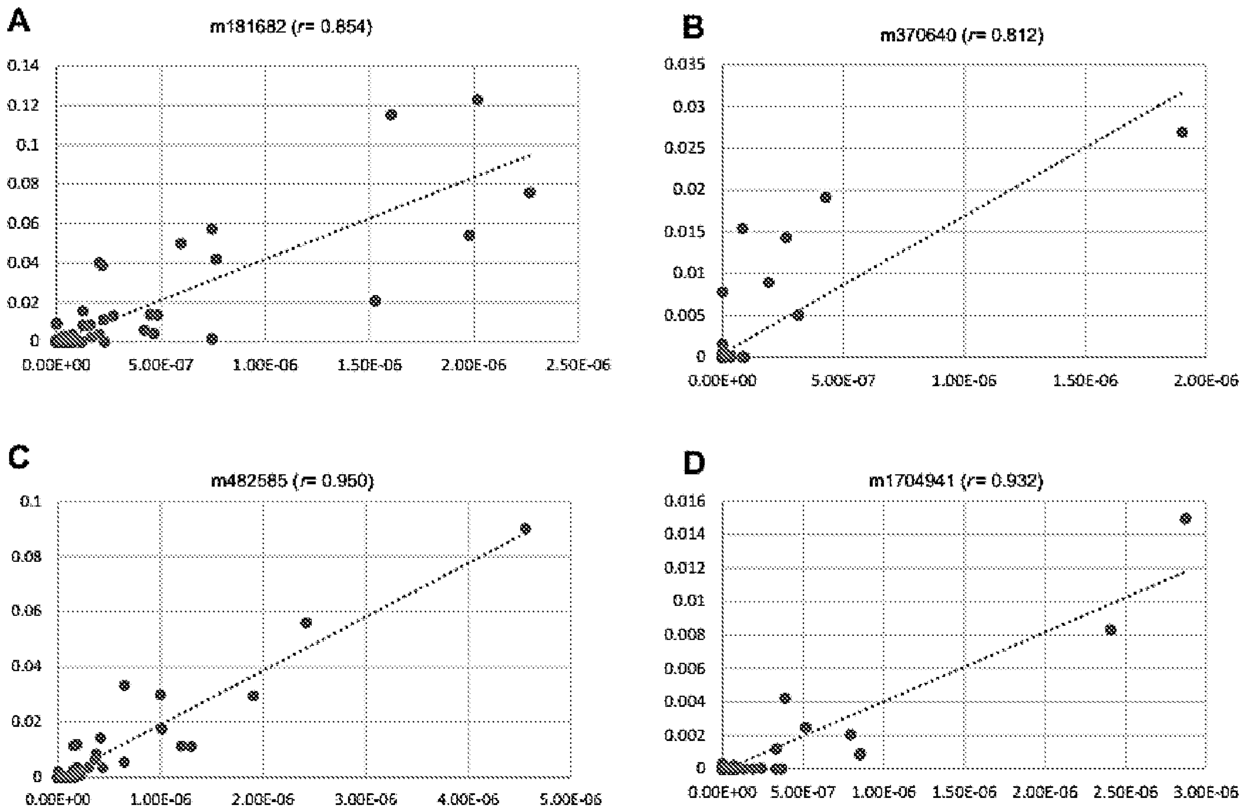


Fig.13

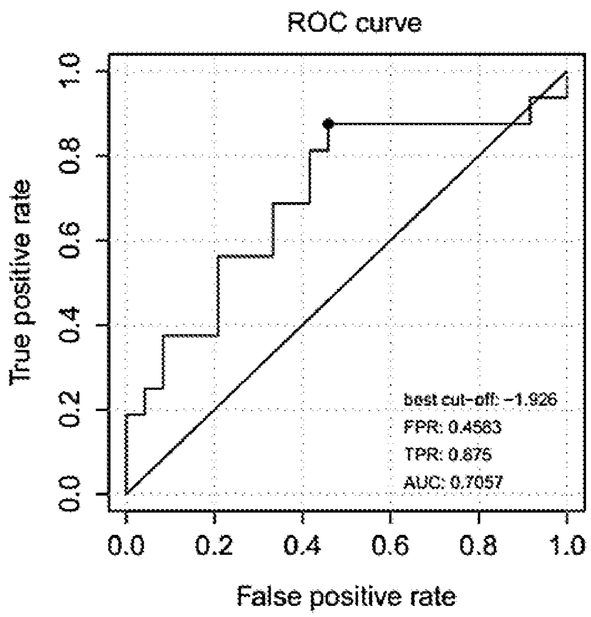


Fig.14-1

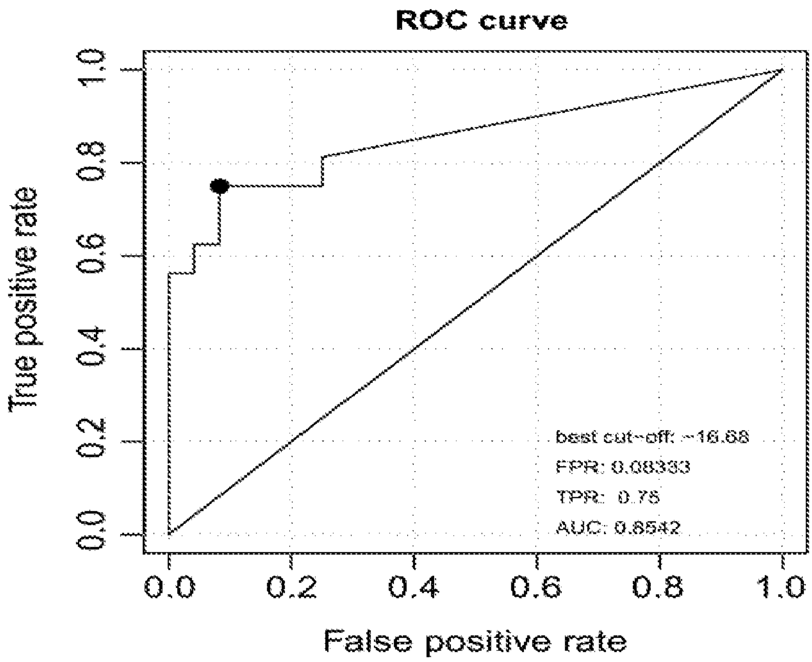


Fig.14-2

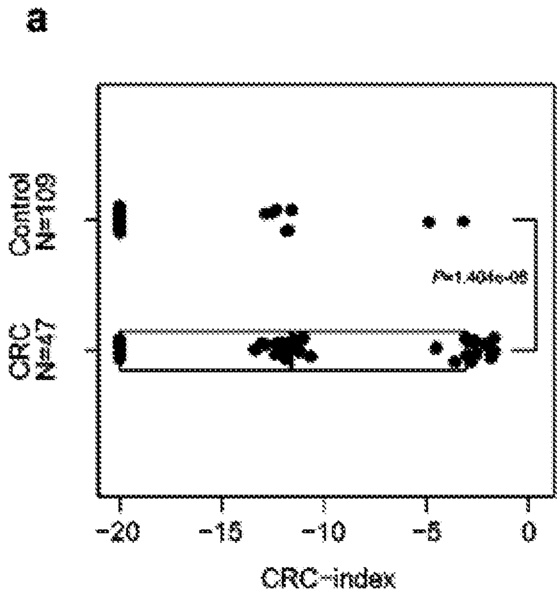


Fig.15a

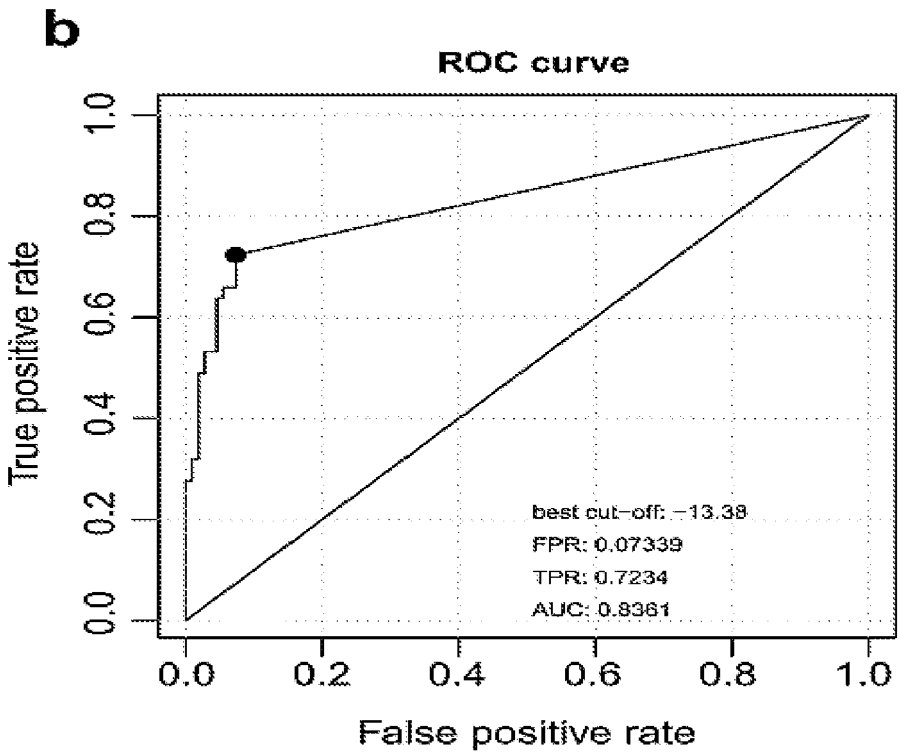
b
ROC curve
True positive rate
False positive rate
0.0
0.2
0.4
0.6
0.8
1.0
best cut-off: -13.38
FPR: 0.07339
TPR: 0.7234
AUC: 0.8361

Fig.15b

c

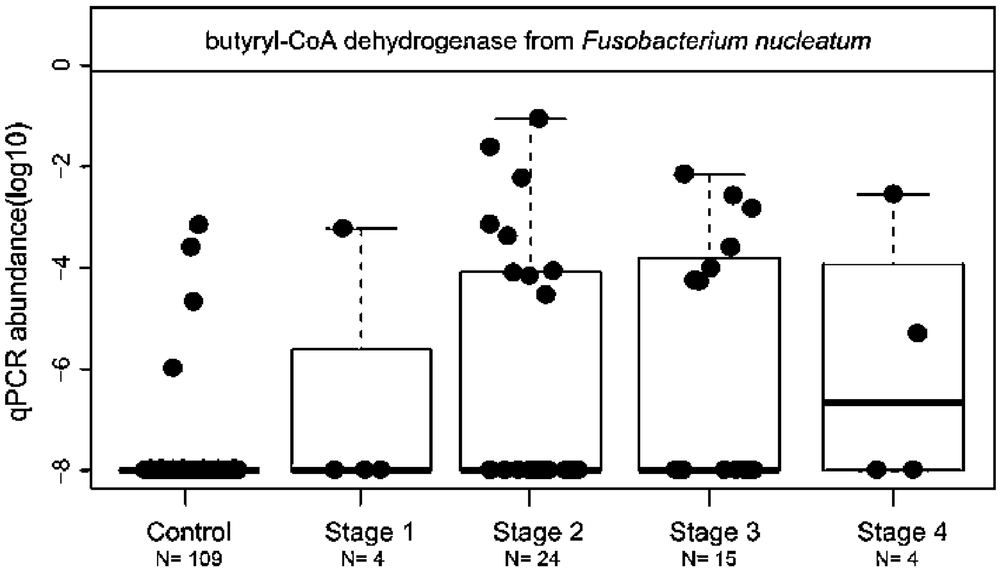


Fig.15c

d

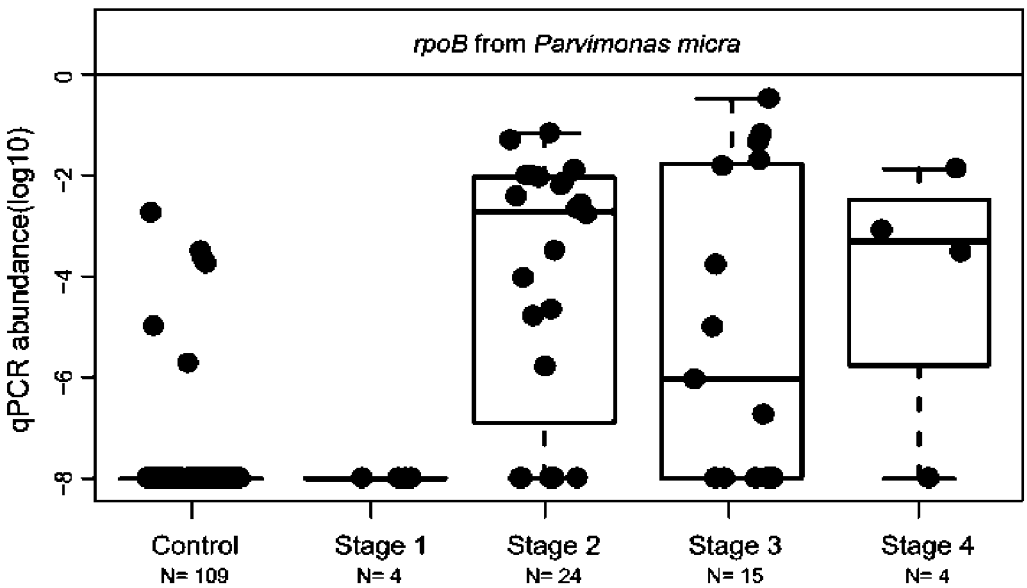


Fig.15d

Fig.15

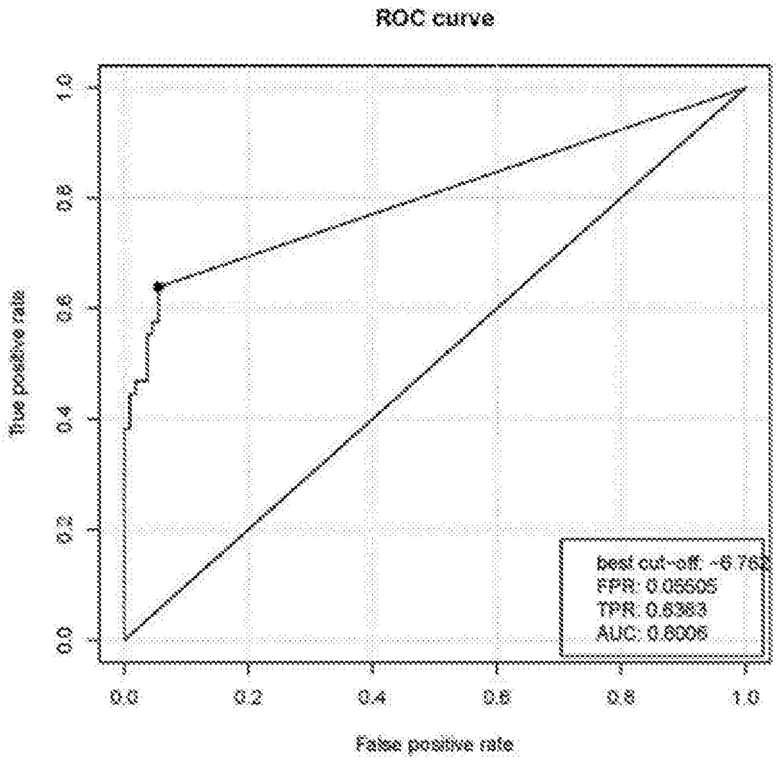
ROC curve
True positive rate
False positive rate
0.0
0.2
0.4
0.6
0.8
1.0
best cut-off:
FPR: 0.05505
TPR: 0.6383
AUC: 0.8006

**Fig.16**

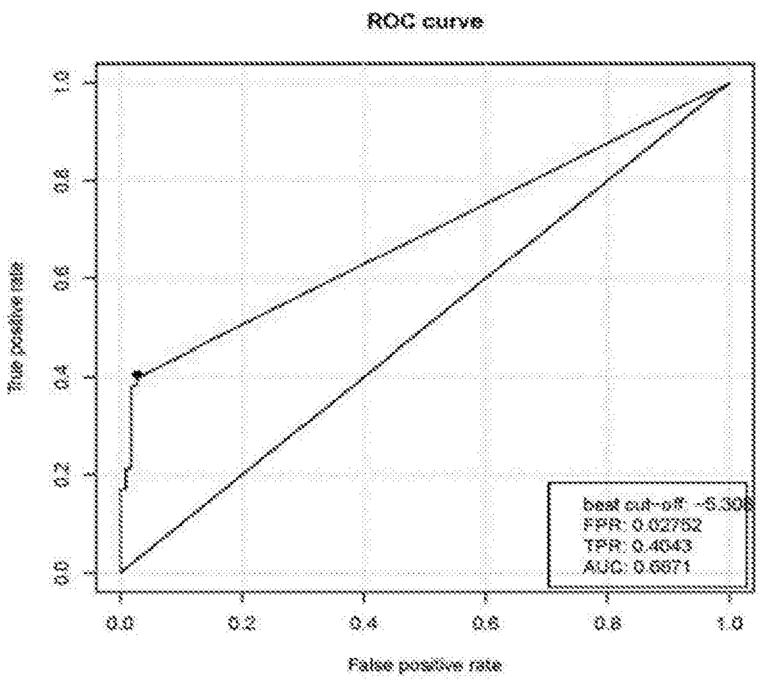
ROC curve
True positive rate
False positive rate
0.0
0.2
0.4
0.6
0.8
1.0
best cut-off:
FPR: 0.02752
TPR: 0.4043
AUC: 0.6871

**Fig.17**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SCHETTER AJ ; HARRIS CRC.** Alterations of microRNAs contribute to colon carcinogenesis. *Semin Oncol,* 2011, vol. 38, 734-742
- **MCGARR SE ; RIDLON JM ; HYLEMON PB.** Diet, anaerobic bacterial metabolism, and colon cancer. *J Clin Gastroenterol.,* 2005, vol. 39, 98-109
- **HATAKKA K ; HOLMA R ; EL-NEZAMI H ; SUOMALAINEN T ; KUISMA M ; SAXELIN M ; POUSSA T ; MYKKÄNEN H ; KORPELA R.** The influence of Lactobacillus rhamnosus LC705 together with Propionibacterium freudenreichii ssp. shermanii JS on potentially carcinogenic bacterial activity in human colon. *Int J Food Microbiol.,* 2008, vol. 128, 406-410
- **CASTELLARIN et al.** *Genome Res.,* 2011, vol. 22, 299-306
- **RUBINSTEIN et al.** *Cell Host & Microbe,* 2013, vol. 14, 195-206
- **ZELLER et al.** *Molecular Systems Biology,* 2014, vol. 10, 766
- **KOSTIC et al.** *Genome Res.,* 2011, vol. 22, 292-298
- **QIN, J. et al.** A metagenome-wide association study of gut microbiota in type 2 diabetes. *Nature,* 2012, vol. 490, 55-60
- **E. M. GONÇALVES ; E. M. SALOMÃO ; M. C. C. GOMES-MARCONDES.** Leucine modulates the effect of Walker factor, a proteolysis-inducing factor-like protein from Walker tumours, on gene expression and cellular activity in C2C12 myotubes. *Cytokine,* 2013, vol. 64, 343
- **M. R. RUBINSTEIN et al.** Fusobacterium nucleatum promotes colorectal carcinogenesis by modulating E-cadherin/beta-catenin signaling via its FadA adhesin. *Cell Host Microbe,* 14 August 2013, vol. 14, 195
- **J. J. GODON ; E. ZUMSTEIN ; P. DABERT ; F. HABOUZIT ; R. MOLETTA.** Molecular microbial diversity of an anaerobic digestor as determined by small-subunit rDNA sequence analysis. *Applied and environmental microbiology,* July 1997, vol. 63, 2802
- **S. SUNAGAWA et al.** Metagenomic species profiling using universal phylogenetic marker genes. *Nature methods,* December 2013, vol. 10, 1196
- **V. M. MARKOWITZ et al.** IMG: the Integrated Microbial Genomes database and comparative analysis system. *Nucleic acids research,* January 2012, vol. 40, D115, http://ftp.jgi-psf.org.
- **R. LI et al.** SOAP2: an improved ultrafast tool for short read alignment. *Bioinformatics,* 01 August 2009, vol. 25, 1966
- **J. D. STOREY ; R. TIBSHIRANI.** Statistical significance for genomewide studies. *Proceedings of the National Academy of Sciences of the United States of America,* 05 August 2003, vol. 100, 9440
- **V. M. MARKOWITZ et al.** IMG: the Integrated Microbial Genomes database and comparative analysis system. *Nucleic acids research,* January 2012, vol. 40, D115
- **D. KNIGHTS ; E. K. COSTELLO ; R. KNIGHT.** Supervised classification of human microbiota. *FEMS microbiology reviews,* March 2011, vol. 35, 343
- **H. PENG ; F. LONG ; C. DING.** Feature selection based on mutual information: criteria of max-dependency, max-relevance, and min-redundancy. *IEEE transactions on pattern analysis and machine intelligence,* August 2005, vol. 27, 1226
- **J. QIN et al.** A human gut microbial gene catalogue established by metagenomic sequencing. *Nature,* 04 March 2010, vol. 464, 59
- **J. LI et al.** An integrated catalog of reference genes in the human gut microbiome. *Nature biotechnology,* August 2014, vol. 32, 834
- **J. AHN et al.** Human gut microbiome and risk for colorectal cancer. *Journal of the National Cancer Institute,* 18 December 2013, vol. 105, 1907
- **F. D. CICCARELLI et al.** Toward automatic reconstruction of a highly resolved tree of life. *Science,* 03 March 2006, vol. 311, 1283
- **G. ZELLER et al.** Potential of fecal microbiota for early-stage detection of colorectal cancer. *Molecular systems biology,* 2014, vol. 10, 766
- **J. P. ZACKULAR ; M. A. ROGERS ; M. T. T. RUFFIN ; P. D. SCHLOSS.** The human gut microbiome as a screening tool for colorectal cancer. *Cancer prevention research,* November 2014, vol. 7, 1112